(19) Europäisches Patentamt
European Patent Office
Office européen des brevets

(11) EP 4 420 666 A1

(12) EUROPEAN PATENT APPLICATION
published in accordance with Art. 153(4) EPC

(43) Date of publication:
**28.08.2024 Bulletin 2024/35**

(21) Application number: **22883677.1**

(22) Date of filing: **21.10.2022**

(51) International Patent Classification (IPC):
***A61K 31/575*** (2006.01) ***A61K 31/57*** (2006.01)
***A61K 31/58*** (2006.01) ***A61K 39/395*** (2006.01)
***A61K 45/00*** (2006.01) ***A61P 35/00*** (2006.01)
***A61P 37/02*** (2006.01) ***A61P 43/00*** (2006.01)
***C07J 9/00*** (2006.01) ***C07K 16/28*** (2006.01)
***C12N 15/13*** (2006.01)

(52) Cooperative Patent Classification (CPC):
**A61K 31/57; A61K 31/575; A61K 31/58; A61K 39/395; A61K 45/00; A61P 35/00; A61P 37/02; A61P 43/00; C07J 9/00; C07K 16/00; C07K 16/28**

(86) International application number:
**PCT/JP2022/039416**

(87) International publication number:
**WO 2023/068376 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.10.2021 JP 2021173456**

(71) Applicants:
- **Kyushu University, National University Corporation**
**Nishi-ku**
**Fukuoka-shi**
**Fukuoka 819-0395 (JP)**
- **RIKEN**
**Wako-shi, Saitama 351-0198 (JP)**
- **Keio University**
**Tokyo, 108-8345 (JP)**

(72) Inventors:
- **FUKUI, Yoshinori**
**Fukuoka-shi, Fukuoka 819-0395 (JP)**
- **URUNO, Takehito**
**Fukuoka-shi, Fukuoka 819-0395 (JP)**
- **HONMA, Teruki**
**Wako-shi, Saitama 351-0198 (JP)**
- **TAKAYA, Daisuke**
**Wako-shi, Saitama 351-0198 (JP)**
- **KANAI, Motomu**
**Tokyo 113-8654 (JP)**
- **OISAKI, Kounosuke**
**Tokyo 113-8654 (JP)**
- **TOGO, Takaya**
**Tokyo 113-8654 (JP)**
- **SUGIURA, Yuki**
**Tokyo 160-8582 (JP)**

(74) Representative: **Vossius & Partner**
**Patentanwälte Rechtsanwälte mbB**
**Siebertstrasse 3**
**81675 München (DE)**

Remarks:
The complete document including Reference Table(s) and the Sequence Listing(s) can be downloaded from the EPO website

# (54) PHARMACEUTICAL COMPOSITION HAVING ANTI-CANCER IMMUNOSTIMULATING EFFECT AND/OR IMMUNE CHECKPOINT INHIBITION POTENTIATING EFFECT

(57) Provided is a pharmaceutical composition effective for anti-cancer immunostimulating therapies. The pharmaceutical composition contains a compound or a salt thereof as an active ingredient, the compound represented by general formula (I): [Formula 1] {in the formula, one of $X_1$ and $X_2$ is a hydroxy group, a morpholino alkylcarbonyloxy group, an alkylcarbonyloxy group, a phosphate group, an alkoxy group, a di(alkyl)amino alkyltriazolyl group, an acetoxy group in which at least one hydrogen atom is optionally replaced with a halogen at-

EP 4 420 666 A1

om, an azide group, or a sulfate group, and the other is a hydrogen atom, or $X_1$ and $X_2$ are combined to represent an oxy group; Y represents a $C_{1-6}$ alkyl group or a hydrogen atom; $Z_1$ and $Z_2$ are different and each represent a group represented by formula (II): [Formula 2], a hydroxy group, an ethynyl group, an alkylcarbonyloxy group, a hydrogen atom, or a heterocyclic group optionally having a substituent, or $Z_1$ and $Z_2$ are combined to represent an oxy group; and each double line composed of a solid line and a dotted line indicates a single bond or a double bond, wherein signs present in formula (II) are as follows: $R_1$ is an alkyl group or a hydrogen atom; $R_2$ is a hydroxy group, an alkyl group, an alkoxy group, a group represented by formula (III): [Formula 3] [in formula (III), * indicates a bond to the carbon atom of the carbonyl group in formula (II)], or $-N(R_3)(R_4)$ ($R_3$ and $R_4$ are the same or different, a hydrogen atom, an alkyl group, a phenyl alkyl group, a group represented by formula (IV): [Formula 4] (in formula (IV), p denotes an integer of 1 to 10, and * indicates a bond to the carbon atom of the carbonyl group in formula (II)), or $R_3$ and $R_4$ are combined to form a heterocyclic ring together with the nitrogen atom); n is an integer of 0, 1, or 2; and m is an integer of 1 or 0}.

## Description

Technical Field

The present invention relates to a pharmaceutical composition having an anti-cancer immunostimulating effect and/or an immune checkpoint inhibition potentiating effect. The present invention also relates to a novel compound effective as an active ingredient of a pharmaceutical composition having an anti-cancer immunostimulating effect and/or an immune checkpoint inhibition potentiating effect.

Background Art

Immunotherapies including immune checkpoint inhibition have brought about a revolution in cancer therapy, whereas there are many patients who have not yet received the clinical benefits (NPL 1). Multiple mechanisms peculiar to tumor that affect the generation and functions of cancer-specific T cells may cause resistance to cancer immunotherapy. Those include lack of antigen mutation, destroy of antigen processing mechanisms, loss of major histocompatibility complex (MHC) expression, and defects of signaling via interferon-$\gamma$ (IFN-$\gamma$) or a tumor necrosis factor (TNF) that acts to enhance tumor immune sensitivity. Tumors often constitute a special microenvironment that eliminates T cells from the vicinity of cancer cells even when functional cancer-specific T cells are present in regional lymph nodes (immune evasion, immune privilege). Effective cancer immunotherapy needs physical contact of T cells with cancer cells, and hence development of more effective cancer immunotherapy requires elucidation of the mechanism of the immune evasion.

Previously, epigenetic silencing (epigenetic gene function suppression) of expression of genes encoding chemokines in tumor microenvironments and oncogenic pathways via WNT/$\beta$-catenin signaling in tumors have been reported to be associated with the T cell exclusion. However, other mechanisms to prevent tumor infiltration by T cells are still almost unknown.

DOCK2 (dedicator of cytokinesis 2) is a mammalian homolog of *Caenorhabditis elegans* CED-5 and *Drosophila melanogaster* Myoblast City, and primarily expressed in hematopoietic cells. DOCK2 does not contain a Dbl homology (DH) domain, which is typically found in guanine-nucleotide exchange factors (GEFs), but mediates the GTP-GDP exchange reaction of Rac via a DOCK homology region (DHR)-2 domain. Evidences accumulated in mice have revealed that DOCK2 is a main Rac GEF that acts in the downstream of chemotactic receptors and antigen receptors in lymphocytes, and plays an important role in the migration and activation of lymphocytes. In addition, DOCK2 mutation has been reported to cause severe immunodeficiency in humans. Accordingly, DOCK2 is a molecule essential for the immune surveillance in humans and mice.

Sulfation plays key roles in the bioactivities of many endogenous molecules.

We have reported that cholesterol 3-sulfate (CS: cholesterol sulfate: CAS No. 1256-86-6) (herein, referred to as "CS"), a sulfated product of cholesterol, acts as an endogenous inhibitory factor for DOCK2. CS directly binds to a catalytic DHR-2 domain of DOCK2 to inhibit the Rac GEF activity. Such an inhibitory effect is specific to CS, and not found for other cholesterol derivatives. In humans and mice, sulfation of cholesterol by addition of a sulfate group to the hydroxy group at position 3 of cholesterol is mediated by the sulfotransferase SULT2B1b and, to a lesser degree, SULT2B1a, which is generated from the same Sult2b1 gene through alternative splicing (so far, NPL 2). Furthermore, high expression of Sult2b1 has been reported to correlate with poor prognoses of colon cancer (NPL 3). Moreover, mass spectrometry imaging of human prostate cancer and normal tissues has revealed that CS is found almost specifically in cancerous tissues (NPL 4). Therefore, CS possibly plays an important role in the immune evasion of tumors.

Focusing on such an immune control mechanism via DOCK2 inhibition by CS, we have proposed methods for controlling immunity by controlling functional expression of CS (PTL 1). Specifically proposed are: a method in which an immune-privileged site is generated by allowing the enzyme SULT2B1b, which performs sulfation of cholesterol, to generate CS in vivo, thereby suppressing rejection; and a method of promoting immune response in cancer tissues (activation of cancer immunity) by inhibiting expression/production of SULT2B1b in cancer tissues to cancel the immune privilege.

Cholenoic acid (3$\beta$-hydroxy-$\Delta$5-cholenoic acid: CAS No. 5255-17-4) is a component constituting significant part of bile acid contained in human meconium and amniotic fluid (NPLs 5, 6). However, the physiological functions are still unknown.

Citation List

Patent Literature

PTL 1: International Publication No. WO 2017/086436 Non-patent Literature

NPL 1: A Ribas, J. D. Wolchok, Cancer immunotherapy using checkpoint blockade. Science 359, 1350-1355 (2018).
NPL 2: T. Sakurai, T. Uruno, Y. Sugiura, T. Tatsuguchi, K. Yamamura, M. Ushijima, Y. Hattori, M. Kukimoto-Niino, C. Mishima-Tsumagari, M. Watanabe, M. Suematsu, Y Fukui, Cholesterol sulfate is a DOCK2 inhibitor that mediates tissue-specific immune evasion in the eye. Sci. Signal. 11, eaao4874 (2018).
NPL 3: L. Hu, G. Z. Yang, Y. Zhang, D. Feng, Y. X. Zhai, H. Gong, C. Y. Qi, H. Fu, M. M. Ye, Q. P. Cai, C. F. Gao, Overexpression of SULT2B1b is an independent prognostic indicator and promotes cell growth and invasion in colorectal carcinoma. Lab. Invest. 95, 1005-1018 (2015).
NPL 4: L. S. Eberlin, A. L. Dill, A. B. Costa, D. R. Ifa, L. Cheng, T. Masterson, M. Koch, T. L. Ratliff, R. G. Cooks, Cholesterol sulfate imaging in human prostate cancer tissue by desorption electrospray ionization mass spectrometry. Anal. Chem. 82, 3430-3434 (2010).
NPL 5: G. Deleze, G. Paumgartner, G. Karlaganis, W. Giger, M. Reinhard, D. Sidiropoulos, Bile acid pattern in human amniotic fluid. Eur. J. Cli. Invest. 8, 41-45 (1978).
NPL 6: E. I. Minder, G. Karlaganis, G. Paumgartner, Radioimmunological determination of serum 3β-hydroxy-5-cholenoic acid in normal subjects and patients with liver disease. J. Lipid. Res. 20, 986-993 (1979).
NPL 7: Veryser, C.; Demaerel, J.; Bieliunas, V.; Gilles, P.; De Borggraeve, W. M. Org. Lett. 2017, 19, 5244.
NPL 8: T. H. Corbett, D. P. Griswold Jr., B. J. Roberts, J. C. Peckham, F. M. Schabel Jr., Tumor induction relationship in development of transplantable cancers of the colon in mice for chemotherapy assays with a note on carcinogen structure. Cancer Res. 35, 2434-2439 (1975).
NPL 9: R. J. Motzer, B. Escudier, D.F. McDermott, S. George, H.J. Hammers, S. Srinivas, S.S. Tykodi, J.A. Sosman, G. Procopio, E.R. Plimack, D. Castellano, T.K. Choueiri, H. Gurney, F. Donskov, P. Bono, J. Wagstaff, T.C. Gauler, T. Ueda, Y. Tomita, F.A. Schutz, C. Kollmannsberger, J. Larkin, A. Ravaud, J.S. Simon, L. A. Xu, I.M. Waxman, P. Sharma, Nivolumab versus Everolimus in advanced renal-cell carcinoma. N. Engl. J. Med. 373, 1803-1813 (2015).
NPL 10: M. W. Moore, F. R. Carbone, M. J. Bevan, Introduction of soluble protein into the class I pathway of antigen processing and presentation. Cell 54, 777-785 (1988).
NPL 11: K. A. Hogquist, S. C. Jameson, W. R. Heath, J. L. Howard, M. J. Bevan, F. R. Carbone, T cell receptor antagonist peptides induce positive selection. Cell 76, 17-27 (1994).
NPL 12: C. N. Falany, K. J. Rohn-Glowacki, SULT2B1: unique properties and characteristics of a hydroxysteroid sulfotransferase family. Drug Metab. Rev. 45, 388-400 (2013).
NPL 13: W. Chen, G. Chen, D. L. Head, D. J. Mangelsdorf, D. W. Russell, Enzymatic reduction of oxysterols impairs LXR signaling in cultured cells and the livers of mice. Cell Metab. 5, 73-79 (2007).
NPL 14: Thornqvist, V.; Manner, S.; Wendt, O. F.; Frejd, T. Tetrahedron 2006, 62, 11793.
NPL 15: Domon, K.; Puripat, M; Fujiyoshi, K; Hatanaka, M.; Kawashima, S. A.; Yamatsugu, K.; Kanai, M. ACS Cent. Sci. 2020, 6, 2, 283.
NPL 16: Bauder, C. Org. Biomol. Chem., 2008, 6, 2952.

Summary of Invention

Technical Problem

An object of the present invention is to provide a pharmaceutical composition having an anti-cancer immunostimulating effect and/or an immune checkpoint inhibition potentiating effect. Another object of the present invention is to provide a novel compound effective as an active ingredient of the pharmaceutical composition.

Solution to Problem

Through diligent study, the present inventors have found that: 1) CS is abundantly produced in human cancer tissues of specific type such as colon cancer and prostate cancer; 2) CS-producing cancer cells exhibit resistance to transplantation of cancer-specific T cells and immune checkpoint inhibition; 3) cholenoic acid, which is an endogenous component present in vivo, exerts SULT2B1b (sulfotransferase family cytosolic 2B member 1 isoform b) inhibitory effect in vivo to suppress CS production; and 4) oral administration of cholenoic acid to mice allows immunotherapy to effectively work against CS-producing cancer cells.

These findings have convinced the present inventors that cholenoic acid or a salt thereof is an effective and safe component that cancels immune privilege by inhibiting the functions of SULT2B1b to suppress CS production and thereby potentiates cancer immunotherapies.

Based on those findings, diligent study has been further conducted to find, in addition to cholenoic acid and salts thereof, compounds that have the same effect as cholenoic acid and are effective as an active ingredient of a

SULT2B1b inhibitor, a CS production inhibitor, or a pharmaceutical composition having an anti-cancer immunostimulating effect (anti-cancer immunostimulator), resulting in the completion of the present invention, which includes the following embodiments.

(I) SULT2B1b inhibitor

(I-1) A SULT2B1b inhibitor containing a compound or a salt thereof as an active ingredient, the compound represented by general formula (I) (hereinafter, referred to as "compound I"):

[Formula 1]

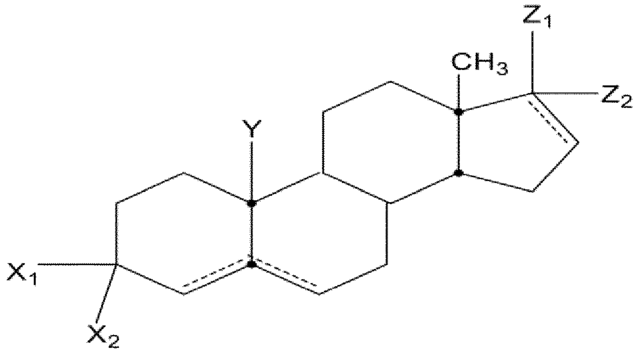

(I)

wherein

one of $X_1$ and $X_2$ is a hydroxy group, a morpholino $C_{1-6}$ alkylcarbonyloxy group, a $C_{1-6}$ alkylcarbonyloxy group, a phosphate group, a $C_{1-6}$ alkoxy group, a di ($C_{1-6}$ alkyl) amino $C_{1-6}$ alkyltriazolyl group, an acetoxy group in which at least one hydrogen atom is optionally replaced with a halogen atom, an azide group, or a sulfate group, and the other is a hydrogen atom, or $X_1$ and $X_2$ are combined to represent an oxy group;
Y represents a $C_{1-6}$ alkyl group or a hydrogen atom;
$Z_1$ and $Z_2$ are different and each represent a hydroxy group, an ethynyl group, a $C_{1-6}$ alkylcarbonyloxy group, a hydrogen atom, a heterocyclic group optionally having a substituent, or a group represented by formula (II):

[Formula 2]

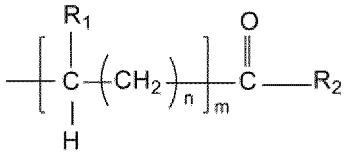

(II)

wherein

$R_1$ represents a $C_{1-6}$ alkyl group or a hydrogen atom,
$R_2$ represents a hydroxy group, a $C_{1-6}$ alkyl group, a $C_{1-6}$ alkoxy group, a group represented by formula (III):

[Formula 3]

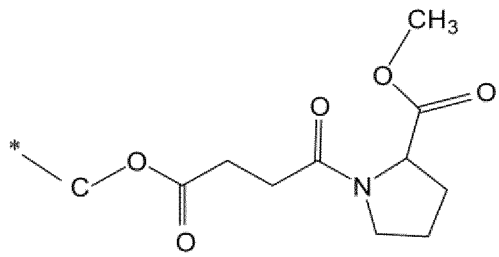

(III)

wherein * indicates a bond to the carbon atom of the carbonyl group in formula (II), or

$-N(R_3)(R_4)$,

wherein $R_3$ and $R_4$ are the same or different, each being a hydrogen atom, a $C_{1-6}$ alkyl group, a phenyl $C_{1-6}$ alkyl group, or a group represented by formula (IV):

[Formula 4]

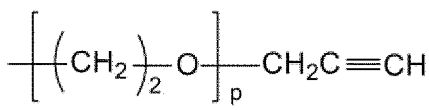

(IV)

wherein p denotes an integer of 1 to 10, and in this case, one of $R_3$ and $R_4$ is a hydrogen atom, or $R_3$ and $R_4$ are combined to form a heterocyclic ring together with the nitrogen atom,

n is an integer of 0, 1, or 2, and
m is an integer of 1 or 0, or
$Z_1$ and $Z_2$ are combined to represent an oxo group; and
each double line composed of a solid line and a dotted line in formula (I) indicates a single bond or a double bond.

(1-2) The SULT2B1b inhibitor according to (I-1), wherein, in formula (I),

one of $X_1$ and $X_2$ is a hydroxy group, a morpholino $C_{1-6}$ alkylcarbonyloxy group, or trihalogenoacetoxy group, and the other is a hydrogen atom;
Y is a $C_{1-6}$ alkyl group; and
one of $Z_1$ and $Z_2$ is a group represented by formula (II) :

[Formula 5]

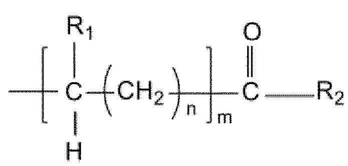

(II)

wherein

$R_1$ is a $C_{1-6}$ alkyl group or a hydrogen atom,
$R_2$ is a hydroxy group, a $C_{1-6}$ alkyl group, or a $C_{1-6}$ alkoxy group,
n is an integer of 0, 1, or 2, and
m is 1, and

the other is a hydrogen atom, or
$Z_1$ and $Z_2$ are combined to form an oxo group.

(II) CS production inhibitor

(II-1) An inhibitor for SULT2B1b-mediated production of cholesterol-3-sulfate (CS), the inhibitor containing compound I according to (I-1) or a salt thereof as an active ingredient.
(II-2) The CS production inhibitor according to (II-1), wherein compound I is a compound represented by general formula (I) described in (I-1), wherein

one of $X_1$ and $X_2$ is a hydroxy group, a morpholino $C_{1-6}$ alkylcarbonyloxy group, or a trihalogenoacetoxy group, and the other is a hydrogen atom;
Y is a $C_{1-6}$ alkyl group; and
one of $Z_1$ and $Z_2$ is a group represented by formula (II) :

[Formula 6]

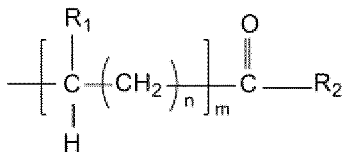

(II)

wherein

$R_1$ is a $C_{1-6}$ alkyl group or a hydrogen atom,
$R_2$ is a hydroxy group, a $C_{1-6}$ alkyl group, or a $C_{1-6}$ alkoxy group,
n is an integer of 0, 1, or 2, and
m is 1, and

the other is a hydrogen atom, or
$Z_1$ and $Z_2$ are combined to form an oxo group.

(III) Pharmaceutical composition

(III-1) A pharmaceutical composition containing compound I according to (I-1) or a salt thereof as an active ingredient.
(III-2) The pharmaceutical composition according to (III-1), wherein compound I is a compound represented by general formula (I) described in (I-1), wherein

one of $X_1$ and $X_2$ is a hydroxy group, a morpholino $C_{1-6}$ alkylcarbonyloxy group, or a trihalogenoacetoxy group, and the other is a hydrogen atom;
Y is a $C_{1-6}$ alkyl group; and
one of $Z_1$ and $Z_2$ is a group represented by formula (II) :

[Formula 7]

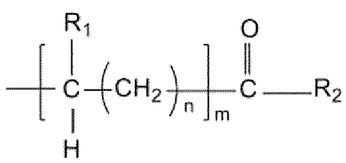

(II)

wherein

$R_1$ is a $C_{1-6}$ alkyl group or a hydrogen atom,
$R_2$ is a hydroxy group, a $C_{1-6}$ alkyl group, or a $C_{1-6}$ alkoxy group,
n is an integer of 0, 1, or 2, and
m is 1, and

the other is a hydrogen atom, or
$Z_1$ and $Z_2$ are combined to form an oxo group.

(III-3) The pharmaceutical composition according to (III-1) or (III-2), being an anti-cancer immunostimulator or an immune checkpoint inhibition potentiator.
(III-4) The pharmaceutical composition according to any of (III-1) to (III-3), for suppressing growth or progress of a tumor cell that produces CS.
(III-5) The pharmaceutical composition according to any of (III-1) to (III-4), wherein the pharmaceutical composition is used in combination with an anti-cancer agent or an anti-cancer therapy.
(III-6) The pharmaceutical composition according to (III-5), wherein the anti-cancer agent is an immune checkpoint inhibitory drug, and the anti-cancer therapy is a cancer immunotherapy.
(III-7) The pharmaceutical composition according to (III-6), wherein the immune checkpoint inhibitory drug is an anti-PD-1 antibody or an anti-PD-L1 antibody, and the cancer immunotherapy is CAR-T therapy.
(III-8) The pharmaceutical composition according to (III-6) or (III-7), wherein the pharmaceutical composition is used in combination with an immune checkpoint inhibitory drug for suppressing growth or progress of a tumor cell resistant to an anti-cancer therapy with the immune checkpoint inhibitory drug.

(IV) Compound having anti-cancer immunostimulating effect and/or immune checkpoint inhibition potentiating effect

A compound or a salt thereof, the compound represented by general formula (I):

[Formula 8]

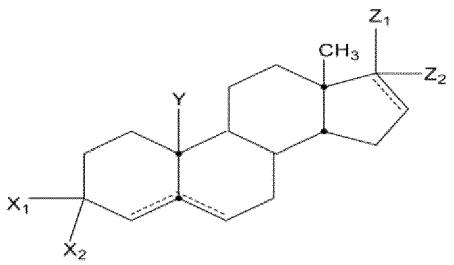

(I)

wherein

one of $X_1$ and $X_2$ is a hydroxy group, a morpholino $C_{1-6}$ alkylcarbonyloxy group, a $C_{1-6}$ alkylcarbonyloxy group, a phosphate group, a $C_{1-6}$ alkoxy group, an acetoxy group in which at least one hydrogen atom is optionally replaced with a halogen atom, a di($C_{1-6}$ alkyl)amino $C_{1-6}$ alkyltriazolyl group, an azide group, or a sulfate group, and the other is a hydrogen atom;
Y is a $C_{1-6}$ alkyl group;
one of $Z_1$ and $Z_2$ is a group represented by formula (II) :

[Formula 9]

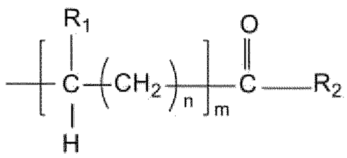

(II)

, and the other is a hydroxy group or a $C_{1-6}$ alkylcarbonyloxy group; and
each double line composed of a solid line and a dotted line indicates a single bond or a double bond, wherein signs present in formula (II) are as follows: $R_1$ is a $C_{1-6}$ alkyl group or a hydrogen atom;
$R_2$ is a hydroxy group, a $C_{1-6}$ alkyl group, a $C_{1-6}$ alkoxy group, a group represented by formula (III):

[Formula 10]

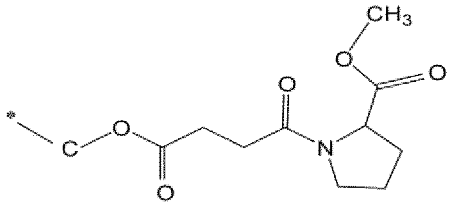

(III)

wherein * indicates a bond to the carbon atom of the carbonyl group in formula (II), or

$-N(R_3)(R_4)$,

wherein $R_3$ and $R_4$ are the same or different, each being a hydrogen atom, a $C_{1-6}$ alkyl group, a phenyl $C_{1-6}$ alkyl group, or a group represented by formula (IV):

[Formula 11]

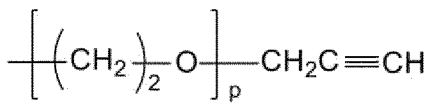

(IV)

wherein p denotes an integer of 1 to 10, and in this case, the other of $R_3$ and $R_4$ is a hydrogen atom, or $R_3$ and $R_4$ are combined to form a heterocyclic ring together with the nitrogen atom;

n is an integer of 0, 1, or 2; and
m is an integer of 1 or 0,
provided that the following compounds are excluded:

(i) (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanoic acid;
(ii) (4R)-4-[(3R,5R,8R,9S,10S,13R,14S,17R)-3-hydroxy-10,13-dimethyl-2,3,4,5,6,7,8,9,11,12,14,15,16,17-tetradecahydro-1H-cyclopenta[a]phenanthren-17-yl]pentanoic acid;
(iii) (8R,9S,10R,13S,14S)-17-acetyl-10,13-dimethyl-2,3,4,7,8,9,10,11,12,13,14,15-dodecahydro-1H-cyclopenta[a]phenanthren-3-yl 3-morpholinopropanoate;
(iv) (3R,5S,8R,9S,10S,13S,14S)-17-acetyl-10,13-dimethyl-2,3,4,5,6,7,8,9,10,11,12,13,14,15-tetradecahydro-1H-cyclopenta[a]phenanthren-3-yl 3-morpholinopropanoate;
(v) 3-acetoxy-5-cholenoic acid;
(vi) methyl (4-(2-8((8R,9S,10R,13S,14S,17R)-17-acetoxy-10,13-dimethyl-3-oxo-2,3,6,7,8,9,10,11,12,13,14,15,16,17-tetradecahydro-1H-cyclopenta[a]phenanthren-17-yl)-2-oxoethoxy)-4-oxobutanoyl)-L-prolinate; and
(vii) methyl (3β,5α)-3-hydroxycholan-24-oate.

(V) Method for screening for compound having anti-cancer immunostimulating effect and/or immune checkpoint inhibition potentiating effect

(V-1) A method for screening for a compound having an anti-cancer immunostimulating effect and/or an immune checkpoint inhibition potentiating effect, the method including the step of:
selecting a compound having a CS production inhibitory effect from a candidate compound group.

(V-2) The method according to (V-1), wherein the step of selecting is at least one selected from the group consisting of an in silico screening step, an SULT assay (in vitro SULT2B1b enzymatic activity measurement) step, and a quantification step for amounts of CS and/or oxysterol generated in a cell.

(V-3) The method according to (V-1) or (V-2), wherein the CS production inhibitory effect is an effect to inhibit SULT2B1b-mediated CS production.

(V-4) The method according to any of (V-1) to (V-3), wherein the compound having an anti-cancer immunostimulating effect and/or an immune checkpoint inhibition potentiating effect is a candidate compound for an active ingredient of a pharmaceutical composition having an anti-cancer immunostimulating effect and/or an immune checkpoint inhibition potentiating effect.

Advantageous Effects of Invention

The present invention can provide a pharmaceutical composition having an anti-cancer immunostimulating effect and/or an immune checkpoint inhibition potentiating effect. The pharmaceutical composition can be used even as a SULT2B1b inhibitor or a CS production inhibitor. Moreover, the present invention can provide a novel compound effective as an active ingredient of the pharmaceutical composition, a SULT2B1b inhibitor, or a CS production inhibitor.

Brief Description of Drawings

[Figure 1] Figure 1 is a series of diagrams showing experimental results (1) in Experiment Example 1 (CS production and the infiltration of $CD8^+$ T cells inversely correlate in human cancer tissues). Figure 1A is a graph for comparing Sult2b1 expression among 17 human cancer tissues. The data are derived from Human Protein Atlas (https://www.proteinatlas.org), and expressed as median values of FPKM values. Figure 1B is a graph for comparing CS production among different human cancer tissues. From the left, results for normal colon tissue (control: Normal colon), colon cancer tissue, renal cancer tissue, and prostate cancer tissue samples are shown in order. The data are expressed as mean ± SD. * $P < 0.05$; ** $P < 0.01$ (two-sided Mann-Whitney test).

Figure 1C shows representative images of CS production and $CD8^+$ T cell infiltration in colon cancer tissue samples. Scale bars in the images indicate 1 mm in the second column from the left, and 250 μm in the third and fourth columns from the left. The lower right diagram is a graph for comparing the number of infiltrating $CD8^+$ T cells in a high CS production region and that in a low CS production region (n = 10 for each) . The data are expressed as mean ± SD. ** $P < 0.01$ (unpaired Student t test for two groups).

[Figure 2] Figure 2 shows expression of SULT2B1b in different tumor cell lines used in the present study. Asterisks in the figure indicate SULT2B1b with an HA tag (*) and that without an HA tag (**). As shown in the figure, the tumor cell lines engineered to express SULT2B1b are a mouse breast cancer cell line (E0771-SULT), a mouse Lewis lung cancer cell line (3LL-SULT), a full-length-OVA-introduced T-cell lymphoma cell line (E.G7-OVA-SULT), a mouse colon cancer cell line (MC38-SULT), and a cholesterol 25-hydroxylase-deficient mouse colon cancer cell line (MC38-ΔCH25H-SULT).

[Figure 3] Figure 3 is a series of diagrams showing experimental results (2) in Experiment Example 1 (CS production in tumor tissues inhibits the infiltration of effector T cells). Figure 3A is a pair of graphs for comparing CS productions in cells (n = 6) and culture supernatant (n = 5) of a human breast cancer E0771 cell line for E0771-SULT (SULT2B1b-expressing cell line) and those for E0771-control (SULT2B1b-nonexpressing cell line). The data are expressed as mean ± SD. ** $P < 0.01$ (unpaired Student t test for two groups) . Figure 3B is a graph for comparing in vitro cell growth for E0771-control and that for E0771-SULT (n = 5). Figure 3C is a graph for comparing tumor growth in C57BL/6 mice after the transplantation for E0771-SULT and that for E0771-control (n = 8). The data are expressed as mean ± SD. * $P < 0.05$, ** $P < 0.01$ (unpaired Student t test for two groups). Figure 3D is a series of viSNE plots showing the distribution of tumor-infiltrating lymphocytes in E0771-SULT and E0771-control. Figure 3E is a series of graphs for comparing proportions of $CD8^+$ T cells and NK cells in infiltrative leukocytes ($CD45^+$ cells) and expression of granzyme B therein for E0771-SULT and those for E0771-control (n = 9). The data are expressed as mean ± SD. * $P < 0.05$ (unpaired Student t test for two groups) . Figure 3F is a graph for comparing tumor growth in BALB/c nude mice after the transplantation for E0771-SULT and that for E0771-control (n = 7) .

[Figure 4] Figure 4 is a series of diagrams showing experimental results (3) in Experiment Example 1 (tumor cells exhibit resistance to immune checkpoint inhibition through CS production). Figure 4A is a series of viSNE plots showing the distribution of tumor-infiltrating bone marrow cells in E0771-SULT cells and E0771-control cells. Figure 4B is a pair of graphs for comparing proportions of PD-$L1^+$ cells in infiltrating leukocytes ($CD45^+$ cells) or $CD11b^+$ cells for E0771-SULT and those for E0771-control (n = 9). The data are expressed as mean ± SD. Figure 4C and Figure 4D are graphs for comparing in vivo growths of E0771-control-PD-L1 cells (Figure 4C, n = 9) and E0771-SULT-PD-L1 cells (Figure 4D, n = 7) each transplanted into mice in a group treated with an anti-PD-L1 antibody (Anti-PD-L1) and those in a group treated with a control of the matched isotype (Isotype). The data are expressed as mean ± SD. * $P < 0.05$, ** $P < 0.01$ (two-sided Mann-Whitney test).

[Figure 5] Figure 5 is a series of diagrams showing experimental results (4) in Experiment Example 1 (SULT2B1b-mediated CS production in tumors weakens anti-tumor T-cell response). Figure 5A is a schematic diagram illustrating the interaction between OTII $CD4^+$ T cells (OTII TCR) and tumor cells expressing an I-$A^b$ molecule covalently bound to an OVA peptide (I-$A^b$/OVA). Figure 5B and Figure 5C show results of comparison of the effect of OTII $CD4^+$ T cell transplantation on tumor growth in C57BL/6 mice. Figure 5B shows results of comparison between E0771-control-OVA (n = 8) and E0771-SULT-OVA (n = 7), and Figure 5C shows results of comparison between 3LL-control-OVA (n = 8) and 3LL-SULT-OVA (n = 8). Activated $V\alpha 2^+V\beta 5^+$OTII T cells were intravenously injected on day 9, and the infiltration of OTII T cells was then analyzed on day 14 (B: n = 9; C: n = 7). The data are expressed as mean ± SD. * $P < 0.05$, ** $P < 0.01$ (unpaired Student t test for two groups for tumor growth, and two-sided Mann-Whitney test for the infiltration of OTII T cells). Figure 5D shows results of comparison of the effect of OTI $CD8^+$ T cell transplantation on tumor growth in C57BL/6 mice for E.G7-OVA (n = 8) and that for E.G7-OVA-SULT (n = 8). Activated $V\alpha 2^+V\beta 5^+$OTI T cells were intravenously injected on day 10, and the infiltration of OTI T cells was then analyzed on day 15 (n = 7 or 8). The data are expressed as mean ± SD. * $P < 0.05$ (unpaired Student t test for two groups for both tumor growth and the infiltration of OTI T cells) .

[Figure 6] Figure 6 is a series of diagrams showing experimental results (5) in Experiment Example 1 (expression of SULT2B1b suppresses the in vivo growth of MC38 cells that produce 25-HC). Figure 6A is a series of graphs showing expression of Ch25h, Cyp27a1, and Cyp11a1 in tumor cell lines (EO771, 3LL, E.G7-OVA, MC38). Reverse transcription quantitative PCR found high expression of Ch25h in MC38. The data (n = 5 to 7) are expressed as mean ± SD after normalization to expression of Gapdh. ** $P < 0.01$ (one-way analysis of variance followed by Dunnett's post-hoc test). Figure 6B is a graph for comparing tumor growth after the transplantation into C57BL/6 mice for MC38-SULT and that for MC38-control (n = 10). The data are expressed as mean ± SD. ** $P < 0.01$ (two-sided Mann-Whitney test). Figure 6C is a series of graphs showing productions of CS, 25-HC, and 25-HCS in MC38-control cells, MC38-SULT cells, MC38-ΔCH25H cells, and MC38-ΔCH25H-SULT cells (n = 5 for each) as quantified by mass spectrometry. The data are expressed as mean ± SD. ** $P < 0.01$ (one-way analysis of variance followed by Dunnett's post-hoc test). Figure 6D is a graph for comparing the in vitro growth of MC38 cells in the presence of culture supernatant (Sup) of wild-type MC38 (MC38-control), MC38-SULT, or MC38-ΔCH25H (n = 6 for each) . The data are expressed as mean ± SD. ** $P < 0.01$ (one-way analysis of variance followed by Dunnett's post-hoc test). Figure 6E is a graph for comparing tumor growth after transplantation of cancer cells (MC38-ΔCH25H cells, MC38-ΔCH25H-SULT cells) into C57BL/6 mice for MC38-ΔCH25H cells (n = 8) and that for MC38-ΔCH25H-SULT cells (n = 9). The data are expressed as mean ± SD. ** $P < 0.01$ (unpaired Student t test for two groups).

[Figure 7] Figure 7 is a series of graphs showing expression levels of genes encoding oxysterol hydroxylase (CH25H, CYP25A1, CYP11A1) in 17 types of human cancer cells. The data were obtained from Human Protein Atlas (https://www.proteinatlas.org), and expressed as median values of FPKM values.

[Figure 8] Figure 8 is a series of diagrams showing experimental results (6) in Experiment Example 1 (identification of cholenoic acid as a SULT2B1b inhibitor that promotes anti-tumor immunity). Figure 8A shows the structure of 3β-hydroxy-Δ5-cholenoic acid (cholenoic acid). Figure 8B shows CS production levels in culture supernatant resulting from treating E0771-SULT cells with cholenoic acid (the dose response of cholenoic acid to suppress CS production from E0771-SULT cells). This demonstrates that the CS production by E0771-SULT cells is inhibited by treatment with cholenoic acid. Figure 8C shows that oral administration of cholenoic acid (cholenoic acid: +) on days 5, 6, and 7 after transplanting E0771-SULT cells into mice (SULT:+) caused the CS production to be locally inhibited, and $CD8^+$ T cell infiltration was induced in a low CS region (CS low) on day 8. E0771-control cells (SULT:-) were used as a control. The data (n = 3 to 5) are expressed as mean ± SD. ** $P < 0.01$ (one-way analysis of variance followed by Dunnett's post-hoc test). Figure 8D shows that oral administration of cholenoic acid causes the growth of E0771-SULT-PD-L1 cells (tumor volume) to be suppressed by treating with an anti-PD-L1 antibody. The cholenoic acid (▼) and anti-PD-L1 antibody (4<) were administered on days 9, 12, and 15 after transplantation of E0771-SULT-PD-L1 cells into mice. The data (n = 8) are expressed as mean ± SD. * $P < 0.05$, ** $P < 0.01$ (unpaired Student t test for two groups) . In Figure 8E, the left graph shows that when activated OTII T cells were intravenously injected on day 9 (4<) after E0771-SULT-OVA cells were transplanted into mice, and cholenoic acid was then orally administered on days 12, 13, and 14 (▼), the anti-tumor immunity of OTII T cells was promoted and the growth of E0771-SULT-OVA cells was suppressed. The right figure shows results of analysis of the infiltration of OTII T cells on day 14 after the injection. The data (left; n = 8, right: n = 9 or 10) are expressed as mean ± SD. * $P < 0.05$ (two-sided Mann-Whitney test for tumor growth, unpaired Student t test for two groups for the infiltration of OTII T cells). In Figure 8F, the left figure shows that when activated OTI T cells were intravenously injected on day 10 after transplantation of E.G7-OVA-SULT cells into mice (4<), and cholenoic acid was then orally administered on days 13, 14, and 15 (▼solid inverted triangle), the anti-tumor immunity of OTI T cells was promoted and the growth of E.G7-OVA-SULT cells was suppressed. The right figure shows results of analysis of the infiltration of OTII T cells on day 15 after the transplantation. The data (left: n = 8, right: n = 11 or 12) are expressed as mean ± SD. * $P < 0.05$ (unpaired Student t test for two groups for tumor growth, two-sided Mann-Whitney test for the infiltration of OTII T cells).

[Figure 9] Figure 9 shows representative images of CS production and $CD8^+$ T cell infiltration in E0771-SULT cells after oral administration of cholenoic acid (lowermost images) (see experimental results (6) in Experiment Example 1). It was found that when cholenoic acid was orally administered on days 5, 6, and 7 after transplantation of E0771-SULT cells into mice, the CS production was locally inhibited, and $CD8^+$ T cell infiltration was induced in a low CS region (CS low region) on day 8. The right column (CD8 (enlarged)) shows high-magnification images of the areas each surrounded by a rectangle.

[Figure 10] Figure 10 is a series of diagrams showing experimental results (6) in Experiment Example 1 (identification of cholenoic acid as a SULT2B1b inhibitor that promotes anti-tumor immunity). Figure 10A shows results of trans-cancer migration assay, the results demonstrating inhibition of the CCL21-induced T-cell migration by E0771-SULT cells (SULT+) and recovery thereof through cholenoic acid (in the figure, shown as "3β-OH-5-Chln") (5 μM) treatment. The data were acquired from two independent experiments, and expressed as mean ± SD. * $P < 0.05$; ** $P < 0.01$ (one-way ANOVA followed by Dunnett's post-hoc test). The level of CCL21-induced T-cell migration in E0771-MOCK cells (CCL21+, SULT-) was used as a control for comparison. Figures 10B and 10C show results of enzyme kinetic analysis for SULT2B1b inhibition by cholenoic acid (3-OH-5-Chln). SULT2B1b protein (0.3 or 0.6 μM) was preincubated with cholenoic acid (20 or 40 μM) or a vehicle (2% DMSO), and reacted with cholesterol at different concentrations in the presence of 100 μM PAPS (Figure 10B) or reacted with PAPS at different concentrations in the presence of 10 μM cholesterol (Figure 10C). The data were fitted to the Michaelis-Menten equation (the left figures in Figures 10B and 10C), and their inhibition modes were analyzed with Lineweaver-Burk plots (right figures). The results showed that $R^2$ and the Km value in Figure 10B were 0.95 to 0.98 and 0.82, respectively, and $R^2$ and the Km value in Figure 10C were 0.98 and 0.89, respectively.

Description of Embodiments

(I) SULT2B1b inhibitor and CS production inhibitor

The SULT2B1b inhibitor and CS production inhibitor of the present invention are both characterized by containing a compound or a salt thereof as an active ingredient, the compound represented by general formula (I) (compound I):

[Formula 12]

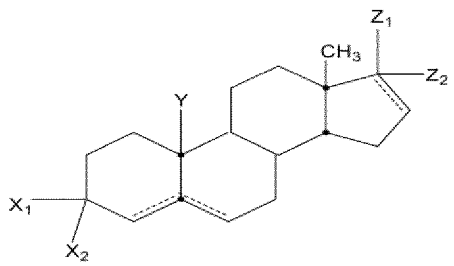

(I)

In formula (I), one of $X_1$ and $X_2$ is a hydroxy group, a morpholino $C_{1-6}$ alkylcarbonyloxy group, a $C_{1-6}$ alkylcarbonyloxy group, a phosphate group, a $C_{1-6}$ alkoxy group, a di ($C_{1-6}$ alkyl)amino $C_{1-6}$ alkyltriazolyl group, an acetoxy group in which at least one hydrogen atom is optionally replaced with a halogen atom, an azide group, or a sulfate group. The other (i.e., $X_2$ if the one is $X_1$, $X_1$ if the one is $X_2$) is a hydrogen atom. Alternatively, $X_1$ and $X_2$ may be combined to form an oxo group. Preferably, one of $X_1$ and $X_2$ is a hydroxy group, a trihalogenoacetoxy group, or a morpholino $C_{1-6}$ alkylcarbonyloxy group.

In the morpholino $C_{1-6}$ alkylcarbonyloxy group, $C_{1-6}$ alkylcarbonyloxy group, $C_{1-6}$ alkoxy group ($C_{1-6}$ alkyloxy group), and di($C_{1-6}$ alkyl)amino $C_{1-6}$ alkyltriazolyl group among those groups, each "alkyl group" used as a prefix or infix may be linear or branched. Each "alkyl group" is preferably a linear or branched alkyl group having one to four carbon atoms. Examples of such alkyl groups include a methyl group, an ethyl group, an n-propyl group, an isopropyl group, an n-butyl group, an isobutyl group, an n-pentyl group, and an n-hexyl group. Each alkyl group is more preferably a methyl group or ethyl group, each having one or two carbon atoms, and even more preferably a methyl group. Examples of the di ($C_{1-6}$ alkyl) amino $C_{1-6}$ alkyltriazolyl group include a group having a 1,2,3-triazole skeleton and a group having a 1,2,4-triazole skeleton. The di ($C_{1-6}$ alkyl) amino $C_{1-6}$ alkyltriazolyl group is preferably a group having a 1,2,3-triazole

skeleton, and more preferably a group in which a di($C_{1-6}$ alkyl)amino $C_{1-6}$ alkyl group is bonded to position 4 of a 1,2,3-triazole skeleton.

Examples of the halogen atom in the acetoxy group in which at least one hydrogen atom is optionally replaced with a halogen atom, wherein the acetoxy group is represented by $X_1$ or $X_2$, include fluorine, chlorine, bromine, and iodine. The halogen atom is preferably fluorine. Preferred examples of the acetoxy group in which at least one hydrogen atom is optionally replaced with a halogen atom include an acetoxy group and a trihalogenoacetoxy group (e.g., a trifluoroacetoxy group, a trichloroacetoxy group). The acetoxy group in which at least one hydrogen atom is optionally replaced with a halogen atom is more preferably a trihalogenoacetoxy group, and particularly preferably a trifluoroacetoxy group.

In formula (I), Y is a $C_{1-6}$ alkyl group or a hydrogen atom. Y is preferably a $C_{1-6}$ alkyl group. Examples of the $C_{1-6}$ alkyl group include the aforementioned alkyl groups, and the $C_{1-6}$ alkyl group is more preferably a methyl group or ethyl group, each having one or two carbon atoms, and even more preferably a methyl group.

In formula (I), $Z_1$ and $Z_2$ are different and each represent a group represented by formula (II):

[Formula 13]

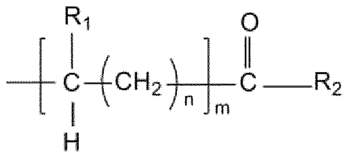

(II)

, a hydroxy group, an ethynyl group, a $C_{1-6}$ alkylcarbonyloxy group, a hydrogen atom, or a heterocyclic group optionally having a substituent. Alternatively, $Z_1$ and $Z_2$ may be combined to form an oxo group.

Examples of the "alkyl group" used as a prefix in the $C_{1-6}$ alkylcarbonyloxy group among those groups include the aforementioned groups. The alkyl group is more preferably a methyl group or ethyl group, each having one or two carbon atoms, and even more preferably a methyl group.

The heterocyclic group is saturated or partially unsaturated and monocyclic with three to six ring-forming atoms. One, two, or three of the ring-forming atoms can be each a heteroatom of a sulfur atom, a nitrogen atom, or an oxygen atom, and bonded to carbon atoms to form a ring. Each of the heteroatoms is preferably a sulfur atom. The heterocyclic group is more preferably a saturated monocyclic group with three ring-forming atoms one of which is a sulfur atom. The heterocyclic group may have one or more substituents in an arbitrary manner. Examples of the substituents include, but are not limited to, an alkyl group, an amino group, a halogen, a cyano group, a nitro group, a carbonyl(oxo) group, and a hydroxy group. Each of the substituents is preferably a $C_{1-6}$ alkyl group, more preferably a methyl group or ethyl group, each having one or two carbon atoms, and even more preferably a methyl group.

In formula (II), $R_1$ represents a $C_{1-6}$ alkyl group or a hydrogen atom. $R_1$ is preferably a $C_{1-6}$ alkyl group. Examples of the "alkyl group" include the aforementioned groups. $R_1$ is more preferably a methyl group or ethyl group, each having one or two carbon atoms, and even more preferably a methyl group.

In formula (II), $R_2$ is a hydroxy group, a $C_{1-6}$ alkyl group, a $C_{1-6}$ alkoxy group, a group represented by formula (III):

[Formula 14]

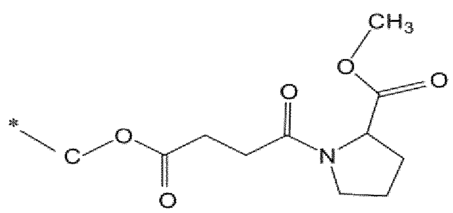

(III)

wherein * indicates a bond to the carbon atom of the carbonyl group in formula (II), or $-N(R_3)(R_4)$. $R_2$ is preferably a hydroxy group or a $C_{1-6}$ alkyl group.

Examples of the "alkyl groups" used as a prefix in the $C_{1-6}$ alkyl group and $C_{1-6}$ alkoxy group among those groups include the aforementioned groups. Each alkyl group is more preferably a methyl group or ethyl group, each having one or two carbon atoms, and even more preferably a methyl group.

In the $-N(R_3)(R_4)$, $R_3$ and $R_4$ are the same or different, each being a hydrogen atom, $C_{1-6}$ alkyl group, a $C_{1-6}$ alkylphenyl group, or a group represented by formula (IV):

[Formula 15]

$$-\left[\left(CH_2\right)_2-O\right]_p-CH_2C\equiv CH \qquad (IV)$$

wherein p denotes an integer of 1 to 10. p is preferably 1 to 8, more preferably 1 to 6, and even more preferably 2 to 6. If one of $R_3$ and $R_4$ is the group represented by formula (IV), the other is preferably a hydrogen atom.

In the -N($R_3$)($R_4$), $R_3$ and $R_4$ may be combined to form a heterocyclic ring together with the nitrogen atom.

Examples of the "alkyl groups" used as a suffix in the $C_{1-6}$ alkyl group and phenyl $C_{1-6}$ alkyl group include the aforementioned groups. Each alkyl group is more preferably a methyl group or ethyl group, each having one or two carbon atoms, and even more preferably a methyl group.

Here, the heterocyclic group is a saturated monocyclic group with six ring-forming atoms. Of the ring-forming atoms, the atoms other than the nitrogen atom (N) shown in -N($R_3$)($R_4$) can be each a carbon atom, a sulfur atom, an oxygen atom, or a nitrogen atom, and the atoms are bonded together to form a monocyclic ring. Preferred examples of the heterocyclic group can include morpholine and thiomorpholine.

In formula (II), m is 1 or 0. m is preferably 1.

Preferred examples of $R_2$ if m is 0 include, but are not limited to, a $C_{1-6}$ alkyl group and the group represented by formula (III) .

Preferred examples of $R_2$ if m is 1 include, but are not limited to, a hydroxy group, a $C_{1-6}$ alkyl group, a $C_{1-6}$ alkoxy group, and -N($R_3$)($R_4$).

In formula (II), n is an integer of 0 to 3. n is preferably 2.

In the steroid skeleton represented by formula (I) (cyclopentanoperhydrophenanthrene core), the carbon atoms at positions 4 and 5, the carbon atoms at positions 5 and 6, and/or the carbon atoms at positions 16 and 17 may be forming a double bond.

Preferred examples of combinations of $Z_1$ and $Z_2$ in formula (I) include, but are not limited to: a combination of a hydrogen atom and a group other than a hydrogen atom (particularly preferably, a group represented by formula (II), a heterocyclic group optionally having a substituent); a combination of a $C_{1-6}$ alkylcarbonyloxy group and a group represented by formula (II) or an ethynyl group; a combination of an ethynyl group and a hydroxy group; and a combination of a heterocyclic group optionally having a substituent and a hydrogen atom.

Preferred compounds as compound I include the following compounds:

(i) compound A

A compound represented by general formula (I), wherein one of $Z_1$ and $Z_2$ is a group represented by formula (II), and the other is a hydrogen atom or a $C_{1-6}$ alkylcarbonyloxy group; one of $X_1$ and $X_2$ is a hydroxy group, a morpholino $C_{1-6}$ alkylcarbonyloxy group, a $C_{1-6}$ alkylcarbonyloxy group, a phosphate group, a $C_{1-6}$ alkoxy group, an acetoxy group in which at least one hydrogen atom is optionally replaced with a halogen atom, a di($C_{1-6}$ alkyl)amino $C_{1-6}$ alkyltriazolyl group, an azide group, or a sulfate group, and the other is a hydrogen atom; and Y is a $C_{1-6}$ alkyl group.

Particularly preferred compounds as such compound A include the following compounds:
wherein the compound names listed below are names of compounds based on the IUPAC nomenclature determined by using the software ChemDraw Professional 15.0,

compound 1: (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanoic acid (CAS No. 5255-17-4) (alias: 3β-hydroxy-$\Delta^5$-cholenoic acid) (herein, also referred to as "cholenoic acid") (C-2650, Sigma-Aldrich Co. LLC)

compound 2: (R)-4-((3S,8S,9S,10R,13R,14S,17R)-10,13-dimethyl-3-(2,2,2-trifluoroacetoxy)-2,3,4,7,8,9,10,11,12,13,14,15,16,17-tetradecahydro-1H-cyclopenta[a]phenanthren-17-yl)pentanoic acid (Production Example 1)

compound 3: methyl (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanoate (Production Example 2)

compound 4: methyl (R)-4-((3S,8S,9S,10R,13R,14S,17R)-10,13-dimethyl-3-(2,2,2-trifluoroacetoxy)-2,3,4,7,8,9,10,11,12,13,14,15,16,17-tetradecahydro-1H-cyclopenta[a]phenanthren-17-yl)pentanoate (Production Example 3)

compound 5: methyl (R)-4-((3S,8S,9S,10R,13R,14S,17R)-3-methoxy-10,13-dimethyl-2,3,4,7,8,9,10,11,12,13,14,15,16,17-tetradecahydro-1H-cyclopenta[a]phenanthren-17-yl)pentanoate) (Production

Example 4)

compound 6: (R)-4-((3S,8S,9S,10R,13R,14S,17R)-3-methoxy-10,13-dimethyl-2,3,4,7,8,9,10,11,12,13,14,15,16,17-tetradecahydro-1H-cyclopenta[a]phenanthren-17-yl)pentanoic acid (Production Example 5)

compound 7: (3S,8S,9S,10R,13R,14S,17R)-17-((R)-5-(dibenzylamino)-5-oxopentan-2-yl)-10,13-dimethyl-2,3,4,7,8,9,10,11,12,13,14,15,16,17-tetradecahydro-1H-cyclopenta[a]phenanthren-3-yl 2,2,2-trifluoroacetate (Production Example 6)

compound 8: (3S,SS,9S,10R,13R,14S,17R)-17-((R)-5-(butylamino)-5-oxopentan-2-yl)-10,13-dimethyl-2,3,4,7,8,9,10,11,12,13,14,15,16,17-tetradecahydro-1H-cyclopenta[a]phenanthren-3-yl 2,2,2-trifluoroacetate (Production Example 7)

compound 9: (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-1-thiomorpholinopentan-1-one (Production Example 8)

compound 10: (3S,8S,9S,10R,13R,14S,17R)-10,13-dimethyl-17-((R)-5-oxo-5-thiomorpholinopentan-2-yl)-2,3,4,7,8,9,10,11,12,13,14,15,16,17-tetradecahydro-1H-cyclopenta[a]phenanthren-3-yl dihydrogen phosphate (Production Example 9)

compound 11: (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-1-morpholinopentan-1-one (Production Example 10)

compound 12: (R)-4-((3R,8S,9S,10R,13R,14S,17R)-3-azido-10,13-dimethyl-2,3,4,7,8,9,10,11,12,13,14,15,16,17-tetradecahydro-1H-cyclopenta[a]phenanthren-17-yl)-1-morpholinopentan-1-one (Production Example 11)

compound 13: (R)-4-((3R,8S,9S,10R,13R,14S,17R)-3-(4-((dimethylamino)methyl)-1H-1,2,3-triazol-1-yl)-10,13-dimethyl-2,3,4,7,8,9,10,11,12,13,14,15,16,17-tetradecahydro-1H-cyclopenta[a]phenanthren-17-yl)-1-morpholinopentan-1-one (Production Example 12)

compound 14: (4R)-4-[(3R,5R,8R,9S,10S,13R,14S,17R)-3-hydroxy-10,13-dimethyl-2,3,4,5,6,7,8,9,11,12,14,15,16,17-tetradecahydro-1H-cyclopenta[a]phenanthren-17-yl]pentanoic acid ((4R)-4-[(3R,5R,8R,9S,10S,13R,14S,17R)-3-hydroxy-10,13-dimethyl-2,3,4,5,6,7,8,9,11,12,14,15,16,17-tetradecahydro-1H-cyclopenta[a]phenanthren-17-yl]pentanoic acid) (alias: 3$\alpha$-hydroxy-5$\beta$-colanic acid (3$\alpha$-hydroxy-5$\beta$-cholanic acid)) (CAS RN. 434-13-9) (commercially available product: obtained from Namiki Shoji Co., Ltd.)

compound 15: (8R,9S,10R,13S,14S)-17-acetyl-10,13-dimethyl-2,3,4,7,8,9,10,11,12,13,14,15-dodecahydro-1H-cyclopenta[a]phenanthren-3-yl3-morpholinopropanoate (CAS No. 1038743-27-9) (commercially available product: obtained from Namiki Shoji Co., Ltd.)

compound 16: (3R,5S,SR,9S,10S,13S,14S)-17-acetyl-10,13-dimethyl-2,3,4,5,6,7,8,9,10,11,12,13,14,15-tetradecahydro-1H-cyclopenta[a]phenanthren-3-yl3-morpholinopropanoate (CAS RN. 1029693-41-1) (commercially available product: obtained from Namiki Shoji Co., Ltd.)

compound 17: (alias) 3-acetoxy-5-cholenoic acid (CAS RN. 19462-13-6) (commercially available product: Sigma-Aldrich Co. LLC, production No. S9614412)

compound 18: methyl (4-(2-8((8R,9S,10R,13S,14S,17R)-17-acetoxy-10,13-dimethyl-3-oxo-2,3,6,7,8,9,10,11,12,13,14,15,16,17-tetradecahydro-1H-cyclopenta[a]phenanthren-17-yl)-2-oxoethoxy)-4-oxobutanoyl)-L-prolinate (CAS RN. 1029696-09-0) (commercially available product: obtained from Kishida Chemical Co., Ltd.)

compound 19: (alias) methyl (3$\beta$,5$\alpha$)-3-hydroxycholan-24-oate (commercially available product: obtained from Sigma-Aldrich Co. LLC)

compound 20: (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-N-(2-(prop-2-yn-1-yloxy)ethyl)pentanamide (Production Example 13)

compound 21: (3S,SS,9S,10R,13R,14S,17R)-10,13-dimethyl-17-((R)-5-oxo-5-((2-(prop-2-yn-1-yloxy)ethyl)amino)pentan-2-yl)-2,3,4,7,8,9,10,11,12,13,14,15,16,17-tetradecahydro-1H-cyclopenta[a]phenanthren-3-yl sulfate or a tetrabutylammonium salt thereof (Production Example 14)

compound 22:(R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-N-(2-(2-(prop-2-yn-1-yloxy)ethoxy)ethyl)pentanamide (Production Example 15)

compound 23: (3S,8S,9S,10R,13R,14S,17R)-10,13-dimethyl-17-((R)-5-oxo-5-((2-(2-(prop-2-yn-1-yloxy)ethoxy)ethyl)amino)pentan-2-yl)-2,3,4,7,8,9,10,11,12,13,14,15,16,17-tetradecahydro-1H-cyclopenta[a]phenanthren-3-yl sulfate or a tetrabutylammonium salt thereof (Production Example 16)

compound 24:(R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-N-(3,6,9,12-tetraoxapentadec-14-yn-1-yl)pentanamide (Production Example 17)

compound 25: (3S,8S,9S,10R,13R,14S,17R)-10,13-dimethyl-17-((R)-17-oxo-4,7,10,13-tetraoxa-16-azahenicos-1-yn-20-yl)-2,3,4,7,8,9,10,11,12,13,14,15,16,17-tetradecahydro-1H-cyclopenta[a]phenanthren-3-yl sulfate or a tetrabutylammonium salt thereof (Production Example 18)

compound 26: (R)-N-(3,6,9,12,15,18-hexaoxahenicos-20-yn-1-yl)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanamide (Production Example 19)

compound 27: (3S,8S,9S,10R,13R,14S,17R)-10,13-dimethyl-17-((R)-23-oxa-4,7,10,13,16,19-hexaoxa-22-azaheptacos-1-yn-26-yl)-2,3,4,7,8,9,10,11,12,13,14,15,16,17-tetradecahydro-1H-cyclopenta[a]phenanthren-3-yl sulfate or a tetrabutylammonium salt thereof (Production Example 20)

(ii) Compound B

A compound represented by general formula (I), wherein $Z_1$ and $Z_2$ are combined to form an oxo group; one of $X_1$ and $X_2$ is a morpholino $C_{1-6}$ alkylcarbonyloxy group, and the other is a hydrogen atom; and Y is a $C_{1-6}$ alkyl group.

Particularly preferred compounds as such compound B include the following compound:

compound 28: (8R, 9S, 10R, 13S, 14S)-10,13-dimethyl-17-oxo-2,3,4,7,8,9,10,11,12,13,14,15,16,17-tetradecahydro-1H-cyclopenta[a]phenanthren-3-yl 3-morpholinopropanoate (CAS RN. 447458-03-9) (commercially available product: obtained from Namiki Shoji Co., Ltd.)

(iii) Compound C

A compound represented by general formula (I), wherein one of $Z_1$ and $Z_2$ is an ethynyl group, and the other is a hydroxy group or a $C_{1-6}$ alkylcarbonyloxy group; one of $X_1$ and $X_2$ is a hydroxy group, and the other is a hydrogen atom, or $X_1$ and $X_2$ are combined to form an oxo group; and Y is a $C_{1-6}$ alkyl group or a hydrogen atom.

Particularly preferred compounds as such compound C include the following compounds:

compound 29: (3S,8R,9S,10R,13S,14S,17R)-17-ethynyl-10,13-dimethyl-2,3,4,7,8,9,10,11,12,13,14,15,16,17-tetradecahydro-1H-cyclopenta[a]phenanthrene-3,17-diol (CAS RN. 3604-60-2) (commercially available product: obtained from Kishida Chemical Co., Ltd.)

compound 30: (alias) 19-norethisterone-acetate (commercially available product: obtained from Sigma-Aldrich Co. LLC) (CAS RN. 51-98-9)

compound 31: (alias) 19-norethindrone (CAS RN. 68-22-4) (commercially available product: obtained from Sigma-Aldrich Co. LLC)

(iv) Compound D

A compound represented by general formula (I), wherein one of $Z_1$ and $Z_2$ is a heterocyclic group optionally having a substituent, and the other is a hydrogen atom; one of $X_1$ and $X_2$ is a hydroxy group, and the other is a hydrogen atom; and Y is a $C_{1-6}$ alkyl group.

Particularly preferred compounds as such compound D include the following compound:

compound 32: (3S,8S,9S,10R,13S,14S)-10,13-dimethyl-17-(2-methylthiiran-2-yl)-2,3,4,7,8,9,10,11,12,13,14,15,16,17-tetradecahydro-1H-cyclopenta[a]phenanthren-3-ol (CAS RN. 1047632-27-8) (commercially available product: obtained from Namiki Shoji Co., Ltd.)

Compound I described above can be in the form of a salt, and the salt can also be used like compound I in the free form. If compound I has an alkaline center, for example, the alkaline center can form an acid addition salt. If compound I has an acidic center, the acidic center can form an alkali addition salt. Furthermore, if compound I has both an acidic center (e.g., carboxyl) and an alkaline center (e.g., an amino group), the acidic and alkaline centers can form an intramolecular salt.

Examples of the acid addition salt include, but are not limited to, a hydrochloride, hydrofluoride, hydrobromide, hydroiodide, sulfate, pyrosulfate, phosphate, nitrate, methanesulfonate, ethanesulfonate, 2-hydroxyethanesulfonate, benzenesulfonate, toluenesulfonate, sulfamate, 2-naphthalenesulfonate, formate, acetoacetate, pyruvate, laurate, cinnamate, benzoate, acetate, glycolate, trifluoroacetate, trimethylacetate, propionate, butyrate, hexanoate, heptanoate, undecanoate, stearate, ascorbate, camphorate, camphor sulfonate, citrate, fumarate, malate, maleate, hydroxymaleate, oxalate, salicylate, succinate, gluconate, quininate, pamoate hydrate, tartrate, lactate, 2-(4-hydroxybenzoyl)benzoate, cyclopentanepropanoate, digluconate, 3-hydroxy-2-naphthoate, nicotinate, pamoate, pectate, 3-phenylpropionate, picrate, pivalate, itaconate, trifluoromethanesulfonate, dodecylsulfate, p-toluenesulfonate, naphthalenedisulfonate, malonate, adipate, arginate, mandelate, glucoheptonate, glycerophosphate, sulfosalicylate, hemisulfate or thiocyanate, and aspartate. The acid addition salt is preferably a pharmaceutically acceptable acid addition salt.

Examples of the alkali addition salt include, but are not limited to, alkali metal salts, alkaline earth metal salts,

and ammonium salts, specifically, a sodium salts, lithium salt, potassium salts, ammonium salt, aluminum salt, magnesium salt, calcium salt, barium salt, iron salt, ferrous salt, manganese salt, manganous salt, and zinc salt, and ammonium salts (a salt formed from $NH_3$ ($NH_4$ salt) and those from an organic amine, including a methylammonium salt, trimethylammonium salt, diethylammonium salt, triethylammonium salt, propylammonium salt, tripropylammonium salt, isopropylammonium salt, tert-butylammonium salt, tetrabutylammonium salt, N,N'-dibenzylethylenediammonium salt, dicyclohexylammonium salt, 1,6-hexanediammonium salt, benzylammonium salt, ethanolammonium salt, N,N-dimethylethanolammonium salt, N,N-diethylethanolammonium salt, triethanolammonium salt, tromethamine salt, lysine salt, arginine salt, histidine salt, glucosamine salt, N-methylglucosamine salt, dimethylglucosamine salt, ethylglucosamine salt, meglumine salt, betaine salt, caffeine salt, chloroprocaine salt, procaine salt, lidocaine salt, pyridine salt, methylpyridine salt, piperidine salt, morpholine salt, piperazine salt, purine salt, theobromine salt, and choline salt). The alkali addition salt is preferably a pharmaceutically acceptable alkali addition salt.

If there exists isomers such as stereoisomers and optical isomers for compound I, those isomers are also encompassed as a mode of compound I of the present invention. Compound I may be a mixture containing different isomers at any ratio. Each isomer may be present in the form of a tautomer. The definition of compound I includes all the possible tautomers, and compound I may be a single tautomer or any mixture containing different tautomers at any ratio. In addition, compound I can be in the form of solvate (including hydrate), and the solvate is also encompassed as a mode of compound I of the present invention.

Commercially available products are applicable as compound I or a salt thereof. For example, compound 1 (3β-hydroxy-Δ5-cholenoic acid) mentioned above is a known compound, and can be purchased from, for example, FUJIFILM Wako Pure Chemical Corporation, Tokyo Chemical Industry Co., Ltd., and Sigma-Aldrich Japan. Compound 14 (3α-hydroxy-5β-colanic acid: CAS RN. 434-13-9) is also a known compound, and can be purchased from those companies.

Alternatively, compound I or a salt thereof can be produced from a commercially available compound as a raw material, for example, with reference to a description shown later in Production Examples. Compound I can be isolated and/or purified by means of a known isolation and/or purification technique from a reaction mixture obtained through any of those production methods. Examples of such isolation or purification techniques can include distillation, recrystallization, solvent extraction, column chromatography, ion-exchange chromatography, gel chromatography, affinity chromatography, and preparative thin-layer chromatography.

Compound I or a salt thereof has an effect to inhibit the catalytic function of sulfotransferase (SULT2B1b), which has an effect to add a sulfate group to the hydroxy group at position 3 of cholesterol. Accordingly, compound I or a salt thereof is effective as an SULT2B1b inhibitor. "SULT2B1b inhibitors" of interest of the present invention include not only ones having an effect to completely eliminate the catalytic function (enzymatic activity) of SULT2B1b to cause sulfation of cholesterol, but also ones having an effect to reduce the catalytic function (enzymatic activity) to a level below the original. In addition, "SULT2B1b inhibitors" of interest of the present invention preferably include ones that exhibit an SULT2B1b inhibitory effect not only in vitro but also in vivo. Each SULT2B1b inhibitor may consist of compound I or a salt thereof, or be in the form of a composition containing an additional component such as an excipient that is commonly used in the field (e.g., the field of biochemistry, the field of medicine), unless the SULT2B1b inhibitory effect is deteriorated. Examples of such excipients can include, but are not limited to, components necessary for formulation such as binders and diluents, and components effective for stabilization of enzymes such as buffers.

In cells and tissues in which SULT2B1b is present, cholesterol in the body is sulfated by the catalysis of SULT2B1b to generate cholesterol-3-sulfate (CS). Hence, compound I or a salt thereof, which exerts an SULT2B1b inhibitory effect in vivo, can inhibit the generation of CS by inhibiting the sulfation of cholesterol in the body. From this reason, compound I or a salt thereof is effective as a CS production inhibitor, in particular, as an in vivo CS production inhibitor. "CS production inhibitors" of interest of the present invention include not only ones having an effect to completely inhibit the production of CS, but also ones having an effect to reduce the amount of CS to be generated to a level below the original. Each CS production inhibitor may consist of compound I or a salt thereof, or be in the form of a composition containing an additional component such as an excipient that is commonly used in the field (e.g., the field of biochemistry, the field of medicine), unless the CS production inhibitory effect is deteriorated. Examples of such excipients can include, but are not limited to, components necessary for formulation such as binders and diluents, and components effective for stabilization of enzymes such as buffers.

(II) Pharmaceutical composition

The pharmaceutical composition according the present invention contains compound I or a pharmaceutically acceptable salt thereof. For the pharmaceutically acceptable salt, appropriate one can be selected with reference to those described in detail in section (I).

The pharmaceutical composition according to the present invention may consist only of compound I or a pharmaceutically acceptable salt thereof, or be a pharmaceutical composition prepared by a known method in combination with any carrier or excipient into a form suitable for a desired application such as a route of administration and an administration method. The pharmaceutical composition is one in a form suitable preferably for intravenous administration or oral administration, more preferably for oral administration.

Examples of the specific dosage form include a tablet, a pill, a powder, a solution, a suspension, an emulsion, a granule, a capsule, a suppository, an injection (e.g., a solution, a suspension), and a drip infusion. The dosage form is preferably one suitable for oral administration such as a tablet, a pill, a powder, a solution, a suspension, an emulsion, a granule, or a capsule.

The amount of compound I or a pharmaceutically acceptable salt thereof to be blended in the pharmaceutical composition according to the present invention is not limited, as long as a CS production inhibitory effect is exerted by compound I or a salt thereof. For example, the amount can be appropriately adjusted within the range of about 0.001 to 99% by mass or less, preferably of about 0.01 to 50% by mass, more preferably of about 0.05 to 10% by mass to 100% by mass of the pharmaceutical composition.

As described in "Background of Invention", DOCK2 plays an important role in the migration and activation of lymphocytes in vivo in mammals such as humans and mice, thus being an endogenous molecule essential for the immune surveillance in mammals. On the other hand, CS is known to be an endogenous DOCK2 inhibitor that directly binds to a catalytic DHR-2 domain of DOCK2 and exerts an effect to inhibit the Rac GEF activity (NPL 2). In addition, CS is almost specifically found in cancerous tissues in prostate cancer, and hence the possibility that CS plays a key role in the immune evasion of tumors has been pointed out (NPL 4). In view of those, a method has been proposed in which immune response in cancer tissues is promoted (activation of cancer immunity) by cancelling immune privilege formed in cancer tissues by inhibiting the expression and production of SULT2B1b in cancer tissues to suppress the CS production (PTL 1).

Thus, the pharmaceutical composition according to the present invention exerts an effect to promote immune response in cancer tissues (activation of cancer immunity) by cancelling immune privilege formed in a cancer tissue and/or regions therearound through the SULT2B1b inhibitory effect of compound I or a pharmaceutically acceptable salt thereof as an active ingredient or the CS production inhibitory effect based on the SULT2B1b inhibitory effect. It follows that the pharmaceutical composition of the present invention is effective as an anti-cancer immunostimulator (cancer immunotherapy potentiator), and when used in combination with an immunotherapy for cancer, the pharmaceutical composition reinforces the cancer immunotherapy and allows the immunotherapy to exert the original therapeutic effect, thus being applicable for suppressing the exacerbation of cancer cells (including the growth of cancer cells and the progression of tumors). In this sense, applications of the pharmaceutical composition of the present invention include an "immunostimulant" and "anti-cancer immunostimulator (cancer immunotherapy potentiator)" for cancer cells and tissues that express SULT2B1b and produce CS.

In the present invention, the term "cancer" means a broad range of "malignant tumors".

It is known that when a T cell and a tumor cell come into contact, a programmed cell death molecule (PD-1) and a programmed cell death ligand 1 (PD-L1) that are expressed on the cells interact with each other (PD-L1/PD-1), and as a result an immune checkpoint operates to induce immunosuppression. Inhibition of the immune checkpoint is one of current strategies of cancer immunotherapies, and therefor T cells must infiltrate into a tumor. However, experiments carried out by the present inventors have revealed that tumor cells exhibit resistance to immune checkpoint inhibition (hereinafter, also referred to as "ICI") through CS production, which serves for immune evasion (see experimental results (3)). From this, combined use of the pharmaceutical composition of the present invention, which has a CS production inhibitory effect, and a drug having an ICI effect (immune checkpoint inhibitory drug, hereinafter also referred to as "ICI drug") blocks the ICI resistance of cancer tissues, allowing the ICI effect to be successfully exhibited in an effective manner and thereby the anti-cancer effect to be exerted. ICI drugs preferably include drugs having an effect to inhibit the immune checkpoint effect caused by the interaction between PD-L1 and PD-1 to cancel the suppressed state of cytotoxic T cells and thereby activate immunity (immunostimulation).

In this sense, applications of the pharmaceutical composition of the present invention include an "immune checkpoint inhibition potentiator" (ICI potentiator) or "immune checkpoint inhibition resistance suppressor" (ICI resistance suppressor). Thus, cancer cells and tissues that particularly express SULT2B1b, produce CS, and can express PD-L1 on cell membranes temporarily, constitutively, or variably in the course of development, progression, or exacerbation of tumor can be targeted.

<u>Anti-cancer immunostimulator (cancer immunotherapy potentiator)</u>

<u>ICI potentiator, ICI resistance suppressor</u>

Cancer (malignant tumor) cells and tissues that the anti-cancer immunostimulator of the present invention targets include cancer cells and tissues that can express SULT2B1b and generate CS. Cancer cells and tissues that the present invention targets include cancer cells and tissues that express and produce SULT2B1b and lack production

capability for oxysterol such as 25-hydroxycholesterol (25-HC).

Moreover, tumors that the ICI potentiator or ICI resistance suppressor of the present invention targets can include tumors that can express and produce SULT2B1b, produce CS, and express PD-L1 on cell membranes temporarily, constitutively, or variably in the course of development, progression, or exacerbation of tumor.

In this regard, the tumors include both hematopoietic tumors and non-epithelial and epithelial malignant solid tumors. Examples of those cancers (malignant tumors) include, but are not limited to, head and neck tumors; glioblastomas (including glioblastoma multiforme); respiratory malignant tumors that develop from the bronchus or lung (including advanced-stage non-small-cell lung carcinoma); gastrointestinal malignant tumors that develop from the nasopharynx, esophagus, stomach, duodenum, jejunum, ileum, cecum, appendix, ascending colon, transverse colon, sigmoid colon, large intestine, rectum, or anal region; liver cancer, hepatocellular cancer; pancreatic cancer; urinary malignant tumors that develop from the bladder, ureter, or kidney (e.g., bladder cancer, renal cell cancer); female genital malignant tumors that develop from the ovary, oviduct, and uterus (uterine cervix, corpus uteri); breast cancer; prostate cancer; skin cancers (including melanoma); endocrine malignant tumors in the hypothalamus, pituitary gland, thyroid, parathyroid gland, and adrenal gland; central nervous system malignant tumors; malignant tumors that develop from the bone/soft tissue, sarcomas (so far, solid tumors); and myelodysplastic syndrome; leukemias such as acute lymphocytic leukemia, acute myeloid leukemia, chronic lymphocytic leukemia, chronic myeloid leukemia, acute myelomonocytic leukemia, chronic myelomonocytic leukemia, acute monocytic leukemia, chronic monocytic leukemia, acute promyelocytic leukemia, acute megakaryocytic leukemia, erythroleukemia, eosinophilic leukemia, chronic eosinophilic leukemia, chronic neutrophilic leukemia, adult T-cell leukemia, hairy cell leukemia, and plasmacytic leukemia; hematopoietic malignant tumors such as thymic carcinoma, multiple myeloma, B-cell lymphoma, and malignant lymphoma; and lymphatic malignant tumors (so far, hematopoietic tumors).

The malignant tumors include tumors known to be expressing PD-L1 (see Sandip Pravin Patel et al., Molecular Cancer Therapeutics, 2015; 14(4); 847-856, Table 1). Examples of tumor cells for which conventionally known ICI preparations such as an anti-PD-1 antibody and an anti-PD-L1 antibody are known to successfully exhibit the effects can include hematopoietic tumors (including chronic myeloid leukemia, acute myeloid leukemia, chronic eosinophilic leukemia, and acute lymphocytic leukemia), ovarian cancer, cervical cancer, corpus uteri cancer, soft tissue sarcoma, breast cancer, esophageal cancer, gastric cancer, gastroesophageal junction cancer, lung cancer (non-small-cell lung cancer, small-cell lung cancer), hepatocellular cancer, renal cell cancer, malignant melanoma, Hodgkin's lymphoma, head and neck cancer, malignant pleural mesothelioma, glioblastoma, urothelial cancer, bladder cancer, pancreatic cancer, prostate cancer, Merkel cell cancer, central nervous system primary lymphoma, testicular primary lymphoma, biliary cancer, and colon cancers that primarily develop in the ascending colon, transverse colon, sigmoid colon, and rectum.

The anti-cancer immunostimulator of the present invention (hereinafter, referred to as "the present anti-cancer immunostimulator") is in such a form that the anti-cancer immunostimulator is combined with at least one selected from the group consisting of anti-cancer agents and additional immunostimulants (hereinafter, these are collectively referred to as "the anti-cancer agent(s) or the like") and the two are simultaneously or separately administered. The present anti-cancer immunostimulator can also be administered in combination with a cancer immunotherapy such as CAR-T therapy, and can be in a form therefor. The ICI potentiator or ICI resistance suppressor of the present invention (hereinafter, collectively referred to as "the present ICI potentiator") is in such a form that the present ICI potentiator is combined with an ICI drug and the two are simultaneously or separately administered.

Examples of the anti-cancer agent or the like to be combined with the present anti-cancer immunostimulator can include common anti-cancer agents. For example, an appropriate agent for the target tumor can be selected from alkylating drugs, antimetabolites, anti-tumor antibiotics, microvascular inhibitory drugs, hormone or hormone analog drugs, platinum preparations, topoisomerase inhibitory drugs, cytokines, hormone therapy drugs, radioimmunotherapy drugs, molecular target drugs (including antibody drugs and ICI drugs), non-specific immunostimulating drugs, and other anti-cancer agents.

Examples of the alkylating drugs include, but are not limited to, cyclophosphamide, ifosfamide, busulfan, melphalan, bendamustine hydrochloride, nimustine hydrochloride, ranimustine, dacarbazine, procarbazine hydrochloride, and temozolomide. Examples of the antimetabolites include methotrexate, pemetrexed sodium, fluorouracil, doxifluridine, capecitabine, tegafur, cytarabine, cytarabine ocfosphate hydrate, enocitabine, gemcitabine hydrochloride, mercaptopurine hydrate, fludarabine phosphate, nelarabine, pentostatin, cladribine, calcium levofolinate, calcium folinate, hydroxycarbamide, L-asparaginase, and azacytidine. Examples of the anti-tumor antibiotics include doxorubicin hydrochloride, daunorubicin hydrochloride, pirarubicin, epirubicin hydrochloride, idarubicin hydrochloride, aclarubicin hydrochloride, amrubicin hydrochloride, mitoxantrone hydrochloride, mitomycin C, actinomycin D, bleomycin, peplomycin sulfate, and zinostatin stimalamer. Examples of the microvascular inhibitory drugs include vincristine sulfate, vinblastine sulfate, vindesine sulfate, vinorelbine ditartrate, paclitaxel, docetaxel hydrate, and eribulin mesylate. Examples of the hormone or hormone analog drugs (hormonal agents) include anastrozole, exemestane, letrozole, tamoxifen citrate, toremifene citrate, fulvestrant, flutamide, bicalutamide, medroxyprogesterone acetate, estramustine phosphate sodium hydrate,

goserelin acetate, and leuprorelin acetate. Examples of the platinum preparations include cisplatin, miriplatin hydrate, carboplatin, nedaplatin, and oxaliplatin. Examples of the topoisomerase inhibitory drugs include topoisomerase I inhibitory drugs such as irinotecan hydrochloride hydrate and nogitecan hydrochloride, and topoisomerase II inhibitory drugs such as etoposide and sobuzoxane. Examples of the cytokines include interferon gamma-1a, teceleukin, and celmoleukin. Examples of the radioimmunotherapy drugs include the combination drug ibritumomab tiuxetan. Examples of the molecular target drugs include gefitinib, imatinib mesylate, bortezomib, erlotinib hydrochloride, sorafenib tosylate, sunitinib malate, thalidomide, nilotinib hydrochloride hydrate, dasatinib hydrate, lapatinib tosilate hydrate, everolimus, lenalidomide hydrate, dexamethasone, temsirolimus, vorinostat, tretinoin, and tamibarotene. Examples of antibody drugs among molecular target drugs include trastuzumab (anti-HER2 antibody), rituximab (anti-CD20 antibody), bevacizumab (anti-VEGFR antibody), panitumumab, cetuximab (these are anti-EGFR antibodies), ramucirumab (anti-VEGFR antibody), mogamulizumab (anti-CCR4 antibody), brentuximab vedotin (anti-CD30 antibody), alemtuzumab (anti-CD52 antibody), and gemtuzumab ozogamicin (anti-CD33 antibody). Examples of ICI drugs include nivolumab (anti-PD-1 antibody). Examples of non-specific immunostimulating drugs include OK-432, dried BCG, *Coriolus versicolor* polysaccharide preparations, lentinan, and ubenimex. Examples of other anti-cancer agents can include aceglatone, porfimer sodium, talaporfin sodium, ethanol, and arsenic trioxide.

Preferred molecular target drugs are imatinib mesylate, nilotinib hydrochloride hydrate, and dasatinib hydrate for hematopoietic tumors, and gefitinib, erlotinib hydrochloride, and lapatinib tosilate hydrate for solid tumors. Imatinib mesylate is particularly preferred.

Examples of ICI drugs other than those shown above can include anti-PD-1 antibodies (e.g., pembrolizumab, PDR001 (code name), cemiplimab), anti-PD-L1 antibodies (e.g., avelumab, atezolizumab, durvalumab, MPDL3280A, MEDI4736), and anti-CTLA-4 antibodies (e.g., ipilimumab, tremelimumab), each targeting PD-1, PD-L1, or CTLA-4 as an immune checkpoint molecule. These ICI drugs are drugs that are currently under development as tumor therapeutics. Preferred are anti-PD-1 antibodies and anti-PD-L1 antibodies each targeting PD-1 or PD-L1 as an immune checkpoint molecule.

Examples of modes in which any of those anti-cancer agents or the like and the present anti-cancer immunostimulator or any of the ICI drugs and the present ICI potentiator are simultaneously administered can include a product obtained by formulating any of those anti-cancer agents or the like and the present anti-cancer immunostimulator or any of those ICI drugs and the present ICI potentiator into a single pharmaceutical composition (combination drug). The pharmaceutical composition is a combination drug of desired dosage form containing a pharmaceutically acceptable carrier or excipient, as necessary. The carrier or excipient to be used therefor can be any one that does not interfere with the effects and actions of the present anti-cancer immunostimulator or present ICI potentiator of the present invention. Although an appropriate form for the route of administration (mode of administration) can be set for the combination drug, a form suitable for oral administration is preferred.

The route of administration (administration method) is not limited, and examples thereof for different forms of preparations can include oral administration; and parenteral administration such as intravenous administration, intramuscular administration, subcutaneous administration, transmucosal administration, transdermal administration, and intrarectal administration. Oral administration and intravenous administration are preferred, and oral administration is more preferred.

Examples of other modes in which any of the anti-cancer agents or the like and the present anti-cancer immunostimulator or any of the ICI drugs and the present ICI potentiator are administered in combination can include a mode in which any of the anti-cancer agents or the like and the present anti-cancer immunostimulator or any of the ICI drugs and the present ICI potentiator which have been separately packaged are mixed immediately before application to a cancer patient, specifically, for example, a mode in which the present anti-cancer immunostimulator or the present ICI potentiator is added to and mixed with any of the anti-cancer agents or the like or any of the ICI drugs in the form of a solution (e.g., a drip infusion or an oral solution) for use immediately before administration to a cancer patient (before use). In the case that the anti-cancer agent or the like or the ICI drug is a preparation for parenteral administration such as a drip infusion, the present anti-cancer immunostimulator or the present ICI potentiator can be administered to a cancer patient prior to administration of the anti-cancer agent or ICI drug thereto, or concurrently with administration of the anti-cancer agent or ICI drug thereto, or after administration of the anti-cancer agent or ICI drug thereto.

In that case, the ratio for combining the anti-cancer agent or the like and the present anti-cancer immunostimulator or the ICI drug and the present ICI potentiator is not limited as long as the effects and actions of the present invention are exerted, and an appropriate ratio can be set in view of the type and site of a tumor to be treated, cancer stages (seriousness, degree of progress), the pathological condition, age, sex, and body weight of a cancer patient, and the type of the anti-cancer agent or the like or ICI drug to be used in combination.

The pharmaceutical composition of the present invention can be preferably used for the aforementioned cancer treatment. In the present invention, the meaning of treatment includes not only complete disappearance of cancer cells but also partial disappearance thereof (e.g., amelioration, mitigation). Subjects to be treated with the pharmaceutical composition of the present invention are mammals, preferably humans, affected by any of the cancers. Each subject

may be an infant, a child, or an adult, and may be male or female.

The daily dose, time of administration, and frequency of administration of the pharmaceutical composition according to the present invention can be any amount, time, and frequency effective for treatment of those diseases, and are not limited as long as the requirements are met. For example, the pharmaceutical composition according to the present invention in an amount of 1 to 100 mg in terms of compound I or a pharmaceutically acceptable salt thereof can be administered per day, though the dose is not limited to that amount.

(3) Method for screening for compound having anti-cancer immunostimulating effect and/or ICI-potentiating effect

The present invention has elucidated that CS plays an important role in the immune evasion of tumors. Specifically, the following facts were revealed:

(a) in cancer tissues in humans, CS production in cancerous regions and degree of intratumoral infiltration of $CD8^+$ T cells inversely correlate (Figure 1);
(b) CS production in tumor tissues inhibits the infiltration of effector T cells (Figures 2 and 3);
(c) tumor cells exhibit resistance to ICI through CS production (Figure 4);
(d) SULT2B1b-mediated CS production in tumors weakens anti-tumor T-cell response (Figure 5);
(e) expression of SULT2B1b suppresses the in vivo growth of tumor cells that produce oxysterol (Figure 6); and
(f) a compound having an effect to inhibit CS production promotes anti-tumor immunity by inhibiting CS-mediated T-cell exclusion (Figures 7 to 9).

On the basis of these facts, a compound that exerts an anti-cancer immunostimulating effect by promoting anti-tumor immunity and/or a compound that exerts an ICI-potentiating effect can be selected with reference to the CS production inhibitory effect as an index. Accordingly, a selected compound is effective as a candidate compound for an active ingredient of a pharmaceutical composition having an anti-cancer immunostimulating effect and/or an ICI-potentiating effect.

Thus, the present invention provides a method for screening for a compound having an anti-cancer immunostimulating effect and/or an ICI-potentiating effect, the method including the step of: selecting a compound having a CS production inhibitory effect from a candidate compound group.

Examples of the step of selecting can include at least one selected from the group consisting of an in silico screening step, an SULT assay (in vitro SULT2B1b enzymatic activity measurement) step, and a quantification step for amounts of CS and/or oxysterol generated in a cell. Specifically, compounds of interest included in candidate compound groups can be first selected by an in silico screening method as described in experimental procedure (13) and experimental results (6) in Experiment Example 1 shown later. To select a compound having a CS production inhibitory effect from the thus-selected candidate compound group, any method that allows quantitative measurement of the CS production inhibitory activity of compounds can be used, and examples thereof can include, but are not limited to, quantification (mass spectrometry) of amounts of CS and/or oxysterol generated in cells, which is described in experimental procedure (12) in Experiment Example 1 shown later, and SULT assay (in vitro SULT2B1b enzymatic activity measurement), which is described in experimental procedure (14), and any of these can be performed singly; alternatively, the two can be performed in combination.

Hereinbefore, the meanings of the terms "include" and "contain" encompass the meanings of "consist of" and "substantially consist of".

Examples

Hereinafter, the present invention will be described on the configuration and effects thereof with experiment examples as an aid for understanding. However, the present invention is by no means limited to those experiment examples. The experiments below were carried out under conditions of room temperature (25 $\pm$ 5°C) and the atmospheric pressure, unless otherwise stated. "%" and "part" in the descriptions below indicate "% by mass" and "part by mass", respectively, unless otherwise stated.

Production Examples

Introduction

$^1$D NMR spectra were measured with JEOL JNM-LA 500, JEOL ECX500 (500 MHz for $^1$H NMR), and JEOL ECS400 spectrometers (400 MHz for $^1$H NMR, 369 MHz for $^{19}$F NMR). Chemical shifts were recorded in parts per million (ppm) relative to a residual solvent peak ($CDCl_3$: $\delta_H$ 7.26, $CD_3OD$: $\delta_H$ 3.31), and coupling constants (J) are shown in

hertz (Hz). The following abbreviations are used for spin multiplicity: s = singlet, d = doublet, t = triplet, m = multiplet, and br = broad. ESI-MS spectra were measured on Agilent Technologies 6120 (for LC-MS). Column chromatography was performed with Yamazen UNIVERSAL Premium packed silica-gel. Reversed-phase column chromatography was performed with a Yamazen High Flash ODS column. Chemicals and solvents were obtained from commercial sources. In each production example, a specialized COware gas reactor (reaction scale: 1.0 to 5.0 mmol, total volume: 100 mL) was used. All moisture-sensitive reactions were performed under atmosphere of argon. All compounds used had been confirmed to have a purity of 95% or more by $^1$H NMR, unless otherwise noted.

Production Example 1

Production of (R)-4-((3S,8S,9S,10R,13R,14S,17R)-10,13-dimethyl-3-(2,2,2-trifluoroacetoxy)-2,3,4,7,8,9,10,11,12,13,14,15,16,17-tetradecahydro-1H-cyclopenta[a]phenanthren-17-yl)pentanoic acid (compound 2)

[Formula 16]

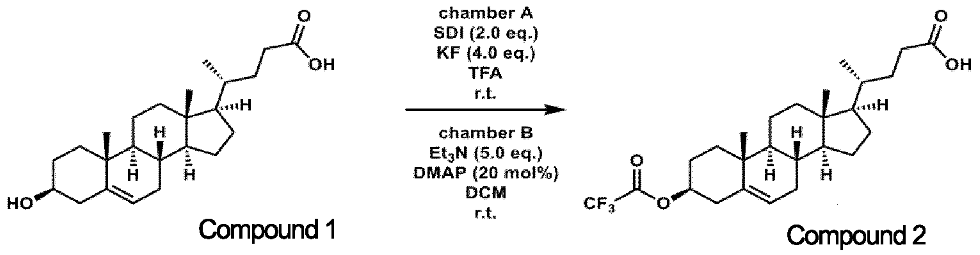


Compound 1 Compound 2

The titled compound was synthesized with reference to a method described in NPL 7.

Specifically, first, chamber A of a fire-dried COware was filled with 1,1'-sulfonyldiimidazole (SDI, 254 mg, 1.3 mmol, 2.0 eq.) and potassium fluoride (KF, 149 mg, 2.6 mmol, 4.0 eq.). Chamber B was charged with compound 1 (cholenoic acid) (240 mg, 0.64 mmol), triethylamine (445 μL, 3.2 mmol, 5.0 eq.), 4-dimethylaminopyridine (DMAP, 16 mg, 0.13 mmol, 20 mol%), and dichloromethane (DCM, 6.0 mL). Trifluoroacetic acid (TFA, 6.0 mL) was injected through the rubber septum into chamber A and instant gas (sulfuryl fluoride) formation was observed. After stirring at room temperature for 18 hours, one of the caps was carefully removed to release the residual pressure. The reaction was stirred for another 15 minutes to ensure that all sulfuryl fluoride was extracted out of the fume hood. The content of chamber B was transferred to a 100-mL round-bottomed flask, diluted with water, and subjected to extraction with $CH_2Cl_2$ by using a separatory funnel. The combined organic layer was washed with brine, dried over $Na_2SO_4$, filtered, and concentrated in vacuo. The residue was purified by column chromatography (EtOAc/hexane = 0 → 50%) to give titled compound 2 in 86% yield as a white solid (252.5 mg, 0.55 mmol).

$^1$H NMR (500 MHz, $CDCl_3$) δ 5.43 (1H, s), 4.82-4.79 (1H, m), 2.43-2.39 (3H, m), 2.30-2.24 (1H, m), 2.02-0.93 (27H, m), 0.69 (3H, s). $^{19}$F NMR (369 MHz, $CDCl_3$) δ 75.2.

Production Example 2

Production of methyl (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanoate (compound 3)

[Formula 17]

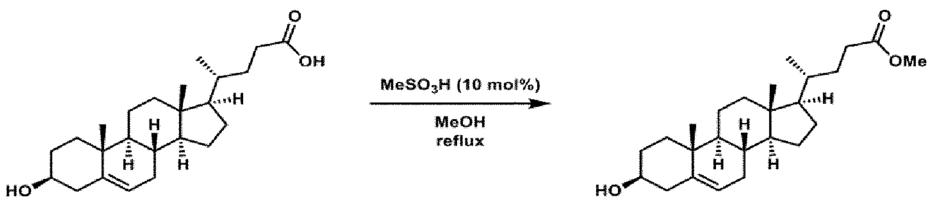


Compound 1 Compound 3

Methanesulfonic acid (1.8 μL, 0.027 mmol, 10 mol%) was added to compound 1 (cholenoic acid) (100 mg, 0.27 mmol) in methanol (10 mL), and the resultant was stirred at reflux for 1 hour. After cooling to room temperature, basic alumina was added into the mixture, which was stirred for 10 minutes. The suspension was filtered to remove basic alumina and the mother liquor was concentrated in vacuo to give titled compound 3 (106 mg, 0.27 mmol, quantitative yield).
$^1$H NMR (500 MHz, $CDCl_3$) δ 5.34 (1H, s), 3.65 (3H, s), 3.53-3.49 (1H, m), 2.36-0.91 (31H, m), 0.67 (3H, s).

Production Example 3

Production of methyl (R)-4-((3S,8S,9S,10R,13R,14S,17R)-10,13-dimethyl-3-(2,2,2-trifluoroacetoxy)-2,3,4,7,8,9,10,11,12,13,14,15,16,17-tetradecahydro-1H-cyclopenta[a]phenanthren-17-yl)pentanoate (compound 4)

[Formula 18]

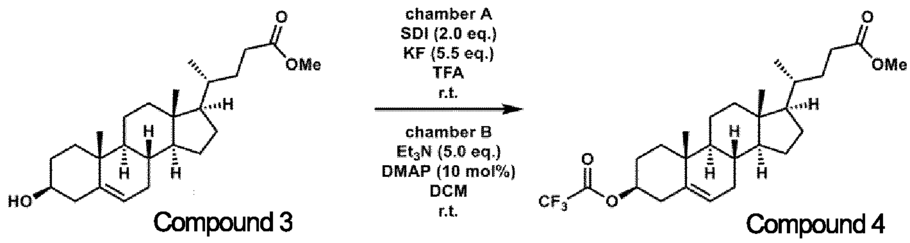


Compound 3 Compound 4

Chamber A of a fire-dried COware was filled with 1,1'-sulfonyldiimidazole (SDI, 71 mg, 0.36 mmol, 2.0 eq.) and potassium fluoride (KF, 57 mg, 0.98 mmol, 5.5 eq.). Chamber B was charged with compound 3 (69 mg, 0.18 mmol), triethylamine (123 μL, 0.89 mmol, 5.0 eq.), 4-dimethylaminopyridine (DMAP, 2.2 mg, 0.018 mmol, 10 mol%), and dichloromethane (DCM, 1.0 mL). Trifluoroacetic acid (TFA, 1.0 mL) was injected through the rubber septum into chamber A and instant gas (sulfuryl fluoride) formation was observed. After stirring at room temperature for 18 hours, one of the caps was carefully removed to release the residual pressure. The reaction was stirred for another 15 minutes to ensure that all sulfuryl fluoride was extracted out of the fume hood. The content of chamber B was transferred to a 50-mL round-bottomed flask, diluted with water, and subjected to extraction with $CH_2Cl_2$ by using a separatory funnel. The combined organic layer was washed with brine, dried over $Na_2SO_4$, filtered, and concentrated in vacuo. The residue was purified by column chromatography (EtOAc /hexane = 0 → 20%) to give titled compound 4 in 70% yield as a white solid (58 mg, 0.12 mmol).
$^1$H NMR (500 MHz, $CDCl_3$) δ 5.42 (1H, s), 4.82-4.78 (1H, m), 3.66 (3H, s), 2.35-0.92 (31H, m), 0.68 (3H, s).

Production Example 4

Production of methyl (R)-4-((3S,8S,9S,10R,13R,14S,17R)-3-methoxy-10,13-dimethyl-2,3,4,7,8,9,10,11,12,13,14,15,16,17-tetradecahydro-1H-cyclopenta[a]phenanthren-17-yl)pentanoate) (compound 5)

[Formula 19]

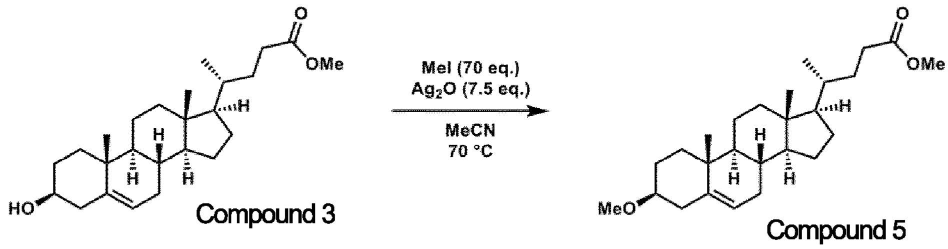


Compound 3 Compound 5

The titled compound was synthesized with reference to a method reported in NPL 14.

Specifically, first, MeI (1.1 mL, 18 mmol, 70 eq.) and $Ag_2O$ (450 mg, 1.9 mmol, 7.5 eq.) were added to a solution

of compound 3 (100 mg, 0.26 mmol) in acetonitrile (MeCN, 2.0 mL). The resulting mixture was stirred at 70°C for 14 hours. The reaction mixture was cooled to room temperature, and filtered through paper, which was rinsed with EtOAc. After concentration of the combined organic layer at reduced pressure, the residue was purified by column chromatography (EtOAc/hexane = 0 → 10%) to give titled compound 5 in 80% yield as a white solid (83 mg, 0.21 mmol) .
$^1$H NMR (400 MHz, $CDCl_3$) δ 5.35 (1H, s), 3.66 (3H, s), 3.35 (3H, s), 3.06 (1H, m), 2.39-0.91 (31H, m), 0.68 (3H, s).

Production Example 5

Production of (R)-4-((3S,8S,9S,10R,13R,14S,17R)-3-methoxy-10,13-dimethyl-2,3,4,7,8,9,10,11,12,13,14,15,16,17-tetradecahydro-1H-cyclopenta[a]phenanthren-17-yl)pentanoic acid (compound 6)

[Formula 20]

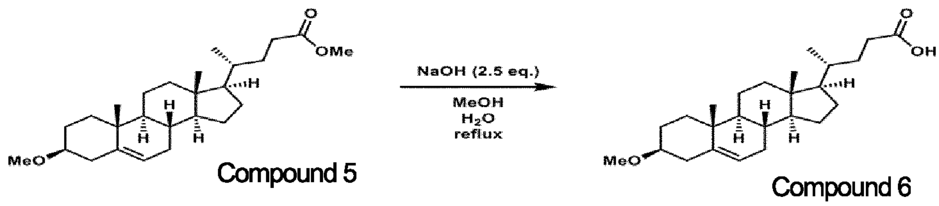

Compound 5
Compound 6

Compound 5 (20 mg, 0.050 mmol) was hydrolyzed with a methanol solution of sodium hydroxide (100 mg, 0.13 mmol, 2.5 eq.) in water (5 mL) by stirring overnight at reflux. The resulting solution was acidified with 1 M HCl to pH 3, and then precipitated solids were washed with water three times and dried in vacuo to give titled compound 6 in 54% yield as a white solid (10 mg, 0.027 mmol).
$^1$H NMR (400 MHz, $CDCl_3$) δ 5.35 (1H, s), 3.36 (3H, s), 3.09-3.03 (1H, m), 2.41-0.92 (31H, m), 0.68 (3H, s).

Production Example 6

General procedure for amide formation

Exemplified by synthesis of (3S,8S,9S,10R,13R,14S,17R)-17-((R)-5-(dibenzylamino)-5-oxopentan-2-yl)-10,13-dimethyl-2,3,4,7,8,9,10,11,12,13,14,15,16,17-tetradecahydro-1H-cyclopenta[a]phenanthren-3-yl 2,2,2-trifluoroacetate (compound 7)

[Formula 21]

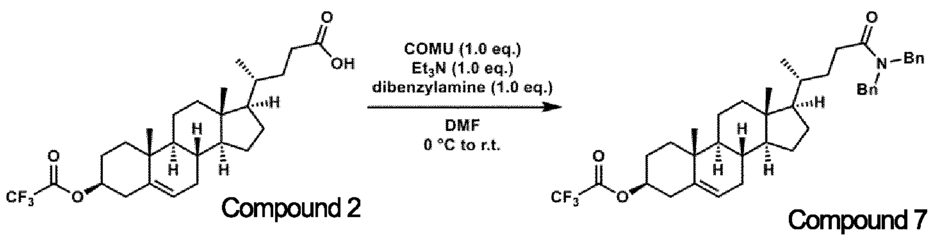

Compound 2
Compound 7

(1-Cyano-2-ethoxy-2-oxoethylidenaminooxy)dimethylaminomorpholinocarbenium hexafluorophosphate (COMU, 19 mg, 0.044 mmol, 1.0 eq.) and dibenzylamine (8.4 μL, 0.044 mmol, 1.0 eq.) were added to a solution of compound 2 (20 mg, 0.044 mmol) and $Et_3N$ (6.1 μL, 0.044 mmol, 1.0 eq.) in DMF (0.20 mL) (0°C). The resulting mixture was stirred at room temperature for 5 hours. The mixture was diluted with water and subjected to extraction with $CH_2Cl_2$. The combined organic layer was washed with brine, dried over $Na_2SO_4$, filtered, and concentrated in vacuo. The residue was purified by column chromatography (EtOAc/hexane = 0 → 20%) to give titled compound 7 in 72% yield as a colorless oil (20 mg, 0.032 mmol).

$^{1}H$ NMR (400 MHz, $CDCl_3$) δ 7.39-7.15 (10H, m), 5.42 (1H, s), 4.82-4.78 (1H, m), 4.66 (1H, d, J = 14.7 Hz), 4.54 (1H, d, J = 15.1 Hz), 4.47 (1H, d, J = 17.4 Hz), 4.42 (1H, d, J = 17.9Hz), 2.46-0.87 (31H, m), 0.66 (3H, s). $^{19}F$ NMR (369 MHz, $CDCl_3$) δ 75.2.

Production Example 7

Production of (3S,8S,9S,10R,13R,14S,17R)-17-((R)-5-(butylamino)-5-oxopentan-2-yl)-10,13-dimethyl-2,3,4,7,8,9,10,11,12,13,14,15,16,17-tetradecahydro-1H-cyclopenta[a]phenanthren-3-yl 2,2,2-trifluoroacetate (compound 8)

[Formula 22]

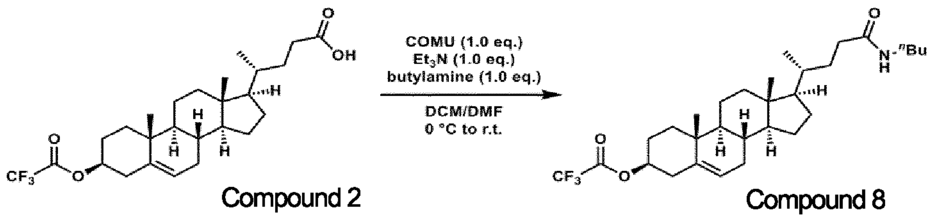


Compound 2 Compound 8

The reaction was conducted by following the general procedure for amide formation descried in Production Example 6, with use of butylamine (8.4 μL, 0.044 mmol) instead of dibenzylamine. Purification by column chromatography (EtOAc/hexane = 0 → 50%) gave titled compound 8 in 32% yield as a colorless oily matter (7.0 mg, 0.023 mmol).

$^{1}H$ NMR (500 MHz, $CDCl_3$) δ 5.58 (1H, br s), 5.42 (1H, s), 4.81 (1H, m), 4.41 (2H, dd, J = 14.3 Hz, 6.9 Hz), 3.26 (2H, dd, J = 12.6 Hz, 6.9 Hz), 2.43-0.91 (36H, m), 0.68 (3H, s).
$^{19}F$ NMR (369 MHz, $CDCl_3$) δ 75.2.

Production Example 8

Production of (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-1-thiomorpholinopentan-1-one (compound 9)

[Formula 23]

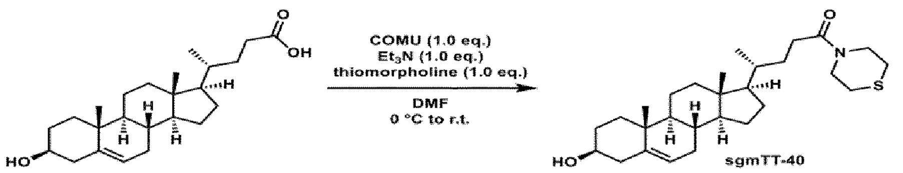


sgmTT-40

Compound 1 Compound 9

The reaction was conducted by following the general procedure for amide formation descried in Production Example 6, with use of compound 1 (cholenoic acid) (300 mg, 0.80 mmol) instead of compound 2 and use of thiomorpholine (76 μL, 0.80 mmol, 1.0 eq.) instead of dibenzylamine. Purification by column chromatography (EtOAc/hexane = 0 → 50%) gave titled compound 9 in 97% yield as a white solid (358 mg, 0.78 mmol).
$^{1}H$ NMR (500 MHz, $CDCl_3$) δ 5.35 (1H, s), 3.87 (2H, s), 3.73 (2H, s), 3.54-3.49 (1H, m), 2.61 (4H, m), 2.28-0.90 (31H, m), 0.68 (3H, s).

Production Example 9

Production of (3S,8S,9S,10R,13R,14S,17R)-10,13-dimethyl-17-((R)-5-oxo-5-thiomorpholinopentan-2-yl)-2,3,4,7,8,9,10,11,12,13,14,15,16,17-tetradecahydro-1H-cyclopenta[a]phenanthren-3-yl dihydrogen phosphate (compound 10)

[Formula 24]

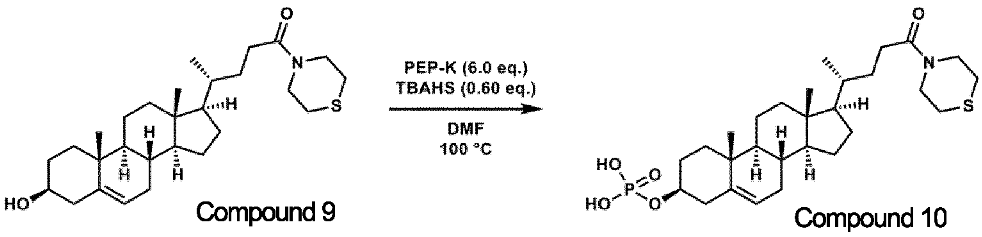


Compound 9 Compound 10

The titled compound was synthesized with reference to a procedure reported in NPL 15. Specifically, an argon gas-flushed test tube containing a magnetic stirrer bar was charged with compound 9 (20 mg, 0.044 mmol), tetrabutylammonium hydrogen sulfate (TBAHS, 8.9 mg, 0.026 mmol, 0.60 eq.), and phosphoenolpyruvate monopotassium salt (PEP-K, 54 mg, 0.26 mmol, 6.0 eq.). To the reaction mixture, N,N-dimethylformamide (DMF, 0.22 mL) was added at room temperature, and then the mixture was warmed to 100°C. After stirring for 6 hours, the reaction mixture was cooled to room temperature and purified by reversed-phase column chromatography (ODS, MeOH/$H_2O$ = 50 -> 100%) to give titled compound 10 in 74% yield as a brown solid (17 mg, 0.032 mmol). $^1$H NMR (500 MHz, $CD_3OD$) $\delta$5.39 (1H, s), 4.02 (1H, m), 3.82-3.78 (4H, m), 2.65-2.56 (4H, m), 2.43-0.98 (33H, m), 0.72 (3H, s).

Production Example 10

Production of (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-1-morpholinopentan-1-one (compound 11)

[Formula 25]

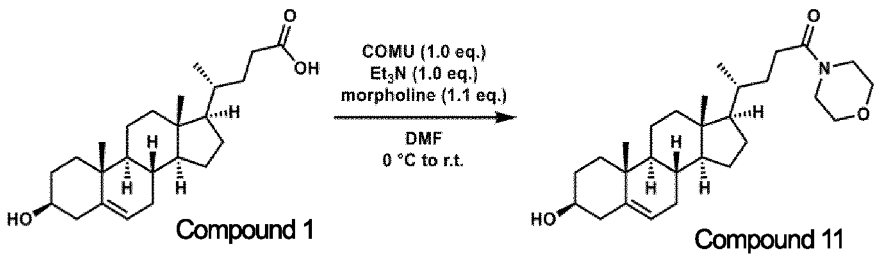


Compound 1 Compound 11

The reaction was conducted by following the general procedure for amide formation descried in Production Example 6, with use of compound 1 (cholenoic acid) (1.0 g, 2.7 mmol) instead of compound 2 and use of morpholine (244 $\mu$L, 2.8 mmol, 1.1 eq.) instead of dibenzylamine. Purification by column chromatography (EtOAc/hexane = 0 -> 100%) gave titled compound 11 in 90% yield as a white solid (1.0 g, 2.4 mmol).
$^1$H NMR (500 MHz, $CDCl_3$) $\delta$ 5.35 (1H, s), 3.67-3.65 (4H, m), 3.62 (2H, d, J = 5.2 Hz), 3.56-3.50 (1H, m), 3.46 (2H, d, J = 4.6 Hz), 2.36-0.92 (31H, m), 0.68 (3H, s).

Production Example 11

Production of (R)-4-((3R,8S,9S,10R,13R,14S,17R)-3-azido-10,13-dimethyl-2,3,4,7,8,9,10,11,12,13,14,15,16,17-tetradecahydro-1H-cyclopenta[a]phenanthren-17-yl)-1-morpholinopentan-1-one (compound 12)

[Formula 26]

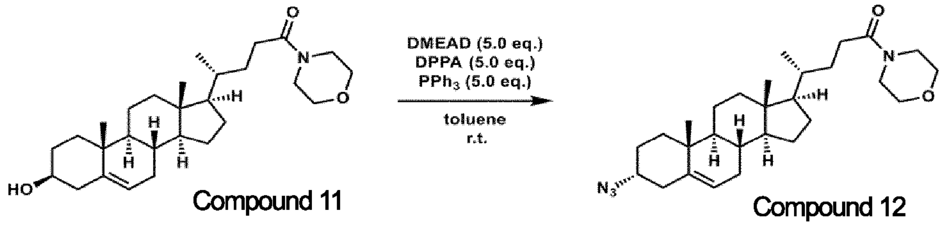

Compound 11 Compound 12

The titled compound was synthesized with reference to a procedure reported in NPL 16.

Specifically, bis(2-methoxyethyl) azodicarboxylate (DMEAD, 265 mg, 1.1 mmol, 5.0 eq.) and diphenylphosphorylazide (DPPA, 311 mg, 1.1 mmol, 5.0 eq.) in toluene (2.5 mL) were added slowly in a mixture of compound 11 (100 mg, 0.23 mmol) and triphenylphosphine (PPh3, 296 mg, 1.1 mmol, 5.0 eq.) in toluene (2.5 mL) at room temperature. The heterogeneous reaction mixture was stirred at room temperature overnight. After removal of toluene in vacuo, the residue was diluted with water, and subjected to extraction with EtOAc. The combined organic layer was washed with brine. The organic layer was dried over $Na_2SO_4$, filtered, and concentrated in vacuo. The residue was purified by column chromatography (EtOAc/hexane = 0 $\rightarrow$ 100%) to give titled compound 12 in quantitative yield as a white solid (116 mg, 0.25 mmol) .
$^1H$ NMR (500 MHz, $CDCl_3$) $\delta$ 5.39 (1H, s), 3.88 (1H, m), 3.66 (4H, m), 3.61 (2H, s), 3.46 (2H, s), 2.50-0.90 (31H, m), 068 (3H, s).

Production Example 12

Production of (R)-4-((3R,8S,9S,10R,13R,14S,17R)-3-(4-((dimethylamino)methyl)-1H-1,2,3-triazol-1-yl)-10,13-dimethyl-2,3,4,7,8,9,10,11,12,13,14,15,16,17-tetradecahydro-1H-cyclopenta[a]phenanthren-17-yl)-1-morpholinopentan-1-one (compound 13)

[Formula 27]

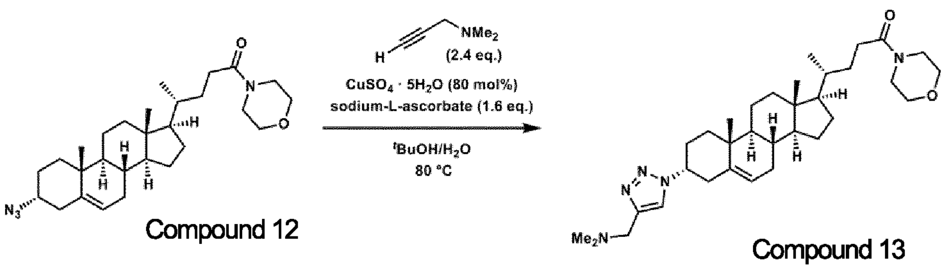

Compound 12 Compound 13

To a mixture of compound 12 (12 mg, 0.026 mmol) and N,N-dimethylpropargylamine (3.2 $\mu$L, 0.031 mmol, 2.4 eq.) in $^tBuOH/H_2O$ (1:1, 1.0 mL), $CuSO_4 \cdot 5H_2O$ (2.6 mg, 0.010 mmol, 80 mol%) and sodium-L-ascorbate (4.0 mg, 0.020 mmol, 1.6 eq.) were added. The reaction mixture was stirred at 80°C for 11 hours. The mixture was concentrated in vacuo and the residue was purified by column chromatography (MeOH/EtOAc = 0 -> 20%) to give titled compound 13 in 60% yield as a colorless oily matter (8.5 mg, 0.015 mmol).
$^1H$ NMR (500 MHz, $CDCl_3$) $\delta$ 8.13 (1H, s), 5.54 (1H, s), 4.90 (1H, s), 3.94 (2H, s), 3.68-3.45 (9H, m), 2.98-2.95 (1H, m), 2.56-0.93 (35H, m), 0.68 (3H, s). LRMS (ESI+) m/z 552 $[M + H]^+$

Production Example 13

Production of (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-N-(2-(prop-2-yn-1-yloxy)ethyl)pentanamide (compound 20)

(1) Synthesis of tert-butyl (2-hydroxyethyl)carbamate

To a solution of 2-aminomethanol (610.8 mg, 10 mmol, 1.0 eq.) in THF (10 mL), $Boc_2O$ (2.61 g, 12 mmol, 1.2 eq.) in THF (9.8 mL) was added dropwise, and the mixture was stirred at room temperature overnight. The solvent was removed in vacuo. The residue was purified by column chromatography ($SiO_2$, SNAP ultra 25 g, EtOAc /hexane= 40% -> 80%) to give the titled compound as a colorless oily matter (1.75 g, 10.9 mmol, 100% yield).
$^1$H NMR (400 MHz, $CDCl_3$) δ 4.98 (1H, m), 3.68 (2H, m), 3.28 (2H, t, J = 5.1 Hz), 2.57 (1H, m), 1.43 (9H, s)

(2) Synthesis of tert-butyl (2-(prop-2-yn-1-yloxy)ethyl)carbamate

To a solution of NaH (60% in mineral oil, 818.0 mg, 21.8 mmol, 2.0 eq.) in THF (20 mL) and DMF (20 mL), tert-butyl (2-hydroxyethyl)carbamate (1.75 g, 10.9 mmol, 1.0 eq.), which was produced in (1), in THF (9 mL) was added at 0°C, and the mixture was stirred for 30 minutes at room temperature. The solution was cooled to 0°C, propargyl bromide (1.54 mL, 21.8 mmol, 2.0 eq.) was slowly added, and the resultant was stirred at room temperature overnight. The reaction mixture was diluted with water, and subjected to extraction with hexane/EtOAc = 1:1. The extracts were dried over $Na_2SO_4$, filtered, and concentrated. The residue was purified by column chromatography ($SiO_2$, SNAP ultra 25 g, EtOAc/hexane= 10% -> 30%) to give the titled compound as a yellow oily matter (747 mg, 3.75 mmol, 34% yield).
$^1$H NMR (500 MHz, $CDCl_3$) δ 4.89 (1H, m), 4.15 (2H, s), 3.58 (2H, t, J = 5.1 Hz), 3.34 (2H, t, J = 5.1 Hz), 2.44 (1H, s), 1.43 (9H, s)

(3) Production of (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-N-(2-(prop-2-yn-1-yloxy)ethyl)pentanamide (compound 20)

[Formula 28]

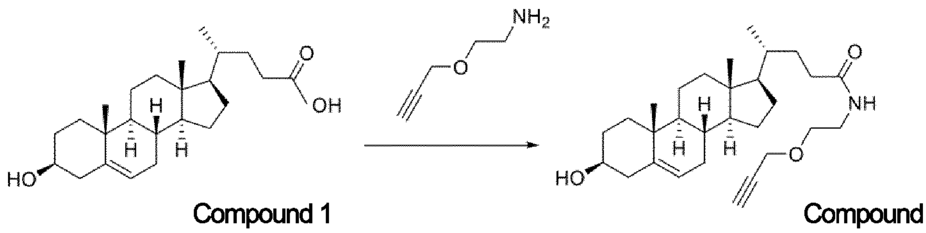

Compound 1

Compound 20

(i) To a solution of tert-butyl (2-(prop-2-yn-1-yloxy)ethyl)carbamate (747 mg, 3.75 mmol), which was produced in (2), in $CH_2Cl_2$ (6 mL), TFA (1.5 mL) was added dropwise at 0°C, and the mixture was stirred at room temperature overnight. The solvent was removed in vacuo. The residue was dissolved in dry MeOH, and anion-exchange resin (OH form) (Dowex MONOSPHERE 550A) was added, and the resultant was stirred for 10 minutes. The reaction mixture was filtered, concentrated, and used for the next step without further purification.
(ii) To a solution of compound 1 (cholenoic acid) (38.0 mg, 0.10 mmol, 1.0 eq.) in $CH_2Cl_2$ (1 mL), the amine compound obtained in (i) (31 μL, 0.25 mmol, 2.5 eq.), DMAP (15.9 mg, 0.13 mmol, 1.3 eq.), and EDCI (73.6 mg, 0.383 mmol, 3.8 eq.) were added at room temperature, and the mixture was stirred for 2.5 hours. The reaction mixture was diluted with 1 M HCl aqueous solution, and subjected to extraction with EtOAc. The extracts were dried over $Na_2SO_4$, filtered, and concentrated. The residue was purified by column chromatography ($SiO_2$, SNAP ultra 10 g, EtOAc/$CH_2Cl_2$ = 20% -> 100%) to give titled compound 20 as a white solid (41.4 mg, 0.090 mmol, 90% yield).

$^1$H NMR (500 MHz, $CDCl_3$) δ 5.81 (1H, m), 5.34 (1H, m), 4.16 (2H, s), 3.59 (2H, t, J = 5.1 Hz), 3.51 (1H, m), 3.47 (2H, t, J = 5.1 Hz), 2.45 (1H, s), 2.22-2.28 (3H, m), 2.10-2.04 (1H, m), 1.93-2.01 (2H, m), 1.88-1.75 (4H, m), 1.59 (3H, m), 1.43-1.49 (6H, m), 1.24-1.36 (2H, m), 1.04-1.15 (4H, m), 1.00 (3H, s), 0.92 (3H, d, J = 6.3 Hz), 0.67 (3H, s)

Production Example 14

Production of tetrabutylammonium salt of (3S,8S,9S,10R,13R,14S,17R)-10,13-dimethyl-17-((R)-5-oxo-5-((2-(prop-2-yn-1-yloxy)ethyl)amino)pentan-2-yl)-2,3,4,7,8,9,10,11,12,13,14,15,16,17-tetradecahydro-1H-cyclopenta[a]phenanthren-3-yl sulfate (compound 21)

[Formula 29]

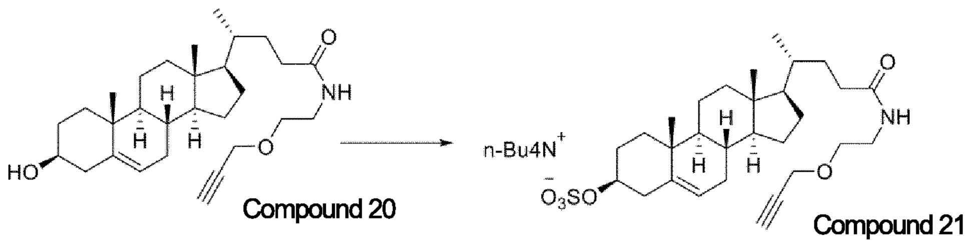

Compound 20

Compound 21

To a solution of compound 20 (41.4 mg, 0.090 mmol, 1.0 eq.) obtained in Production Example 13 in pyridine (0.4 mL), chlorosulfonic acid (26.6 μL, 0.272 mmol, 3.0 eq.) was added dropwise at 0°C, and the mixture was stirred at room temperature for 5 hours or more. The solvent was removed in vacuo. The residue was dissolved in water, $K_2HPO_4$ (158.1 mg, 0.908 mmol, 10.0 eq.) and tetrabutylammonium hydrogen sulfate (35.1 mg, 0.0953 mmol, 1.05 eq.) were added, and the resultant was stirred overnight. The reaction mixture was subjected to extraction with EtOAc, and the resultant was washed with water and brine, dried over $Na_2SO_4$, filtered, and concentrated to give tetrabutylammonium salt of titled compound 21 as a light yellow oily matter (51.1 mg, 0.065 mmol, 72% yield).
$^1$H NMR (500 MHz, $CDCl_3$) δ 5.81 (1H, m), 5.33 (1H, m), 4.23 (1H, m), 4.16 (2H, s), 3.59 (2H, t, J = 5.1 Hz), 3.47 (2H, t, J = 5.1 Hz), 3.27-3.30 (6H, m), 2.59-2.61 (1H, m), 2.45 (1H, s), 1.79-2.25 (5H, m), 1.61 (3H, m), 1.41-1.47 (4H, m), 0.98-1.29 (9H, m), 0.92 (3H, d, J = 6.8 Hz), 0.66 (3H, s)

Production Example 15

Production of (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-N-(2-(2-(prop-2-yn-1-yloxy)ethoxy)ethyl)pentanamide (compound 22)

(1) Synthesis of 2-(2-(prop-2-yn-1-yloxy)ethoxy)ethan-1-ol

Synthesis according to a procedure described later in Production Example 17(1) with use of diethylene glycol (2.37 mL, 25 mmol, 1.0 eq.) instead of tetraethylene glycol, which was used in Production Example 17(1), gave the titled compound as a yellow oily matter (2.41 g, 16.7 mmol, 67% yield).
$^1$H NMR (400 MHz, $CDCl_3$) δ 4.21 (2H, s), 3.68-3.76 (6H, m), 3.61 (2H, t, J= 4.7 Hz), 2.44 (1H, s), 2.27 (1H, m)

(2) Synthesis of 3-(2-(2-azidoethoxy)ethoxy)prop-1-yne

Synthesis according to a procedure described later in Production Example 17(2) with use of 2-(2-(prop-2-yn-1-yloxy)ethoxy)ethan-1-ol (500.0 mg, 3.47 mmol, 1.0 eq.) instead of 3,6,9,12-tetraoxapentadec-14-yn-1-ol, which was used in Production Example 17(2), gave the titled compound as a colorless oily matter (587 mg, 3.47 mmol, 100% yield).
$^1$H NMR (500 MHz, $CDCl_3$) δ 4.22 (1H, s), 3.65-3.73 (6H, m), 3.41 (2H, t, J = 5.1 Hz), 2.44 (1H, s)

(3) Synthesis of tert-butyl (2-(2-(prop-2-yn-1-yloxy)ethoxy)ethyl)carbamate

Synthesis according to a procedure described later in Production Example 17(3) for Staudinger reaction and Boc protection with use of 3-(2-(2-azidoethoxy)ethoxy)prop-1-yne (587 mg, 3.47 mmol, 1.0 eq.) instead of 1-azido-3,6,9,12-tetraoxapentadec-14-yne, which was used in Production Example 17(3), gave the titled compound as a colorless oily matter (321 mg, 1.32 mmol, 38% yield).
$^1$H NMR (500 MHz, $CDCl_3$) δ 4.98 (1H, m), 4.20 (2H, s), 3.69 (2H, t, J = 4.2 Hz), 3.63 (2H, t, J= 4.2 Hz), 3.53 (2H, t, J

= 4.8 Hz), 3.31 (2H, t, J = 4.8 Hz), 1.43 (s, 9H)

(4) Production of (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-N-(2-(2-(prop-2-yn-1-yloxy)ethoxy)ethyl)pentanamide (compound 22)

[Formula 30]

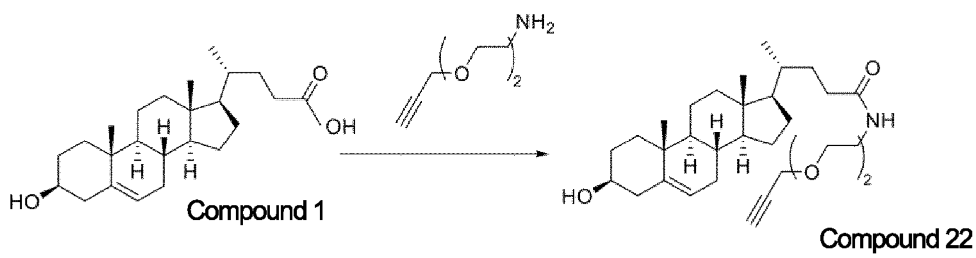

According to Production Example 13(3) with use of tert-butyl (2-(2-(prop-2-yn-1-yloxy)ethoxy)ethyl)carbamate synthesized in (3) instead of tert-butyl (2-(prop-2-yn-1-yloxy)ethyl)carbamate, which was used in step (i) of Production Example 13(3), 2-(2-(prop-2-yn-1-yloxy)ethoxy)ethan-1-amine was synthesized. Synthesis according to a procedure described in Production Example 13(3) with use of this amine compound (18.8 mg, 0.13 mmol, 1.3 equivalents) instead of the amine compound used in step (ii) of Production Example 13(3) gave titled compound 22 as a colorless oily matter (45.2 mg, 0.090 mmol, 90% yield).
$^1$H NMR (500 MHz, $CDCl_3$) δ 5.97 (1H, m), 5.34 (1H, m), 4.20 (2H, s), 3.70 (2H, m), 3.65 (2H, t, J = 5.1 Hz), 3.55 (2H, t, J = 5.1 Hz), 3.52 (1H, m), 3.45 (2H, t, J = 4.5 Hz), 2.44 (1H, s), 2.22-2.27 (3H, m), 2.04-2.09 (1H, m), 1.95-1.98 (2H, m), 1.82-1.84 (4H, m), 1.59 (3H, m), 1.43-1.49 (6H, m), 1.25-1.35 (2H, m), 1.04-1.18 (4H, m), 1.00 (3H, s), 0.93 (3H, d, J = 6.3 Hz), 0.67 (3H, s)

Production Example 16

Production of tetrabutylammonium (3S,8S,9S,10R,13R,14S,17R)-10,13-dimethyl-17-((R)-5-oxo-5-((2-(2-(prop-2-yn-1-yloxy)ethoxy)ethyl)amino)pentan-2-yl)-2,3,4,7,8,9,10,11,12,13,14,15,16,17-tetradecahydro-1H-cyclopenta[a]phenanthren-3-ylsulfate (compound 23)

[Formula 31]

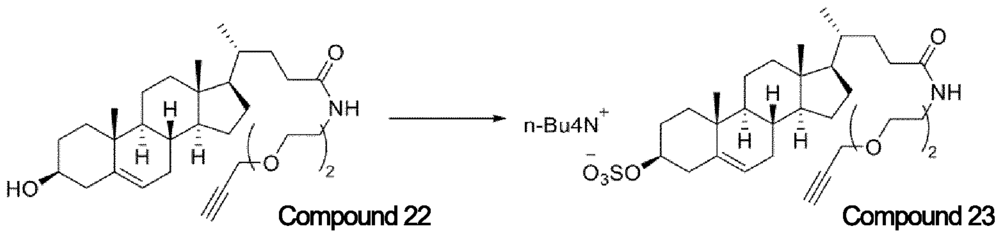

Synthesis according to a procedure described in Production Method 14 with use of compound 22 (45.2 mg, 0.090 mmol, 1.0 eq.) produced in Production Method 15 instead of compound 20 in Production Method 14 gave titled compound 23 as a yellow oily matter (48.0 mg, 0.058 mmol, 64% yield).
$^1$H NMR (400 MHz, $CDCl_3$) δ 5.99 (1H, m), 5.32 (1H, m), 4.22-4.25 (1H, m), 4.20 (2H, s), 3.69 (2H, t, J = 4.9 Hz), 3.65 (2H, t, J = 5.3 Hz), 3.55 (2H, t, J = 5.3 Hz), 3.45 (2H, t, J = 4.9 Hz), 3.26-3.30 (6H, m), 2.58-2.61 (1H, m), 2.44 (1H, s), 1.78-2.24 (5H, m), 1.60-1.66 (3H, m), 1.39-1.46 (4H, m), 0.97-1.28 (9H, m), 0.92 (3H, d, J = 6.2 Hz), 0.65 (3H, s)

Production Example 17

Production of (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-N-(3,6,9,12-tetraoxapentadec-14-yn-1-yl)pentanamide (compound 24)

(1) Synthesis of 3, 6, 9, 12-tetraoxapentadec-14-yn-1-ol

To a solution of NaH (60% in mineral oil, 800.0 mg, 20 mmol, 1.0 eq.) in THF (20 mL), tetraethylene glycol (3.44 mL, 20 mmol, 1.0 equivalent) was added at 0°C, and the mixture was stirred at room temperature for 20 minutes. The solution was cooled to 0°C, propargyl bromide (1.51 mL, 20 mmol, 1.0 eq.) was added, and the resultant was stirred at room temperature overnight. The reaction mixture was diluted with saturated $NH_4Cl$ aqueous solution, and subjected to extraction with EtOAc. The extracts were dried over $Na_2SO_4$, filtered, and concentrated. The residue was purified by column chromatography ($SiO_2$, SNAP ultra 25 g, EtOAc/hexane = 20% -> 100%, MeOH/EtOAc = 0% -> 4%) to give the titled compound as a colorless oily matter (2.45 g, 10.5 mmol, 53% yield).
$^1$H NMR (500 MHz, $CDCl_3$) δ 4.19 (2H, s), 3.64-3.71 (14H, m), 3.60 (2H, t, J = 4.2 Hz), 2.74 (1H, m), 2.42 (1H, s)

(2) Synthesis of 1-azido-3,6,9,12-tetraoxapentadec-14-yne

To a solution of the compound synthesized in (1) (962.0 mg, 4.14 mmol, 1.0 eq.) in DMF (8 mL), diphenylphosphorylazide (1.1 mL, 4.96 mmol, 1.2 eq.) and DBU (0.74 mL, 4.96 mmol, 1.2 eq.) were added, and the mixture was stirred at 70°C overnight. The solution was cooled to room temperature and diluted with water, and subjected to extraction with hexane/EtOAc = 1:1, and then with EtOAc. The extracts were washed with brine, dried over $Na_2SO_4$, filtered, and concentrated. The residue was purified by column chromatography ($SiO_2$, SNAP ultra 10 g, EtOAc/hexane = 50% —> 100%) to give the titled compound as a colorless oily matter (562.0 mg, 2.18 mmol, 53% yield).
$^1$H NMR (500 MHz, $CDCl_3$) δ 4.20 (2H, s), 3.66-3.70 (14H, m), 3.39 (2H, t, J = 5.1 Hz), 2.42 (1H, s)

(3) Synthesis of tert-butyl (3,6,9,12-tetraoxapentadec-14-yn-1-yl)carbamate

To a solution of the compound synthesized in (2) (562 mg, 2.18 mmol, 1.0 eq.) in THF (7 mL), triphenylphosphine (734 mg, 2.6 mmol, 1.2 equivalents) and $H_2O$ (1.5 mL) were added, and the mixture was stirred at room temperature overnight. Then, $Boc_2O$ (30% in THF, 2.1 mL, 2.6 mmol, 1.2 eq.) was added to the solution, which was stirred for 4.5 hours. $Na_2SO_4$ was added to the reaction, which was filtered and concentrated. The residue was purified by column chromatography ($SiO_2$, SNAP ultra 10 g, THF/hexane = 20% -> 40%) to give the titled compound as a colorless oily matter (653 mg, 1.97 mmol, 90% yield).
$^1$H NMR (500 MHz, $CDCl_3$) δ 5.07 (1H, m), 4.20 (2H, s), 3.61-3.70 (12H, m), 3.53 (2H, t, J = 5.1 Hz), 3.31 (2H, t, J = 5.1 Hz), 2.42 (1H, s,), 1.43 (9H, s)

(4) Production of (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)-N-(3,6,9,12-tetraoxapentadec-14-yn-1-yl)pentanamide (compound 24)

[Formula 32]

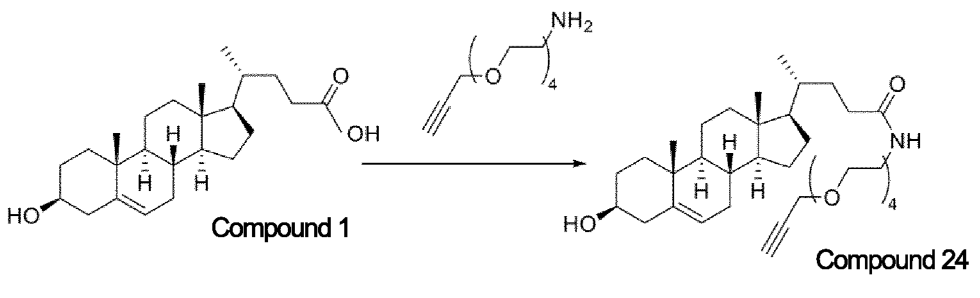

Compound 1

Compound 24

According to Production Example 13(3) with use of tert-butyl (3,6,9,12-tetraoxapentadec-14-yn-1-yl)carbamate synthesized in (3) instead of tert-butyl (2-(prop-2-yn-1-yloxy)ethyl)carbamate used in step (i) of Production Example

13(3), 3,6, 9, 12-tetraoxapentadec-14-yn-1-amine was synthesized. Synthesis according to the procedure described in Production Example 13(3) with use of this amine compound (25.6 mg, 0.11 mmol, 1.3 eq.) instead of the amine compound used in step (ii) of Production Example 13(3) gave titled compound 24 as a colorless oily matter (51.8 mg, 0.088 mmol, 100% yield).
$^1$H NMR (500 MHz, $CDCl_3$) δ 6.30 (1H, m), 5.34 (1H, m), 4.25 (2H, s), 3.65-3.73 (12H, m), 3.48-3.56 (4H, m), 2.43 (1H, s), 2.22-2.27 (3H, m), 2.04-2.11 (1H, m), 1.94-1.97 (2H, m), 1.82-1.85 (7H, m), 1.40-1.56 (6H, m), 1.25-1.36 (2H, m), 1.06-1.15 (4H, m), 0.99 (3H, s), 0.92 (3H, d, J = 6.3 Hz), 0.66 (3H, s)

Production Example 18

Production of tetrabutylammonium salt of (3S,8S,9S,10R,13R,14S,17R)-10,13-dimethyl-17-((R)-17-oxo-4,7,10,13-tetraoxa-16-azahenicos-1-yn-20-yl)-2,3,4,7,8,9,10,11,12,13,14,15,16,17-tetradecahydro-1H-cyclopenta[a]phenanthren-3-yl sulfate (compound 25)

[Formula 33]

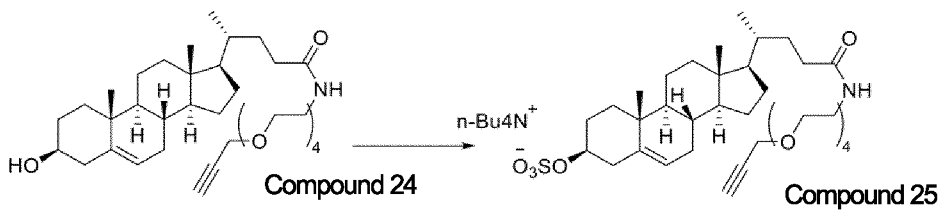

Compound 24 Compound 25

Synthesis according to the procedure described in Production Method 14 with use of compound 24 (51.8 mg, 0.088 mmol, 1.0 eq.) produced in Production Method 17 instead of compound 20 in Production Method 14 gave tetrabutylammonium salt of titled compound 25 as a light yellow oily matter (43.8 mg, 0.048 mmol, 54% yield).
$^1$H NMR (500 MHz, $CDCl_3$) δ 6.25 (1H, m), 5.32 (1H, m), 4.22 (1H, m), 4.19 (2H, s), 3.62-3.69 (12H, m), 3.53 (2H, t, J = 5.1 Hz), 3.43 (2H, t, J = 5.1 Hz), 3.27-3.30 (6H, m), 2.58-2.61 (1H, m), 2.42 (1H, s), 1.94-2.34 (5H, m), 1.61-1.67 (3H, m), 1.40-1.48 (4H, m), 1.00-1.29 (9H, m), 0.92 (3H, d, J = 6.3 Hz), 0.65 (3H, s)

Production Example 19

Production of (R)-N-(3,6,9,12,15,18-hexaoxahenicos-20-yn-1-yl)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanamide (compound 26)

(1) Synthesis of 3, 6, 9, 12, 15, 18-hexaoxahenicos-20-yn-1-ol

Synthesis according to the procedure described in Production Example 17(1) with use of hexaethylene glycol (2.5 mL, 10 mmol, 1.0 equivalent) instead of tetraethylene glycol in Production Example 17(1) gave the titled compound as a colorless oily matter (1.68 g, 5.24 mmol, 52% yield).
$^1$H NMR (500 MHz, $CDCl_3$) δ 4.19 (2H, s), 3.61-3.70 (22H, m), 3.59 (2H, t, J = 4.5 Hz), 2.89 (1H, m), 2.42 (1H, s),

(2) Synthesis of 1-azido-3,6,9,12,15,18-hexaoxahenicos-20-yne

Synthesis according to Production Example 17(2) with use of the compound synthesized in (1) (1.68 g, 5.24 mmol, 1.0 eq.) instead of 3,6,9,12-tetraoxapentadec-14-yn-l-ol in Production Example 17(2) gave the titled compound as a light yellow oily matter (756.0 mg, 2.19 mmol, 42% yield).
$^1$H NMR (500 MHz, $CDCl_3$) δ 4.20 (1H, s), 3.64-3.70 (22H, m), 3.38 (2H, t, J = 5.1 Hz), 2.42 (1H, s), 1.74 (2H, s)

(3) Synthesis of tert-butyl (3,6,9,12,15,18-hexaoxahenicos-20-yn-1-yl)carbamate

Synthesis according to the general procedure described in Production Example 17(3) for Staudinger reaction and Boc protection with use of the synthesized compound (756 mg, 2.19 mmol, 1.0 eq.) instead of 1-azido-3,6,9,12-tetraoxapentadec-14-yne in Production Example 17(3) gave the titled compound as a white solid (625 mg, 1.49 mmol,

68% yield).
$^1H$ NMR (500 MHz, $CDCl_3$) δ 5.11 (1H, m), 4.19 (2H, s), 3.60-3.69 (20H, m), 3.52 (2H, t, J = 5.1 Hz), 3.30 (2H, t, J = 5.1 Hz), 1.43 (9H, s)

(4) Production of (R)-N-(3,6,9,12,15,18-hexaoxahenicos-20-yn-1-yl)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanamide (compound 26)

[Formula 34]

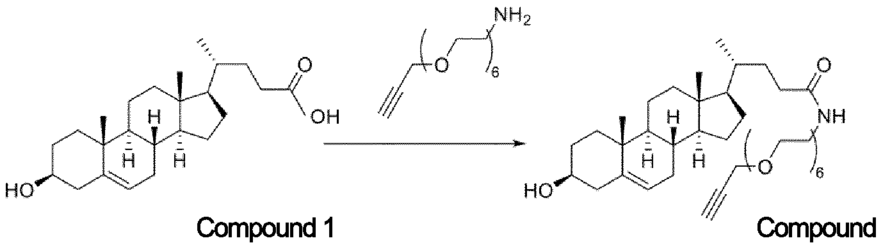

Compound 1 Compound 26

According to Production Example 13(3) with use of tert-butyl (3,6,9,12,15,18-hexaoxahenicos-20-yn-1-yl)carbamate synthesized in (3) instead of tert-butyl (2-(prop-2-yn-1-yloxy)ethyl)carbamate, which was used in step (i) of Production Example 13(3), 3,6, 9, 12, 15, 18-hexaoxahenicos-20-yn-1-amine was synthesized. Synthesis according to the procedure described in Production Example 13(3) with use of this amine compound (45.6 mg, 0.14 mmol, 1.3 eq.) instead of the amine compound used in step (ii) of Production Example 13(3) gave titled compound 26 as a colorless oily matter (58.7 mg, 0.086 mmol, 79% yield).
$^1H$ NMR (500 MHz, $CDCl_3$) δ 6.30 (1H, m), 5.34 (1H, m), 4.20 (2H, s), 3.62-3.68 (20H, m), 3.54 (2H, t, J = 5.1 Hz), 3.43-3.44 (2H, m), 2.42 (1H, s), 2.21-2.29 (3H, m), 2.03-2.09 (1H, m), 1.94-2.00 (2H, m), 1.82-1.84 (7H, m), 1.40-1.56 (6H, m), 1.25-1.33 (2H, m), 1.06-1.17 (4H, m), 0.99 (3H, s), 0.92 (3H, d, J = 6.3 Hz), 0.66 (3H, s)

Production Example 20

Production of tetrabutylammonium salt of (3S,8S,9S,10R,13R,14S,17R)-10,13-dimethyl-17-((R)-23-oxa-4,7,10,13,16,19-hexaoxa-22-azaheptacos-1-yn-26-yl)-2,3,4,7,8,9,10,11,12,13,14,15,16,17-tetradecahydro-1H-cyclopenta[a]phenanthren-3-yl sulfate (compound 27)

[Formula 35]

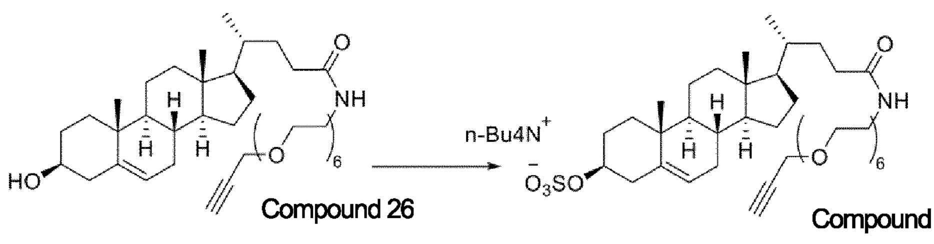


Compound 26 Compound 27

Synthesis according to the procedure described in Production Method 14 with use of compound 26 (58.7 mg, 0.086 mmol, 1.0 eq.) produced in Production Method 19 instead of compound 20 in Production Method 14 gave tetrabutylammonium salt of titled compound 27 as a light yellow oily matter (63.6 mg, 0.063 mmol, 73% yield).
$^1H$ NMR (500 MHz, $CDCl_3$) δ 6.37 (1H, m), 5.32 (1H, m), 4.22 (1H, m), 4.19 (2H, s), 3.62-3.67 (20H, m), 3.53 (2H, t, J = 5.1 Hz), 3.43 (2H, m), 3.25-3.28 (6H, m), 2.57-2.61 (1H, m), 2.43 (1H, s), 1.78-2.23 (5H, m), 1.60-1.64 (3H, m), 1.40-1.44 (4H, m), 0.99-1.26 (9H, m), 0.91 (3H, d, J= 6.3 Hz), 0.65 (3H, s)

Experiment Example 1

I. Experimental materials and preparation methods therefor

(1) Human tissue samples

Human cancer tissue samples (colon cancer, prostate cancer, renal cancer) and normal large intestine tissue samples were collected from patients during surgery in accordance with an ethics committee protocol from the Kyushu University Hospital, the Keio University Hospital, or the Saitama Medical University Hospital. A specimen immediately after resection was put in liquid nitrogen during surgery, and stored for analysis. The research protocol had been approved in advance by the institutional review boards of the participating institutes. Informed consent was obtained through an opt-out/opt-in approach in accordance with a policy of each participating institute.

(2) Cancer cell lines

E0771 cells and E.G7-OVA cells were purchased from CH3 BioSystems LLC and ATCC, respectively. 3LL cells were obtained from Japanese Collection Research Bioresources, and MC38 cells, which are previously reported cells (NPL 8), were donated by Dr. S. A. Rosenberg (National Cancer Institute, Bethesda, State of Maryland). E0771, E.G7-OVA, and 3LL cells were maintained with RPMI-1640 medium (FUJIFILM Wako Pure Chemical Corporation), and MC38 cells were maintained with DMEM medium (FUJIFILM Wako Pure Chemical Corporation). These media each contained 10% heat-inactivated fetal bovine serum (FBS; Life Technologies), 50 $\mu$M 2-mercaptoethanol, 2 mM L-glutamine, 100 U/ml penicillin, 100 $\mu$g/ml streptomycin, 1 mM sodium pyruvate, and 1$\times$ MEM non-essential amino acids (Life Technologies). For obtaining culture supernatant of MC38 cells and derivatives thereof, 5 $\times$ $10^5$ cells were seeded in a 24-well plate and cultured with 3% FBS for 30 hours, and the supernatant was collected after the cells became confluent.

(3) Mice and treatment therefor

C57BL/6 mice and BALB/c nude mice were purchased from CLEA Japan, Inc., and housed under specific pathogen-free conditions in an animal facility in the Kyusyu University. The animal experiment protocol had been approved in advance by the animal experiment ethics committee of the Kyusyu University.

As described later for each experiment, 200 $\mu$l of an anti-mouse PD-L1 antibody (10F.9G2, 0.5 mg/ml, BioXCell) or a control of the matched isotype (LTF-2, 0.5 mg/ml, BioXCell) was intraperitoneally injected into tumor-bearing mice (see experimental results (3)).

In T cell transfer experiment, activated OTII $CD4^+$ T cells (2 $\times$ $10^6$) and OTI $CD8^+$ T cells (5 $\times$ $10^5$) were intravenously injected into tumor-bearing mice (see experimental results (4)).

Cholenoic acid was suspended in 0.5% (w/v) methylcellulose 400 solution (FUJIFILM Wako Pure Chemical Corporation) at a proportion of 50 mg/ml, and a 200-$\mu$l portion (10 mg) of the suspension was orally administered to mice (see experimental results (6)). To examine the effect on the tumor infiltration of OTII cells or OTI T cells, 5 mg of cholenoic acid was orally administered three times at intervals of 6 hours in 2 days before the assay.

II. Experimental procedure

(1) Transfectants

E0771-SULT cells and 3LL-SULT cells were prepared by retroviral infection with pMX vectors encoding mouse Sult2B1b with an HA tag at the C terminus (pMX-SULT2B1b-IRES-GFP and pMX-SULT2B1b-puromycin were used, respectively). Platinum-E packaging cells were transfected with plasmids with use of FuGENE 6 transfection reagent (Promega Corporation). The cell culture supernatant was collected 48 hours after the transfection, supplemented with 5 $\mu$g/ml polybrene, and used for the infection of E0771 cells and 3LL cells. E.G7-OVA-SULT cells and MC38-SULT cells were produced by transfecting E.G7-OVA cells and MC38 cells with a pSI-puro vector encoding mouse Sult2B1b with use of Amaxa Nucleofector (TM) II (for E.G7-OVA cells) or polyethyleneimine (for MC38 cells). After limiting dilution, selection was performed with puromycin (4 $\mu$g/ml for 3LL and E.G7-OVA cells, 2 $\mu$g/ml for MC38 cells) or without puromycin (for E0771 cells) to isolate clones.

For expression of PD-L1, a pBJ-neo vector encoding mouse PD-L1 was used. To express I-$A^b$/OVA complex, cells were transfected with a pBJ-neo vector encoding mouse I-$A^b\alpha$ and a pCAG vector encoding I-$A^b\beta$ covalently bound to an OVA peptide (OVA323-339: ISQAVHAAHAEINEAGR [SEQ ID NO: 1]) at a ratio of 1:5 with use of polyethyleneimine. After selection with G418 (1 mg/ml), clones in which expression of PD-L1 or I-$A^b$/OVA complex was comparable were

selected for use.

(2) Knock-out of Ch25h in MC38 cells

To genetically inactivate CH25H expression in MC38 cells, a CRISPR-Cas9 genome editing system combined with gene targeting by homologous recombination (HR) was used. A target guide RNA sequence was selected from the single exon of the mouse Ch25h gene by using a CHOPCHOP Web tool (https://chopchop.rc.fas.harvard.edu/).

Two complimentary oligonucleotides containing a target sequence and a Bbs I ligation adaptor (5'-CACC GATCTTGTAGCGTCGCAGGA-3' [SEQ ID NO: 2] and 5'-AAACTCCTGCGACGCTACAAGATC-3' [SEQ ID NO: 3]) were synthesized, annealed, and ligated to a Bbs I-digested pX330 vector, giving a single-guide RNA (sgRNA) vector.

Through PCR using primers shown below, 5' and 3' homology arms (961 bp and 1084 bp) were amplified from a genomic DNA isolated from MC38 cells to construct an HR targeting vector.

Target 5'-arm_F: 5'-ACCGGTACCTGTGAGGTTAGAGCCCAGTTCCAG-3' [SEQ ID No. 4]
Target 5'-arm_R: 5'-ACCGCGGCCGCACCCGGACTTCTCGCCTTC -3' [SEQ ID No. 5]
Target 3'-arm_F: 5'-TCCGGATCCTGGGGACCCAGGGATACAGGAC -3' [SEQ ID No. 6]
Target 3'-arm_R: 5'-GACGTCGACCAGTGTCTGAAAGGGTATCCAGG-3' [SEQ ID No. 7]

The amplified DNAs were inserted into Kpn I-Not I and Bam HI-Sal I sites of an HR510PA-1 targeting vector having an RFP expression cassette and a hygromycin-resistant gene between homology arms (System Biosciences, LLC).

On a 60-mm culture dish, MC38 cells were transfected with 1.25 $\mu$g of the sgRNA vector and 1.25 $\mu$g of the HR targeting vector with use of 10 $\mu$l of Lipofectamine 2000 (Invitrogen). Cells were selected with hygromycin (200 $\mu$g/ml) 48 hours after the transfection, and cloned through limiting dilution. Cells for which Ch25h expression was genetically inactivated were identified by RT-qPCR, and the lack of 25-HC production was confirmed by mass spectrometry.

(3) Western blot analysis

The whole cell lysate (30 $\mu$g) was separated by SDS-PAGE, and immunoblotted with a rabbit anti-SULT2B1b antibody (1:1000 dilution, NPL 2) or a goat anti-actin antibody (I-19, 1:1000 dilution; Santa Cruz Biotechnology, Inc.). Subsequently, the resultant was incubated with an HRP-labeled secondary antibody (1:2000 dilution; Santa Cruz Biotechnology, Inc.).

(4) Reverse transcription-qPCR

Total RNA was isolated by using ISOGEN (NIPPON GENE CO., LTD.). After treating with RNase-free DNase I (Thermo Fisher Scientific), the RNA sample (1 $\mu$g) was reverse-transcribed with oligo (dT) primers (Thermo Fisher Scientific) and SuperScript III reverse transcriptase (Thermo Fisher Scientific), and amplified by PCR. Quantitative PCR was carried out by using a CFX Connect real-time PCR detection system (Bio-Rad Laboratories, Inc.) with SYBR Green PCR Master Mix (Thermo Fisher Scientific) in accordance with the instruction from the manufacturer. Primers for Ch25h, Cyp27a1, and Cyp7a1 were provided from Prime PCR (TM) PreAmp for SYBR (R) GreenAssays (Bio-Rad Laboratories, Inc.). For primers for Gapdh, 5'-TGTGTCCGTCGTGGATCTGA-3' [SEQ ID NO: 8] and 5'-TTGCTGTTGAAGTCGCAGGAG-3' [SEQ ID NO: 9] were used. Expressions of target genes were normalized with respect to expression of Gapdh.

(5) Separation and activation of T cells

$CD4^+$ T cells or $CD8^+$ T cells were magnetically selected from the spleen and peripheral lymph nodes of OTII and OTI TCR transgenic mice with Dynabeads mouse CD4 (Invitrogen), and then treated with DETACHaBEAD (TM) mouse CD4 (Invitrogen) or separated with a $CD8a^+$ T Cell Isolation Kit (Miltenyi Biotec) . For priming by antigen stimulation, OTII $CD4^+$ T cells or OTI $CD8^+$ T cells ($1 \times 10^6$ cells per well) were cultured for 3 days in a 24-well plate containing C57BL/6 splenocytes that had been made T-cell-depleted and exposed to radiation ($5 \times 10^6$ cells per well) and OVA323-339 (0.5 $\mu$g/ml) or OVA257-264 (0.5 $\mu$g/ml) in a total volume of 2 ml.

Viable cells were collected by density gradient centrifugation with Lympholyte-M cell separation medium (Cedarlane). Evaluation by flow cytometry confirmed that the purity of live OTII $CD4^+$ T cells ($CD4^+V\alpha2^+V\beta5^+$) or OTI $CD8^+$ T cells ($CD8a^+V\alpha2^+V\beta5^+$) was over 98%.

(6) Transplantation and in vivo tumor growth

After washing with phosphate-buffered saline (PBS), cancer cells in a specific number shown below were resuspended in 200 μl of PBS, and injected into the back of 6- to 8-week-old female C57BL/6 mice or BALB/c nude mice.

E0771-SULT cells and E0771-control cells: $1 \times 10^6$/mouse,
E0771-SULT-PD-L1 cells and E0771-control-PD-L1 cells: $1 \times 10^6$/mouse,
E0771-SULT-OVA cells and E0771-control-OVA cells: $2 \times 10^6$/mouse,
3LL-SULT-OVA cells and 3LL-control-OVA cells: $3 \times 10^6$/mouse,
E.G7-OVA-SULT cells and E.G7-OVA cells: $1 \times 10^6$/mouse,
MC38 cells and MC38-SULT cells: $5 \times 10^5$/mouse,
MC38-DCH25 cells and MC38-DCH25-SULT cells: $5 \times 10^5$/mouse.

The growth of tumors was monitored by measuring the vertical diameters of each tumor with a caliper twice a week and calculating the tumor volumes from the following expression.

[Expression]

$$\text{Tumor volume} = \pi / 6 \times \sqrt{(\text{length} \times \text{width})}^3$$

(7) Separation of tumor-infiltrating cells

Each of tumors surgically dissected from mice was put in a 15-ml polypropylene tube containing 4 ml of Hanks' balanced salt solution (HBSS, 14175, Gibco) to which 2% FBS, 0.05 mg/ml collagenase (C-2139, Sigma-Aldrich Co. LLC), 1 mM sodium pyruvate, 25 mM HEPES, and 10 mg/ml DNase I (F. Hoffmann-La Roche Ltd.) had been added, and incubated in a water bath at 37°C under shaking at 80 rpm for 30 minutes. The digested tumor sample was passed through a 75-μm cell strainer, supplemented with 4 ml of fresh HBSS, and then placed on ice to prevent the tumor sample to be further digested before being subjected to flow cytometry analysis or CyTOF analysis.

(8) Flow cytometry

Flow cytometry analysis was performed with a FACSCalibur (TM) or FACSVerse (TM) cytometer (BD Biosciences). In the flow cytometry analysis, the following antibodies and reagents were used:

anti-Fcy III/II receptor: 2.4G2, 0.5 μg/ml; TONBO biosciences; for blocking,
APC-binding anti-mouse CD274:PD-L1: 10F.9G2, 2 μg/ml; BioLegend,
APC-binding rat IgG2b: RTK4530, 2 μg/ml; BioLegend,
PE-binding anti-MHC II: M5/114.15.2, 0.2 μg/ml; Invitrogen,
PE-binding rat IgG2b: A95-1; 0.2 μg/ml; BD Pharmingen,
FITC-binding anti-mouse CD3ε: 145-2C11, 5 μg/ml; BD Pharmingen,
PE-binding anti-mouse CD4: RM4-5, 2 μg/ml; BD Pharmingen,
PE-binding anti-mouse CD8a: 53-6.7, 2 μg/ml; TONBO biosciences,
PerCP-Cy5.5-binding anti-mouse Vα2: B20.1, 2 μg/ml; BD Pharmingen,
biotin-binding anti-mouse Vβ5: MR9-4, 5 μg/ml; BD Pharmingen, and
APC-streptavidin: 0.4 μg/ml; BD Pharmingen.

(9) CyTOF analysis

Tumor-infiltrating leukocytes were suspended in PBS containing 1 μM Cell-ID cisplatin 194Pt (Fluidigm Corp.) in a 15-ml polypropylene tube, incubated at room temperature for 5 minutes, and then quenched with MaxPar Cell Staining Buffer (Fluidigm Corp.). The cells were precipitated by centrifuging at 4°C and 500 ×g for 7 minutes, then resuspended in 50 μl of MaxPar Cell Staining Buffer (CD16/32; BD Pharmingen) containing Fc block, and incubated at room temperature for 10 minutes. Next, the cells were further reacted with a metal-tagged cell surface antibody (Fluidigm Corp.) at room temperature for 30 minutes, and washed with MaxPar Cell Staining Buffer 500 g at 4°C for 7 minutes. To further stain intracellular proteins, the cells were fixed with 1 ml of fresh 1× Maxpar Fix I Buffer (Fluidigm Corp.) at room temperature for 15 minutes, washed with 4 ml of MaxPar Perm-S Buffer (Fluidigm Corp.) 800 g at 4°C for 7 minutes, and then resuspended in 50 μl of MaxPar Perm-S Buffer containing a metal-tagged cytoplasmic/secretory antibody (Fluidigm Corp.). The cells were incubated at room temperature for 30 minutes, and then washed with MaxPar Cell

Staining Buffer. The sample was resuspended in 1 ml of MaxPar Fix and Perm Buffer (Fluidigm Corp.) containing 125 nM Cell-ID Intercalator-Ir (Fluidigm Corp.) at 4°C overnight. The following day, the cells were washed twice with 2 ml of MaxPar Cell Staining Buffer, and resuspended in MaxPar Cell Acquisition Solution (Fluidigm Corp.). The cells were passed twice through a 35-μm strainer (Falcon) to remove aggregated cells, and the resultant was then subjected to analysis with CyTOF Mass Cytometers (Helios; Fluidigm Corp.). With use of EQ 4 element calibration beads (Fluidigm Corp.), temporal signal intensities were normalized by CyTOF software (Fluidigm Corp.).

The acquired data were uploaded on a Cytobank Web Server 8.0 (Cytobank, Inc.), and processed to gate out dead cells, duplications, and normalization beads.

(10) MALDI-imaging mass spectrometry

MALDI-imaging mass spectrometry was performed in accordance with a previous report (NPL 2) with a 7T FT-ICR-MS (Solarix Bruker Daltonik GmbH) equipped with an Ultraflextreme MALDI-TOF/TOF mass spectrometer and an Nd:YAG laser. Data were acquired in a negative reflectron mode by raster scanning at a pitch distance of 50 μm. Each spectrum was a result of laser shooting 300 times per data point. In TOF/TOF measurement, signals of m/z = 50 to 1000 were collected. In FT-ICR-MS imaging, signals of m/z = 300 to 500 were collected with use of continuous accumulation in a selected ion mode. Image reconstruction from them were performed with FlexImaging 4.1 software (Bruker Daltonics GmbH). Molecular identification was performed with FT-ICR-MS data. The data allow selective ion signals from metabolites to be acquired in a 5 p.p.m. mass window by virtue of high mass precision provided by the FT-ICR-MS, and enable identification of a specific elemental composition in compounds by referring to a database for very accurate mass.

(11) Immunohistochemistry

For clinical specimens, each tissue sample was fixed with neutral buffer containing 10% formalin for 5 minutes, and incubated with EDTA buffer (pH 9.0) for 10 minutes. After endogenous peroxidase was inactivated in methanol containing 0.3% $H_2O_2$ for 15 minutes, the resulting section was incubated first with anti-human CD8 (C8/144B, 1:100; Nichirei Biosciences Inc.) and then reacted with ImmPRESS (R) (anti-mouse IgG polymer detection kit; Vector Laboratories, Inc.), and visualized with a DAB Buffer tablet (Muto Pure Chemicals CO., LTD.).

For transplantation model animals, each tissue sample was fixed with 100% ethanol for 1 minute and with 4% paraformaldehyde for 5 minutes, and washed three times with PBS. After incubating with PBS containing 5% BSA, the section was reacted with anti-mouse CD8 (YTS169.4, 1:200; Abeam) and with an Alexa Fluor546-labeled goat anti-rat IgG (H+L) antibody (Thermo Fisher Scientific). Nuclei were stained with Hoechst (1:2000; Thermo Fisher Scientific).

(12) Quantification of CS and oxysterol by mass spectrometry

CS in each human cancer tissue sample was quantified by a method described in NPL 2. To monitor the amounts of CS and/or oxysterol generated in E0771, 3LL, EG.7-OVA, and MC38 cells irrespective of the presence or absence of SULT2B1b expression, cells of each type were seeded in a 6-well plate at $5 \times 10^5$ cells/well, and the culture supernatant and cell pellet were collected after 24 hours. Subsequently, deuterium-labeled internal standard (IS) compounds (d7-CS [d7-cholesterol] and d6-25-hydroxycholesterol) were added to fractions of the culture supernatant and cell pellet, each of which was subjected to extraction with 1 mL of extraction solvent (methanol/chloroform/water = 5:2:2 [v/v/v]) to extract CS and oxysterol from the culture supernatant or cell pellet.

Each sample was vigorously mixed with a vortex for 1 minute, and then ultrasonicated for 5 minutes. Thereafter, the sample was centrifuged at 16,000 ×g and 4°C for 5 minutes, and the supernatant (700 μl) was collected in a clean tube. Into this, 235 μl of chloroform and 155 μl of water were added and mixed, and the resultant was then subjected to vortexing followed by centrifugation to separate into an aqueous layer and an organic layer. Subsequently, 200 μl of the lowermost layer was dried under a nitrogen flow.

The dried sample was dissolved in 50 μl of methanol, and CS and oxysterol therein were quantified by liquid chromatography-tandem mass spectrometry (LC/MS/MS) with a triple quadrupole mass spectrometer (LCMS-8060; Shimadzu Corporation) equipped with an electrospray ionization (ESI) ion source in a multiple reaction monitoring (MRM) mode.

[Conditions for LC/MS/MS analysis]

Column: Inertsil ODS-4 (2.1 × 150 mm; particle size: 3 μm; GL Sciences Inc.)
Column temperature: 40°C

Flow rate: 0.3 ml/min
Mobile phase: (A) water/acetonitrile (1:2, v/v) containing 5 mM ammonium acetate
(B) methanol/isopropanol (1:19, v/v) containing 5 mM ammonium acetate
Gradient curve: 0 min -> 22 min: (B) 0% -> 100%,

22 min -> 27 min: (B) 100%,
27 min -> 27.1 min: (B) 100% -> 0%,
27.1 min -> 30 min: (B) 0%

Injection volume: 5 μl
Mass spectrometry mode: positive and negative ion modes
Electrospray voltage: 4 kV in positive ion mode, -3.5 kV in negative ion mode
Nebulizer gas flow rate: 3.0 L/min
Drying gas flow rate: 10.0 L/min
Desolvation temperature: 250°C
Heat block temperature: 400°C
Detector voltage: 2.16 kV
Used were MRM mode and 2 msec of dwell time per channel.

Absolute CS and oxysterol contents were calculated from calibration curves. MRM calibration curves were prepared with chromatographic peak areas for the analytes relative to peak areas for internal standards through three times of analysis for standard solutions.

(13) Compounds and screening

With more than 69,000,000 commercially available compounds selected from a merged product of chemicals supplier data including BIOVIA Available Chemicals Directory (ACD) and catalogs provided from Kishida Chemical Co., Ltd., Namiki Shoji Co., Ltd., and Sigma-Aldrich Japan, ligand-based drug design (LBDD) and structure-based drug design (SBDD) were carried out by in silico screening. In the LBDD, similarity search was performed with three different types of fingerprints (MDL public key, ECFP4, and GpiDAPH3) and ROCS (OpenEye Scientific Software Inc.). In the SBDD, docking calculation with Glide (Schrodinger, LLC, New York) was performed for a substrate-binding site of the crystal structure of SULT2B1b (PDB: 1Q20). Compounds to be analyzed were selected from the results of the in silico screening by visual inspection, and commercially purchased.

(14) SULT assay

For screening for a SULT2B1b inhibitor, enzymatic activity measurement for SULT2B1b was carried out by a method described in a previous report (NPL 2) with slight modifications.

The reaction solution (50 μl) was composed of 1 to 5 μg of recombinant human SULT2B1b protein, 10 μM [$^3$H]cholesterol (approximately 4,000 dpm/pmol), 10 mM Tris-HCl (pH 7.5, containing 5 mM $MgCl_2$) containing 0.1 mM 3'-phosphoadenosine 5'-phosphosulfate (PAPS), 0.2 mM 2-hydroxypropyl-β-cyclodextrin, and 5% (v/v) ethanol. Recombinant human SULT2B1b protein was preincubated with 1 μl of a test compound (final concentration: 40 or 200 μM) or DMSO (control) at room temperature for 15 minutes, 2 mM PAPS (2.5 μl) was then added to initiate reaction, the resultant was reacted at 37°C for 15 minutes, and 0.8 ml of ethanol was added to terminate the reaction.

The solution after the reaction was subjected to thin-layer chromatography (TLC) for separation, and then to a liquid scintillation counter to quantify radiolabeled CS.

From the percentage to the amount (100%) of radiolabeled CS generated through reaction in the absence of the test compound (DMSO) (control), the SULT2B1b activity (%activity) of SULT2B1b protein in the presence of the test compound was evaluated, and the effect of the test compound to inhibit SULT2B1b-mediated CS production (CS production inhibitory effect) was evaluated.

Hit compounds identified in the SULT assay were used as query compounds for similarity search for a more effective compound. As a result, cholenoic acid (CAS No. 5255-17-4) was selected as an effective compound.

(15) Quantification and statistical analysis

Statistical analysis was performed with Prism 9.0 (GraphPad Software, Inc.). Data were first tested by Kolmogorov-Smirnov test for normal distribution. Parametric data were analyzed by two-sided unpaired Student t test in comparing two groups. Nonparametric data were analyzed by two-sided Mann-Whitney test in comparing two groups.

Statistical differences among three or more experimental groups were evaluated by analysis of variance (ANOVA) and Dunnett's post-hoc test. P values of less than 0.05 were regarded as being significant.

III. Experimental results

(1) CS production and the infiltration of $CD8^+$ T cells inversely correlate in human cancer tissues (see Figure 1 and the description thereof)

A public Human Protein Atlas database (https://www.proteinatlas.org/ENSG00000088002-SULT2B1/pathology) shows that Sult2b1 is highly expressed in many types of tumors including colon cancer and prostate cancer (see Figure 1A). Mass spectrometry indeed made it clear that CS was abundantly produced in cancer tissues of colon cancer and prostate cancer (see Figure 1B). Importantly, the production level of CS (high CS production region and low CS production region) inversely correlated with the infiltration of $CD8^+$ T cells in the same specimen derived from colon cancer tissue (see Figure 1C). In contrast to this, almost no CS production was detected in renal cancer, which is known to be susceptible to immune checkpoint inhibitors (NPL 9) (see Figure 1B).

(2) CS production in tumor tissues inhibits the infiltration of effector T cells (see Figures 2 and 3, and the descriptions thereof)

To functionally examine the role of CS in tumor immune evasion, SULT2B1b was expressed in the mouse breast cancer cell line E0771 lacking endogenous expression of SULT2B1b (see Figure 2). Figure 2 shows the presence or absence of expression of SULT2B1b in different tumor cell lines used in the present experiment.

The cell line E0771 that expressed SULT2B1b (hereinafter, referred to as "E0771-SULT" or "E0771-SULT cells") produced and secreted a large amount of CS (see Figure 3A). While expression of SULT2B1b did not affect the growth of E0771 cells in vitro (see Figure 3B), the growth of E0771-SULT cells transplanted into C57BL/6 mice was far faster than the growth of E0771-control cells without expression of SULT2B1b (see Figure 3C). A cytometer in CyTOF analysis revealed that the number of infiltrative $CD8^+$ T cells and granzyme expression thereby were less in E0771-SULT cells (Figures 3D and 3E), suggesting that CS production affects anti-tumor T-cell response. In fact, the difference in tumor growth between E0771-SULT cells and E0771-control cells as found for the transplantation into C57BL/6 mice was not found for transplantation into nude mice (see Figure 3F).

(3) Tumor cells exhibit resistance to immune checkpoint inhibition through CS production (see Figure 4 and the description thereof)

The interaction between programmed cell death 1 (PD-1) and programmed cell death ligand 1 (PD-L1) is one of the most marked targets of immune checkpoint inhibition (NPL 1). No difference in expression of PD-L1 in immunocytes including $CD11b^+$ antigen-presenting cells was found between E0771-SULT and E0771-control for transplantation into C57BL/6 mice (see Figure 4A and Figure 4B). To focus on the effector phase of the T cell functions, E0771-SULT cells and E0771-control cells were forced to express PD-L1 (E0771-SULT-PD-L1 cells and E0771-control-PD-L1 cells). FACS profiles confirmed that the two types of cells exhibited the same degree of PD-L1 expression. Through anti-PD-L1 antibody treatment, the in vivo growth of E0771-control-PD-L1 cells in C57BL/6 mice was significantly inhibited as compared with that with treatment with a control of the matched isotype (Isotype) (see Figure 4C). On the other hand, E0771-SULT-PD-L1 cells, which exhibited surface PD-L1 expression comparable to that exhibited by E0771-control-PD-L1 cells, nevertheless, did not respond to anti-PD-L1 antibody therapy (see Figure 4D). From this, CS production in cancer cells is inferred to render cancer cells resistant to immune checkpoint inhibition.

(4) SULT2B1b-mediated CS production in tumors weakens anti-tumor T-cell response (see Figure 5 and the description thereof)

The OTII T cell receptor (TCR) recognizes an OVA peptide (OVA323-339) presented by an MHC class II I-$A^b$ molecule. To precisely examine the role of CS in anti-tumor T-cell response, an I-$A^b$ molecule covalently bound to OVA323-339 (I-$A^b$/OVA complex) was expressed on E0771-SULT cells and E0771-control cells (E0771-SULT-OVA and E0771-control-OVA) as an "artificial tumor-specific antigen" (see Figure 5A). $CD4^+$ T cells of an OTII TCR transgenic mouse were activated in vitro and intravenously injected into C57BL/6 mice on day 9 after transplantation of E0771-control-OVA cells, and the result showed that tumor growth was remarkably suppressed (see Figure 5B). By contrast, transplantation of E0771-SULT-OVA cells did not cause the inhibition of tumor growth, and the number of tumor-infiltrative OTII $CD4^+$ T cells was as low as less than 36.5% of that for the transplantation of E0771-control-OVA cells (see Figure 5B). The same protocol was applied to the mouse Lewis lung cancer cell line 3LL expressing the I-$A^b$/OVA complex

(SULT2B1b-expressing cells: 3LL-SULT-OVA, SULT2B1b-nonexpressing cells: 3LL-control-OVA), and the same results were obtained (Figure 5C, Figure 5D).

Additionally, the role of CS in cancer-specific $CD8^+$ T cells was analyzed with E.G7-OVA, which was developed by introducing a gene encoding full-length OVA into the mouse T-cell lymphoma cell line EL4 (NPL 10). For E.G7-OVA, OVA257-264 peptide is presented by an MHC class I H-$2K^b$ molecule on the surface through antigen processing of OVA protein, and recognized by $CD8^+$ T cells of an OTI TCR transgenic mouse (NPL 11). $CD8^+$ T cells of an OTI TCR transgenic mouse were activated in vitro and intravenously injected into C57BL/6 mice on day 10 after transplantation of E.G7-OVA, and the results showed that tumor growth was suppressed (see Figure 5D). By contrast, E.G7-OVA cells expressing SULT2B1b (E.G7-OVA-SULT) exhibited resistance to that treatment, and the proportion of tumor-infiltrative OTI $CD8^+$ T cells was small (see Figure 5D).

These results revealed that CS production in tumor cells diminishes anti-tumor T-cell response.

(5) Expression of SULT2B1b suppresses the in vivo growth of tumor cells that produce oxysterol such as 25-HC (see Figure 6 and the description thereof)

Oxysterol such as 25-hydroxycholesterol (25-HC) is an oxidated derivative of cholesterol, and regulates expression of a wide range of metabolic and inflammatory genes via liver X receptor (LXR) signaling. In contrast to E0771 cells, 3LL cells, and E.G7-OVA cells, the mouse colon cancer cell line MC38 highly expresses cholesterol 25-hydroxylase (CH25H) (see Figure 6A), being responsible for conversion of cholesterol into 25-HC. It was revealed that in vivo tumor growth was not promoted but rather suppressed in MC38 cells expressing SULT2B1b (MC38-SULT cells) (see Figure 6B). SULT2B1b is an enzyme known to inactivate oxysterol through sulfation of the oxysterol (NPLs 12, 13). Mass spectrometry revealed that the production of 25-HC disappeared but the production level of 25-hydroxycholesterol-3-sulfate (25-HCS) was remarkably high in MC38-SULT cells (Figure 6C). To examine whether 25-HC had direct influence on tumor growth, MC38 cells were treated with culture supernatants of various MC38 derivatives. While supernatant of wild-type MC38 cells (MC38-control-Sup) promoted the growth of MC38 cells in vitro, no cell growth was observed for culture supernatant of MC38-SULT (MC38-SULT-Sup) and culture supernatant of MC38-ΔCH25H with expression of CH25H genetically deleted (MC38-ΔCH25H-Sup) (see Figure 6D). More importantly, it was revealed that expression of SULT2B1b significantly enhanced tumor growth in C57BL/6 mice into which MC38-ΔCH25H cells had been transplanted (Figure 6E). This result suggests the possibility that SULT2B1b inhibition is applicable as a cancer treatment strategy against cancer cells lacking oxysterol production capacity. On the other hand, the public Human Protein Atlas database (aforementioned) shows that expression of an oxysterol-producing enzyme is limited to some cancer types, and generally low in many cancer types expressing Sult2b1 (Figure 1A vs. Figure 7). This suggests that SULT2B1b inhibition can be an effective cancer treatment strategy.

(6) Identification of cholenoic acid as a SULT2B1b inhibitor that promotes anti-tumor immunity (see Figures 8 and 9, and the descriptions thereof)

To develop an SULT2B1b inhibitor, more than 69,000,000 commercially available compounds were screened in silico, and 442 candidate compounds having abilities to inhibit in vitro CS conversion (sulfation of cholesterol) and CS production in E0771-SULT cells were analyzed. Through a series of analyses, 3β-hydroxy-Δ5-cholenoic acid (cholenoic acid; see Figure 8A) (compound 1) was found out. SULT assay confirmed that cholenoic acid inhibited the CS production by E0771-SULT cells at $IC_{50}$ 5.8 μM (Figure 8B).

Oral administration of cholenoic acid to mice with transplanted E0771-SULT cells for 3 days resulted in local reduction in CS production around a region in which the infiltration of $CD8^+$ T cells was detected (CS low region: see Figure 8C) (see Figure 9). While E0771-SULT-PD-L1 cells exhibited no response to anti-PD-L1 antibody treatment as described above (Figure 4D), it was confirmed that oral administration of cholenoic acid renders E0771-SULT-PD-L1 cells susceptible to immune checkpoint inhibition (Figure 8D). In addition, oral administration of cholenoic acid to mice with transplanted E0771-SULT-OVA cells or E.G7-OVA-SULT cells resulted in recovery of the effect of OTII cell or OTI T cell transplantation, and the anti-tumor immunity of OTII or OTI T cells was promoted (Figures 8E and 8F).

These results confirmed that cholenoic acid promotes anti-tumor immunity by inhibiting CS-mediated T-cell exclusion. Moreover, the results of the OTII cell/OTI T cell transfer experiment shown in Figures 8E and 8F suggest that oral administration of cholenoic acid exerts a synergistic effect (even) when combined with an activated T-cell therapy such as CAR-T therapy.

(7) Cholenoic acid inhibits the activity of SULT2B1b even at a cell level

"Trans-cancer" migration assay found that the presence of E0771-SULT cells (SULT+) resulted in reduction of the migration of T cells induced by CCL21 to 63.5% of that in the presence of E0771-MOCK cells (Figure 9A). This

suppression effect was not observed in the case that E0771-SULT cells were pretreated with cholenoic acid (Figure 9A). In carrying out the migration assay, T cells were loaded on E0771-MOCK cells (expressing only a vector; SULT-) or E0771-SULT cells (SULT+) seeded on Matrigel in an upper chamber.

In enzyme assay, SULT assay was performed with use of cholenoic acid as a compound to be tested at different cholesterol and PAPS concentrations (wherein 0.5 to 1 μg of recombinant human SULT2B1b protein was used, the preincubation time and reaction time were each 10 minutes), and the results were fitted to the Michaelis-Menten equation by using Prism 9 (GraphPad Software, Inc.) to perform enzyme kinetic analysis for the inhibition by cholenoic acid. The results of this analysis showed that cholenoic acid inhibited the sulfotransferase activity of SULT2B1b at $IC_{50}$ 30.3 ± 8.1 M (n = 3) non-competitively with cholesterol (substrate) and uncompetitively with PAPS (sulfate donor) (Figure 9B, Figure 9C). These results indicate that cholenoic acid is an inhibitor for SULT2B1b that is effective even at a cell level.

Experiment Example 2: Evaluation of SULT2B1b inhibitory effect, CS production inhibitory effect, and toxicity of compound group represented by formula (I), including compound 1

Compound 1 (cholenoic acid), compounds 2 to 19, 21, 23, 25, and 27 produced by the methods described in Production Examples 1 to 12, 14, 16, 18, and 20, and commercially available compounds 28 to 32 were evaluated on SULT2B1b inhibitory effect (only for some of the compounds), CS production inhibitory effect, and toxicity by the following methods.

(1) Evaluation of SULT2B1b inhibitory effect

SULT assay was performed for the compounds as test compounds by the method described in (14) of "II. Experimental procedure" in Experiment Example 1 to evaluate the SULT2B1b inhibitory effects of the compounds.

(2) Evaluation of CS production inhibitory effect

The CS production effects of test compounds (compounds 1 to 19, 21, 23, 25, and 27 to 32) were evaluated with the mouse breast cancer cell line E0771 expressing SULT2B1b (E0771-SULT cells) .

Specifically, as in the above description in experimental procedure (12), E0771-SULT cells were seeded in a 6-well plate at a proportion of $5 \times 10^5$ cells/well, and cultured in the presence or absence (control) of each test compound for 24 hours. After culturing, CS generated in E0771-SULT cells was quantified by LC/MS/MS according to the description in experimental procedure (12). The CS production capacity (%activity) of cells cultured in the presence of each test compound was evaluated from the percentage relative to the CS production level (control) (100%) in E0771-SULT cells cultured in the absence of the test compound, and the CS production inhibitory effect of the test compound was evaluated.

(3) Evaluation of toxicity

E0771-SULT cells and E0771-control cells were seeded in a 6-well plate, and the next day, each compound was added at 20 μM when performing medium exchange, and the cells were observed for 24 hours. After 24 hours, if visual observation found that 20% or more of the cells were detached off, the case was determined as being cytotoxic (+); if not, the case was determined as being not cytotoxic (-).

The results are shown in Tables 1-1 to 2. In the tables, a blank indicates no measurement.

[Table 1-1]

| Compound number | Structure | CS production/E0771 | | CS production/E0771 | | CS production/E0771 | | IC50 value | In vitro SULT2B1b assay | IC50 value (enzyme assay) |
|---|---|---|---|---|---|---|---|---|---|---|
| | | % activity at 20 $\mu$M | cytotoxicity | % activity at 10 $\mu$M | cytotoxicity | % activity at 5 $\mu$M | cytotoxicity | (uM) | % activity at 200 $\mu$M | (uM) |
| | Compound groupA | | | | | | | | | |
| Compound 1 | 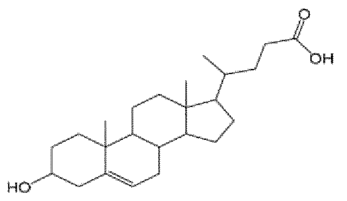 | 30.8 | - | 35.8 | - | 47.1 | - | 5.8 | 20.6 | 24.8 |
| Compound 2 | 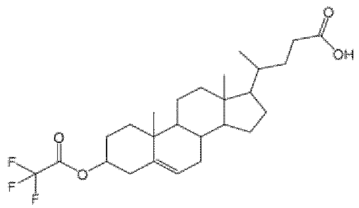 | 36.8 | - | 34.0 | - | 52.5 | - | 6.0 | | |
| Compound 14 | 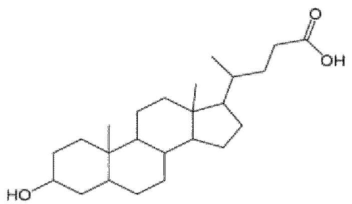 | 31.5 | - | | | 62.0 | - | 7.2 | | |
| Compound 15 | 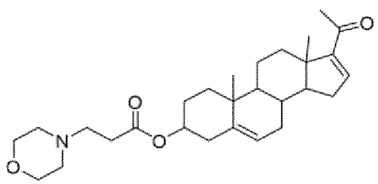 | 38.9 | - | 40.9 | - | 63.0 | - | 7.8 | | |

[Table 1-2]

| | | | | | | | | | | |
|---|---|---|---|---|---|---|---|---|---|---|
| Compound 16 | 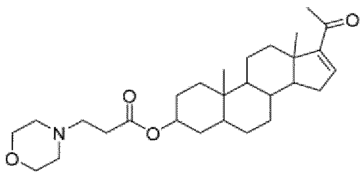 | 42.3 | - | 49.9 | - | 55.7 | - | 9.6 | | |
| Compound 17 | 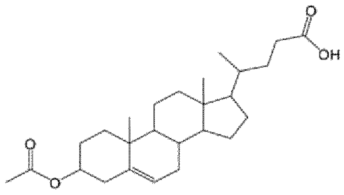 | 52.4 | - | 43.7 | - | 44.9 | - | 7.0 | 66.4 | 186.1 |
| Compound 18 | 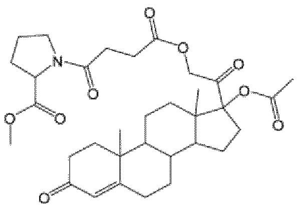 | 54.8 | - | 60.6 | - | 71.7 | - | 16.0 | | |
| Compound 19 | 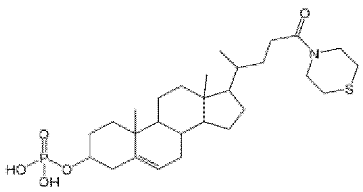 | 52.7 | - | 60.4 | - | 70.8 | - | 19.1 | | |

[Table 1-3]

| | | | | | | | | | | |
|---|---|---|---|---|---|---|---|---|---|---|
| Compound 7 | 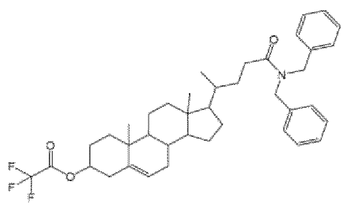 | 57.8 | - | 59.0 | - | 74.6 | - | 17.3 | 6.4 | |
| Compound 3 | 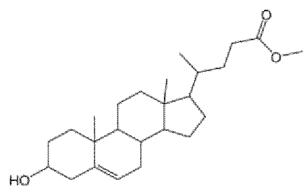 | 56.8 | - | 48.1 | - | 61.4 | - | 17.3 | 19.0 | |
| Compound 4 | 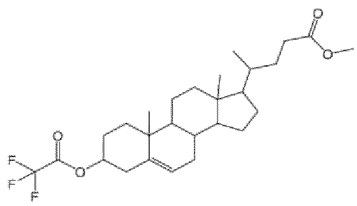 | 49.9 | - | 79.2 | - | 103.5 | - | 20.0 | 13.6 | |

(continued)

| | | | | | | | | | | |
|---|---|---|---|---|---|---|---|---|---|---|
| Compound 6 | 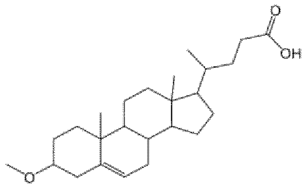 | 70.3 | - | 74.5 | - | 80.7 | - | 32.4 | 22.9 | |
| Compound 8 | 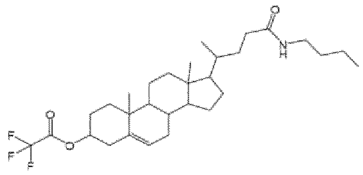 | 60.2 | - | 70.6 | - | 83.6 | - | 42.5 | 10.7 | |

[Table 1-4]

| | | | | | | | | | | |
|---|---|---|---|---|---|---|---|---|---|---|
| Compound 13 | 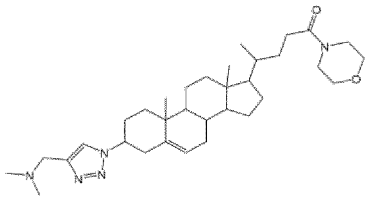 | 65.6 | - | | | | | | | |
| Compound 19 | 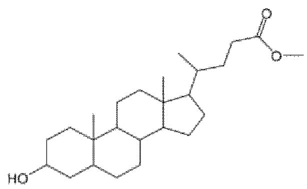 | 68.6 | - | 71.7 | - | 72.8 | - | | | |
| Compound 5 | 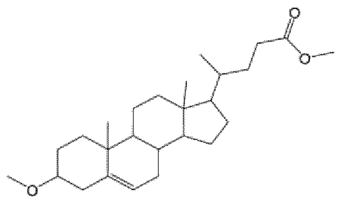 | 92.3 | - | | | | | | 8.3 | 33.1 |
| Compound 12 | 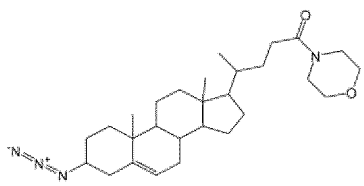 | | | 98.4 | - | | | | | |
| Compound 11 | 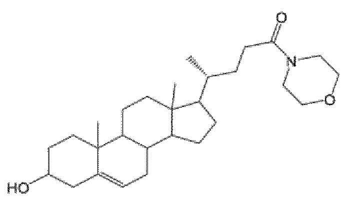 | 87.4 | - | | | | | | | |

[Table 1-5]

| | | | | | | | | | | | |
|---|---|---|---|---|---|---|---|---|---|---|---|
| Compound 9 | 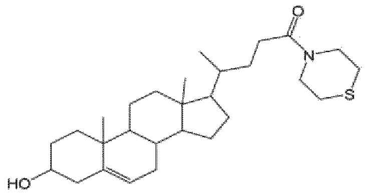 | 87.2 | - | | | | | | | | |
| Compound 21 | 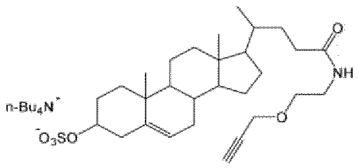 | 85.7 | - | | | | | | | | |
| Compound 23 | 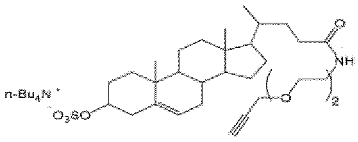 | 70.1 | - | | | | | | | | |
| Compound 25 | 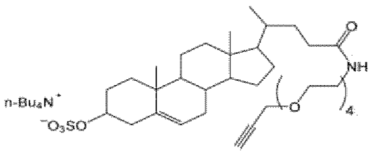 | 60.1 | - | | | | | | | | |
| Compound 27 | 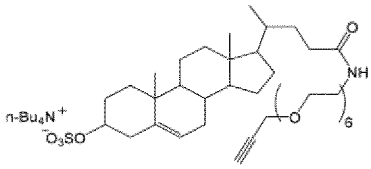 | 59.0 | - | | | | | | | | |

[Table 2]

| Compound number | Structure | CS production/E0771 | | CS production/E0771 | | CS production/E0771 | | IC50 value | In vitro SULT281b assay | IC50 value (enzyme assay) |
|---|---|---|---|---|---|---|---|---|---|---|
| | | % activity at 20 $\mu$M | cytotoxicity | % activity at 10 $\mu$M | cytotoxicity | % activity at 5 $\mu$M | cytotoxicity | (uM) | % activity at 200 $\mu$M | (uM) |
| Compound group B | | | | | | | | | | |
| Compound 28 | 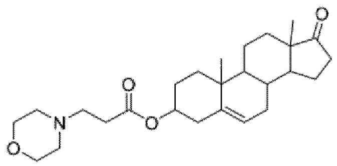 | 42.0 | - | 37.8 | - | 52.5 | - | 6.3 | | |
| Compound group C | | | | | | | | | | |
| Compound 29 | 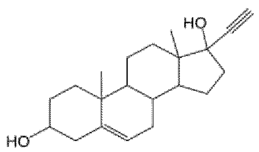 | 45.0 | - | 50.1 | - | 68.3 | - | 11.8 | | |
| Compound 30 | 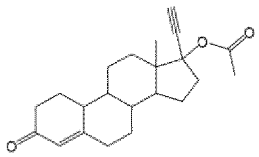 | 61.9 | - | 80.1 | - | 94.5 | - | 34.3 | | |
| Compound 31 | 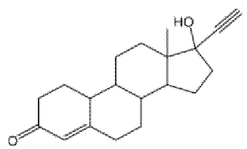 | 77.5 | - | 82.8 | - | 96.9 | - | 68.4 | | |
| Compound group D | | | | | | | | | | |

(continued)

| Compound number | Structure | CS production/E0771 | | CS production/E0771 | | CS production/E0771 | | IC50 value | In vitro SULT281b assay | IC50 value (enzyme assay) |
|---|---|---|---|---|---|---|---|---|---|---|
| | | % activity at 20 $\mu M$ | cytotoxicity | % activity at 10 $\mu M$ | cytotoxicity | % activity at 5 $\mu M$ | cytotoxicity | (uM) | % activity at 200 $\mu M$ | (uM) |
| Compound 32 | 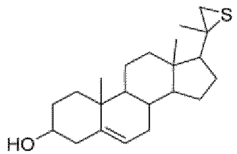 | 54.8 | - | 49.7 | - | 59.4 | - | 12.8 | | |

Sequence Listing Free Text

SEQ ID NO: 1 shows the amino acid sequence of OVA peptide (OVA323-339); SEQ ID NOs: 2 and 3 show the nucleotide sequences of two complementary oligonucleotides containing a target sequence to inactivate CH25H expression and a Bbs I ligation adaptor; SEQ ID NOs: 4 to 7 show the nucleotide sequences of primer sets used for PCR amplification of 5' and 3' homology arms (961 bp and 1084 bp) from a genomic DNA isolated from MC38 cells; and SEQ ID NOs: 8 and 9 show the nucleotide sequences of primer sets for Gapdh.

## Claims

1. A pharmaceutical composition comprising a compound or a salt thereof as an active ingredient, the compound represented by general formula (I) (hereinafter, referred to as "compound I"):

[Formula 1]

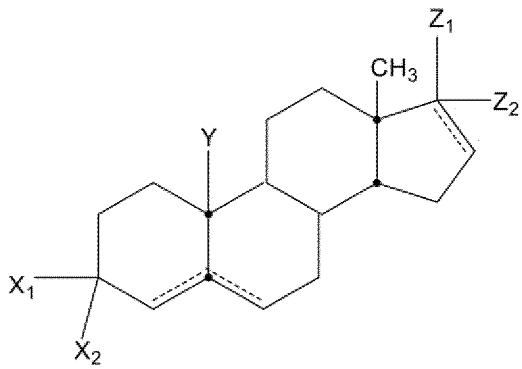

(I)

wherein

one of $X_1$ and $X_2$ is a hydroxy group, a morpholino $C_{1-6}$ alkylcarbonyloxy group, a $C_{1-6}$ alkylcarbonyloxy group, a phosphate group, a $C_{1-6}$ alkoxy group, a di ($C_{1-6}$ alkyl) amino $C_{1-6}$ alkyltriazolyl group, an acetoxy group in which at least one hydrogen atom is optionally replaced with a halogen atom, an azide group, or a sulfate group, and the other is a hydrogen atom, or $X_1$ and $X_2$ are combined to represent an oxo group;
Y represents a $C_{1-6}$ alkyl group or a hydrogen atom;
$Z_1$ and $Z_2$ are different and each represent a group represented by formula (II):

[Formula 2]

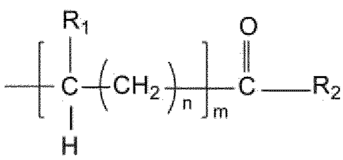

(II)

, a hydroxy group, an ethynyl group, a $C_{1-6}$ alkylcarbonyloxy group, a hydrogen atom, or a heterocyclic group optionally having a substituent, or $Z_1$ and $Z_2$ are combined to represent an oxy group; and
each double line composed of a solid line and a dotted line indicates a single bond or a double bond, wherein signs present in formula (II) are as follows:
$R_1$ is a $C_{1-6}$ alkyl group or a hydrogen atom;
$R_2$ is a hydroxy group, a $C_{1-6}$ alkyl group, a $C_{1-6}$ alkoxy group, a group represented by formula (III):

[Formula 3]

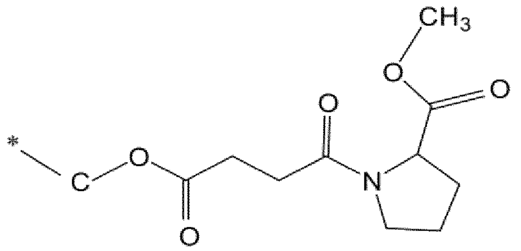

(III)

wherein * indicates a bond to the carbon atom of the carbonyl group in formula (II), or

$-N(R_3)(R_4)$,

wherein $R_3$ and $R_4$ are the same or different, each being a hydrogen atom, a $C_{1-6}$ alkyl group, a phenyl $C_{1-6}$ alkyl group, or a group represented by formula (IV):

[Formula 4]

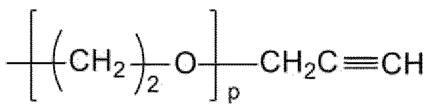

(IV)

wherein p denotes an integer of 1 to 10, and in this case, the other of $R_3$ and $R_4$ is a hydrogen atom, or $R_3$ and $R_4$ are combined to form a heterocyclic ring together with the nitrogen atom;

n is an integer of 0, 1, or 2; and
m is an integer of 1 or 0.

**2.** The pharmaceutical composition according to claim 1, wherein the compound is a compound represented by general formula (I), wherein one of $Z_1$ and $Z_2$ is a group represented by formula (II), and the other is a hydrogen atom or a $C_{1-6}$ alkylcarbonyloxy group; one of $X_1$ and $X_2$ is a hydroxy group, a morpholino $C_{1-6}$ alkylcarbonyloxy group, a $C_{1-6}$ alkylcarbonyloxy group, a phosphate group, a $C_{1-6}$ alkoxy group, an acetoxy group in which at least one hydrogen atom is optionally replaced with a halogen atom, a di($C_{1-6}$ alkyl)amino $C_{1-6}$ alkyltriazolyl group, an azide group, or a sulfate group, and the other is a hydrogen atom; and Y is a $C_{1-6}$ alkyl group.

**3.** The pharmaceutical composition according to claim 1 or 2, wherein the pharmaceutical composition is used as an SULT2B1b inhibitor.

**4.** The pharmaceutical composition according to claim 1 or 2, wherein the pharmaceutical composition is used as an inhibitor for SULT2B1b-mediated cholesterol-3-sulfate production.

**5.** The pharmaceutical composition according to claim 1 or 2, wherein the pharmaceutical composition is used for suppressing growth or progress of a tumor cell that produces cholesterol-3-sulfate.

**6.** The pharmaceutical composition according to claim 1 or 2, being an anti-cancer immunostimulator or an immune checkpoint inhibition potentiator.

**7.** The pharmaceutical composition according to claim 1 or 2, wherein the pharmaceutical composition is used in combination with an anti-cancer agent or an anti-cancer therapy.

**8.** The pharmaceutical composition according to claim 7, wherein the anti-cancer agent is an immune checkpoint inhibitory drug, and the anti-cancer therapy is a cancer immunotherapy.

**9.** The pharmaceutical composition according to claim 8, wherein the immune checkpoint inhibitory drug is an anti-PD-1 antibody or an anti-PD-L1 antibody.

**10.** A compound or a salt thereof, the compound represented by general formula (I):

[Formula 5]

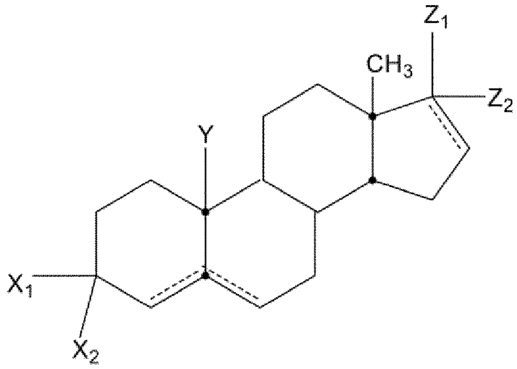

(I)

wherein

one of $X_1$ and $X_2$ is a hydroxy group, a morpholino $C_{1-6}$ alkylcarbonyloxy group, a $C_{1-6}$ alkylcarbonyloxy group, a phosphate group, a $C_{1-6}$ alkoxy group, an acetoxy group in which at least one hydrogen atom is optionally replaced with a halogen atom, a di($C_{1-6}$ alkyl)amino $C_{1-6}$ alkyltriazolyl group, an azide group, or a sulfate group, and the other is a hydrogen atom;
Y is a $C_{1-6}$ alkyl group;
one of $Z_1$ and $Z_2$ is a group represented by formula (II) :

[Formula 6]

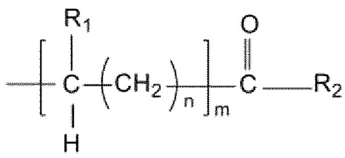

(II)

, and the other is a hydroxy group or a $C_{1-6}$ alkylcarbonyloxy group; and
each double line composed of a solid line and a dotted line indicates a single bond or a double bond, wherein signs present in formula (II) are as follows:

$R_1$ is a $C_{1-6}$ alkyl group or a hydrogen atom;
$R_2$ is a hydroxy group, a $C_{1-6}$ alkyl group, a $C_{1-6}$ alkoxy group, a group represented by formula (III):

[Formula 7]

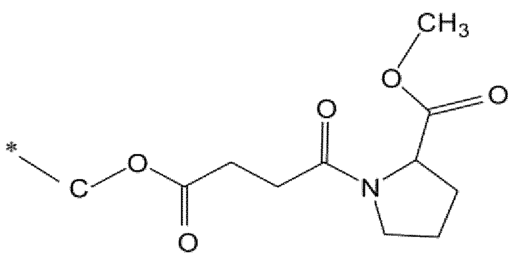

(III)

wherein * indicates a bond to the carbon atom of the carbonyl group in formula (II), or

$-N(R_3)(R_4)$,

wherein $R_3$ and $R_4$ are the same or different, each being a hydrogen atom, a $C_{1-6}$ alkyl group, a phenyl $C_{1-6}$ alkyl group, or a group represented by formula (IV):

[Formula 8]

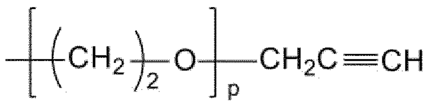

(IV)

wherein p denotes an integer of 1 to 10, and in this case, the other of $R_3$ and $R_4$ is a hydrogen atom, or $R_3$ and $R_4$ are combined to form a heterocyclic ring together with the nitrogen atom;

n is an integer of 0, 1, or 2; and
m is an integer of 1 or 0,
provided that the following compounds are excluded:

(i) (R)-4-((3S,8S,9S,10R,13R,14S,17R)-3-hydroxy-10,13-dimethyl-2,3,4,7,8,9,10,11,12,13,14,15,16,17-tetradecahydro-1H-cyclopenta[a]phenanthren-17-yl)pentanoic acid;
(ii) (4R)-4-[(3R,5R,8R,9S,10S,13R,14S,17R)-3-hydroxy-10,13-dimethyl-2,3,4,5,6,7,8,9,11,12,14,15,16,17-tetradecahydro-1H-cyclopenta[a]phenanthren-17-yl]pentanoic acid;
(iii) (8R,9S,10R,13S,14S)-17-acetyl-10,13-dimethyl-2,3,4,7,8,9,10,11,12,13,14,15-dodecahydro-1H-cyclopenta[a]phenanthren-3-yl 3-morpholinopropanoate;
(iv) (3R,5S,8R,9S,10S,13S,14S)-17-acetyl-10,13-dimethyl-2,3,4,5,6,7,8,9,10,11,12,13,14,15-tetradecahydro-1H-cyclopenta[a]phenanthren-3-yl 3-morpholinopropanoate;
(v) 3-acetoxy-5-cholenoic acid;
(vi) methyl (4-(2-8((8R,9S,10R,13S,14S,17R)-17-acetoxy-10,13-dimethyl-3-oxo-2,3,6,7,8,9,10,11,12,13,14,15,16,17-tetradecahydro-1H-cyclopenta[a]phenanthren-17-yl)-2-oxoethoxy)-4-oxobutanoyl)-L-prolinate; and
(vii) methyl (3$\beta$,5$\alpha$)-3-hydroxycholan-24-oate.

**11.** A method for screening for a compound having an anti-cancer immunostimulating effect and/or an immune checkpoint inhibition potentiating effect, the method comprising the step of:
selecting a compound having a cholesterol-3-sulfate production inhibitory effect from a candidate compound group.

**12.** The method according to claim 11, wherein the step of selecting is at least one selected from the group consisting of an in silico screening step, an SULT assay step, and a quantification step for amounts of CS and/or oxysterol generated in a cell.

**13.** The method according to claim 11 or 12, wherein the CS production inhibitory effect is an effect to inhibit SULT2B1b-mediated cholesterol-3-sulfate production.

[Figure 1]

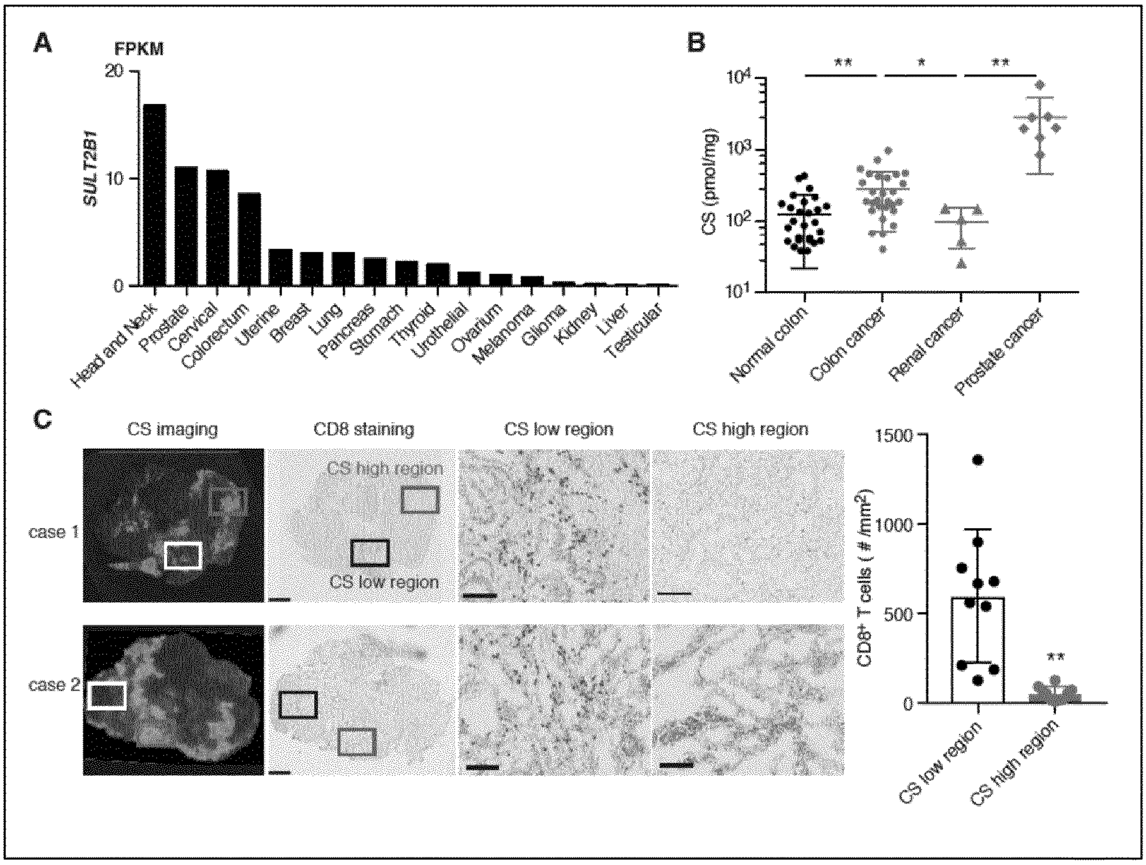


[Figure 2]

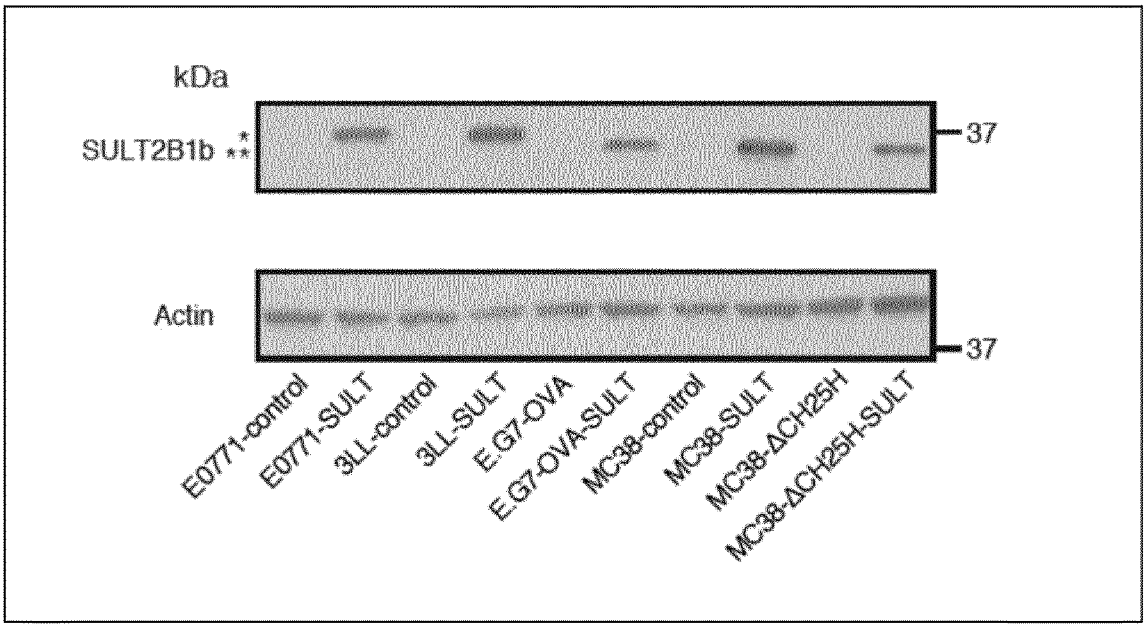

[Figure 3]

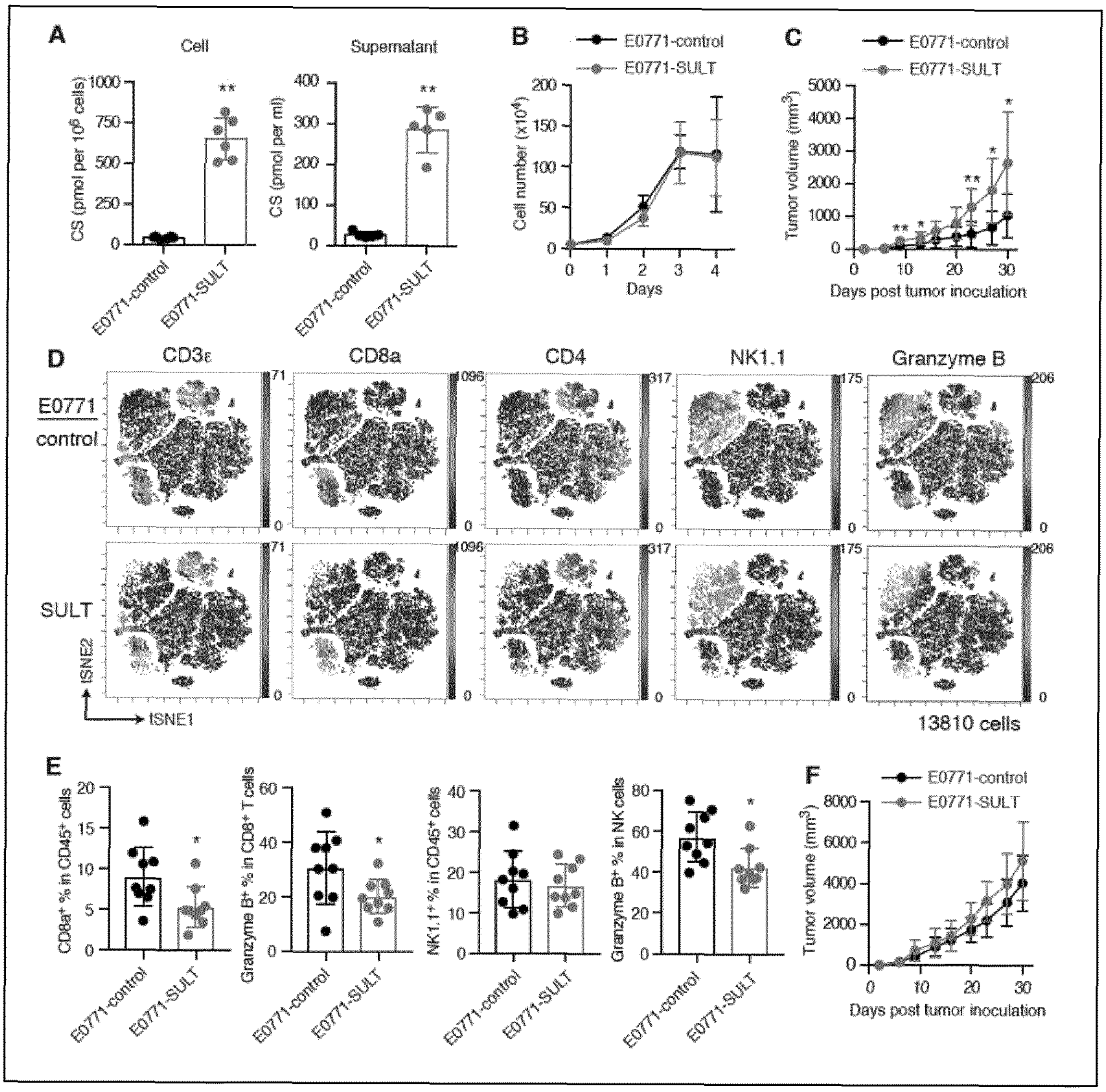
A
Cell
Supernatant
CS (pmol per 10^6 cells)
CS (pmol per ml)
E0771-control
E0771-SULT
B
E0771-control
E0771-SULT
Cell number (x10^4)
Days
C
E0771-control
E0771-SULT
Tumor volume (mm^3)
Days post tumor inoculation
D
CD3ε
CD8a
CD4
NK1.1
Granzyme B
E0771
control
SULT
tSNE2
tSNE1
13810 cells
E
CD8a+ % in CD45+ cells
Granzyme B+ % in CD8+ T cells
NK1.1+ % in CD45+ cells
Granzyme B+ % in NK cells
E0771-control
E0771-SULT
F
E0771-control
E0771-SULT
Tumor volume (mm^3)
Days post tumor inoculation

[Figure 4]

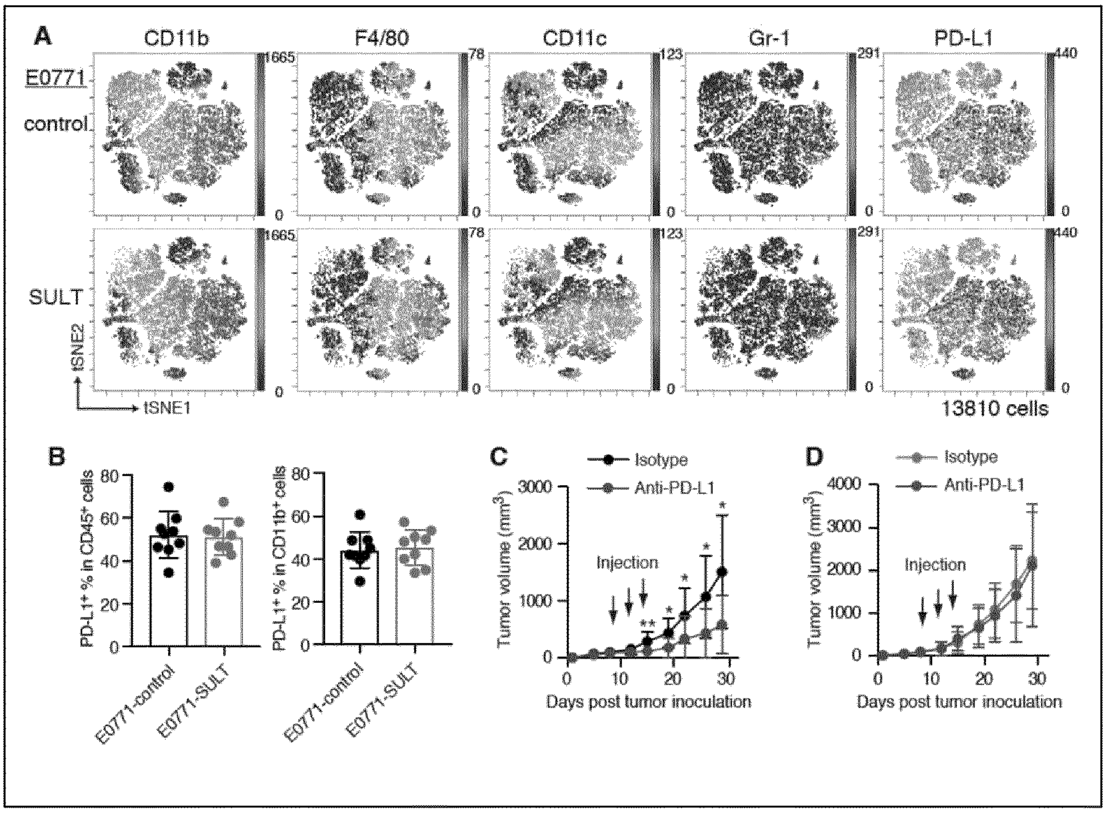


[Figure 5]

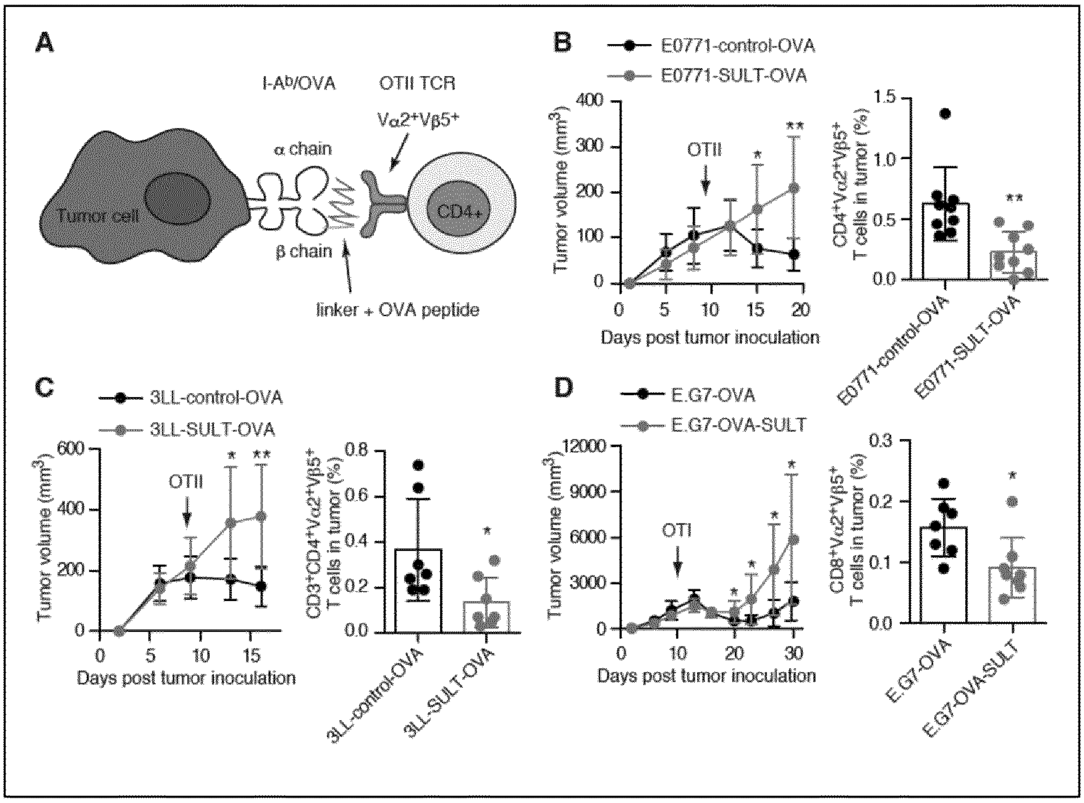

[Figure 6]

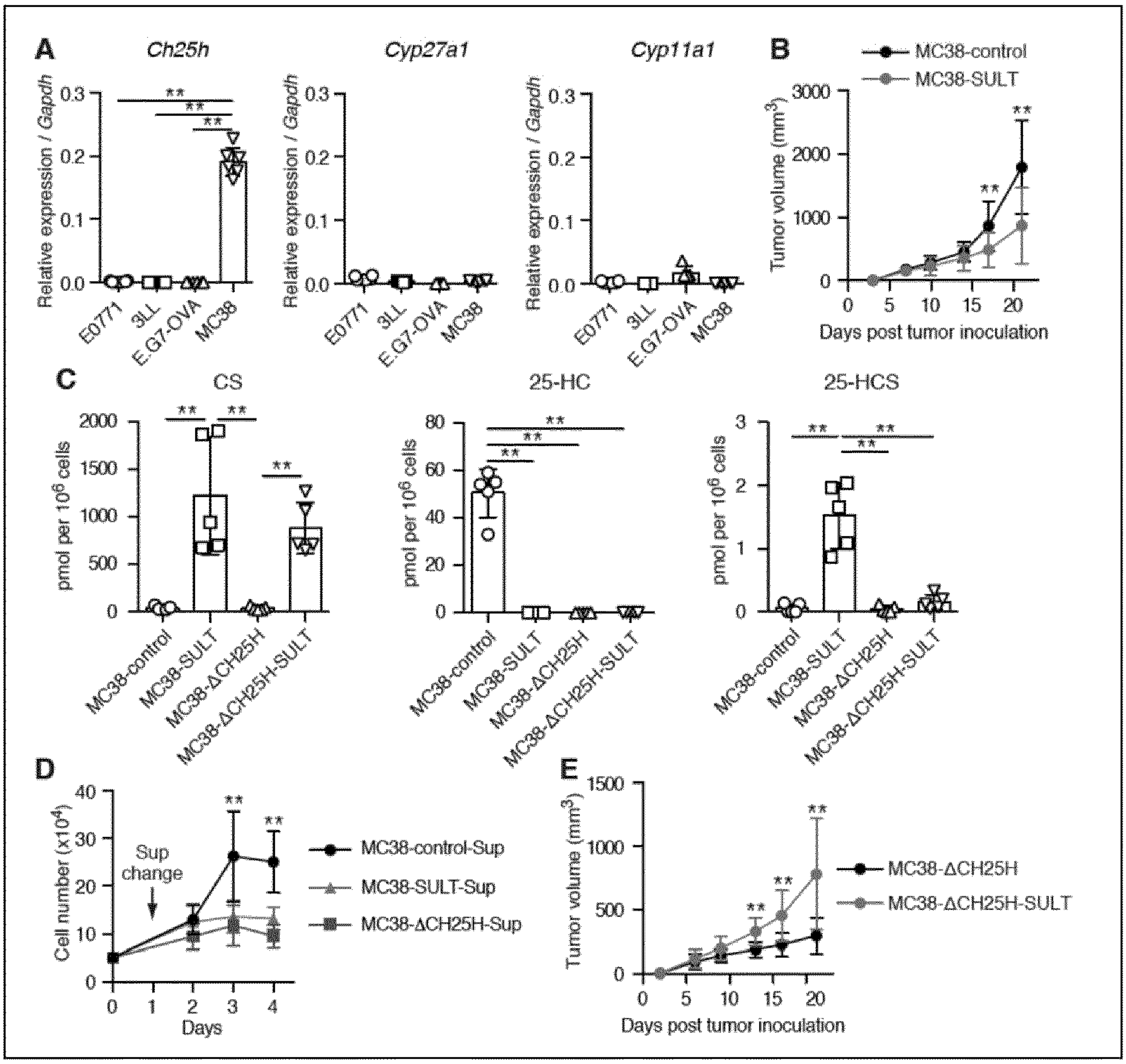
A
Ch25h
Cyp27a1
Cyp11a1
Relative expression / Gapdh
E0771
3LL
E.G7-OVA
MC38
B
MC38-control
MC38-SULT
Tumor volume (mm3)
Days post tumor inoculation
C
CS
25-HC
25-HCS
pmol per 106 cells
MC38-control
MC38-SULT
MC38-ΔCH25H
MC38-ΔCH25H-SULT
D
Sup change
Cell number (x104)
Days
MC38-control-Sup
MC38-SULT-Sup
MC38-ΔCH25H-Sup
E
Tumor volume (mm3)
Days post tumor inoculation
MC38-ΔCH25H
MC38-ΔCH25H-SULT

[Figure 7]

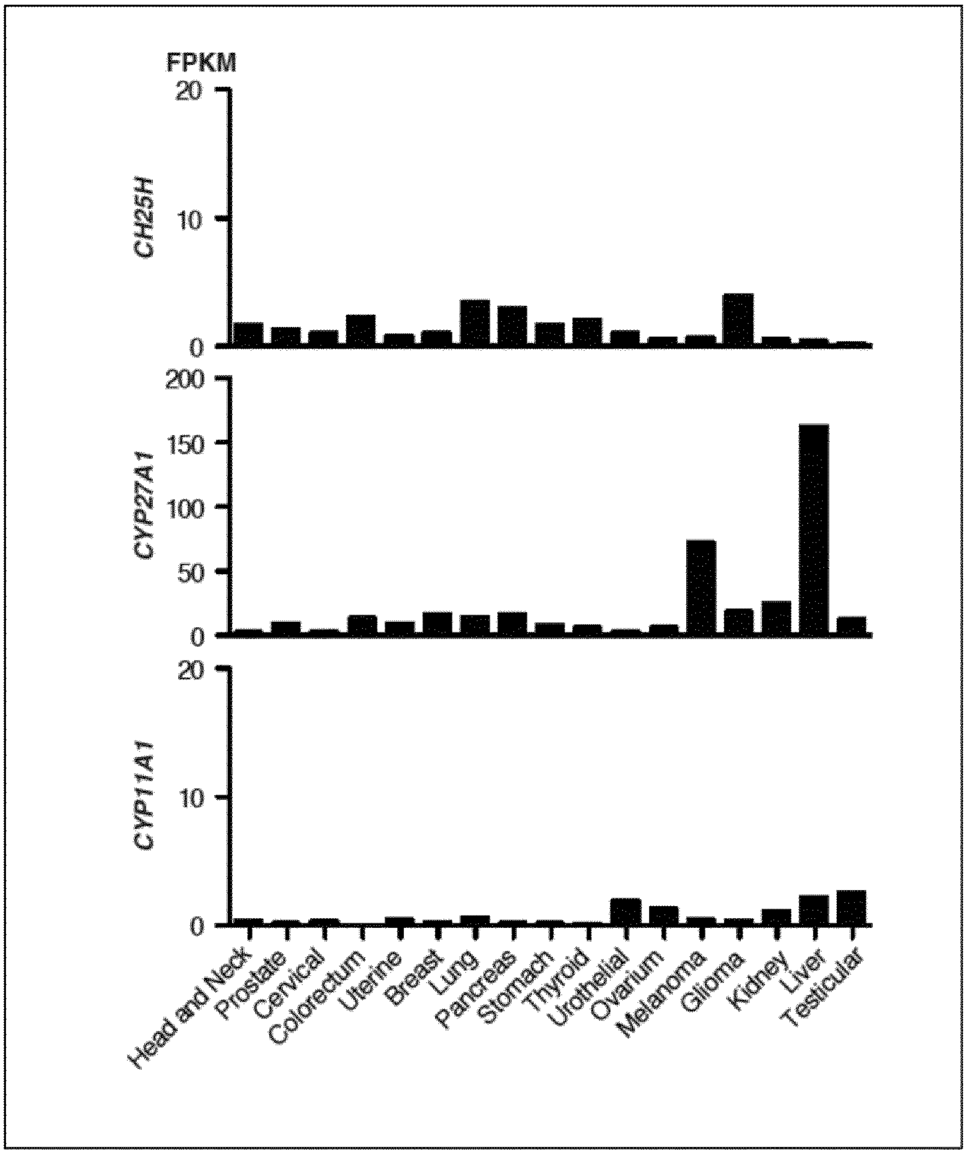
FPKM
CH25H
0
10
20
CYP27A1
0
50
100
150
200
CYP11A1
0
10
20
Head and Neck
Prostate
Cervical
Colorectum
Uterine
Breast
Lung
Pancreas
Stomach
Thyroid
Urothelial
Ovarium
Melanoma
Glioma
Kidney
Liver
Testicular

[Figure 8]

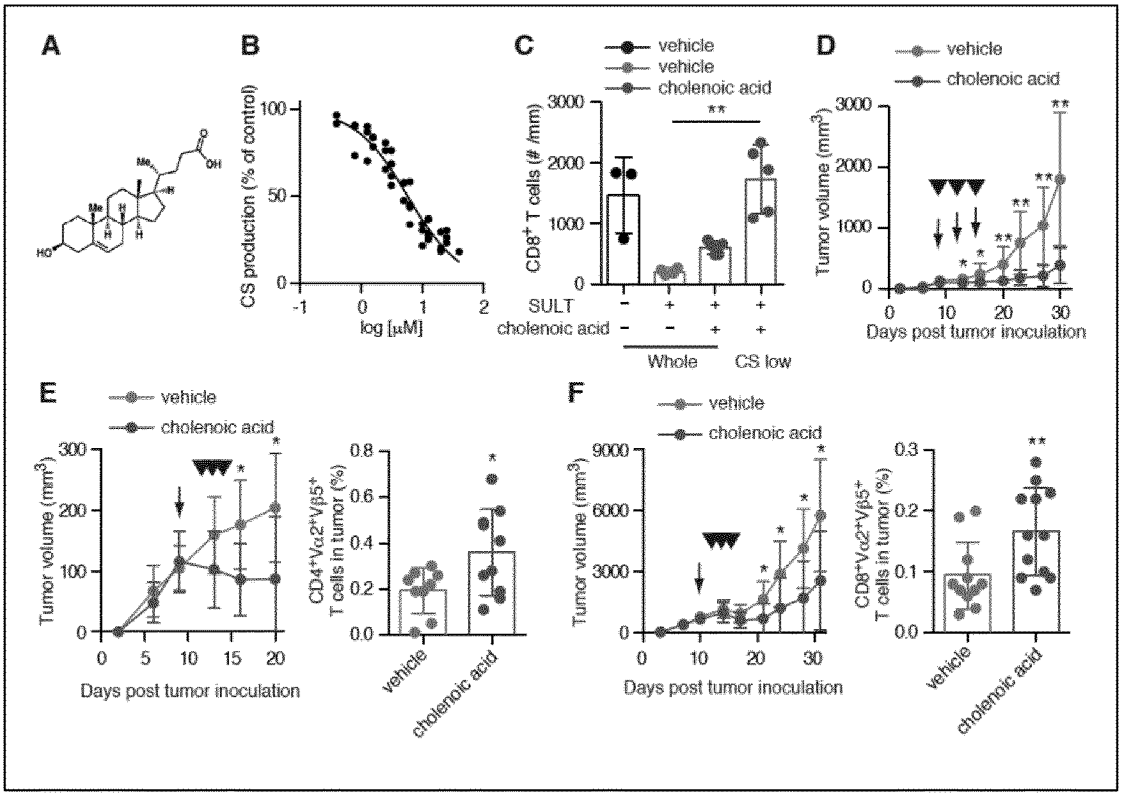


[Figure 9]

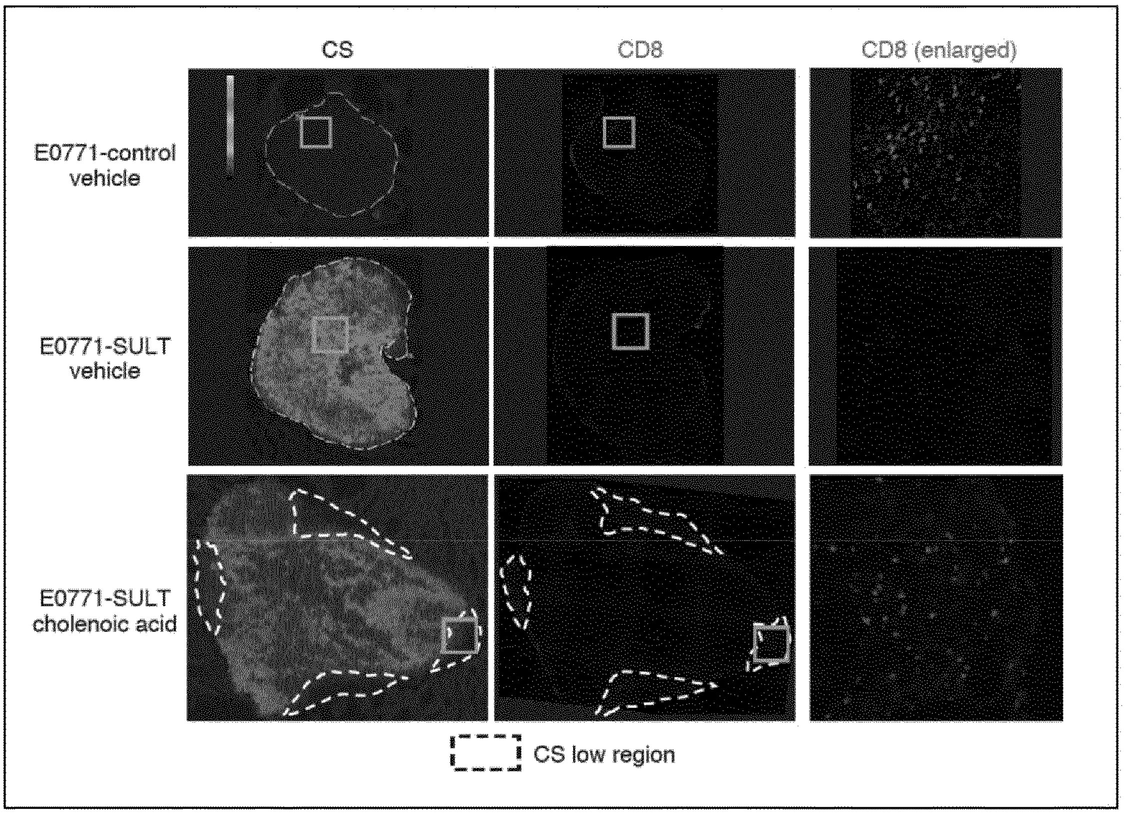

[Figure 10]

A

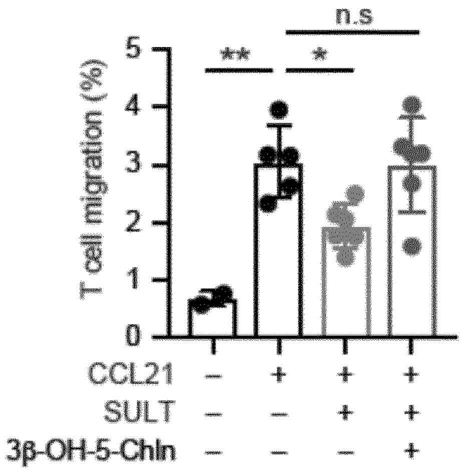


B

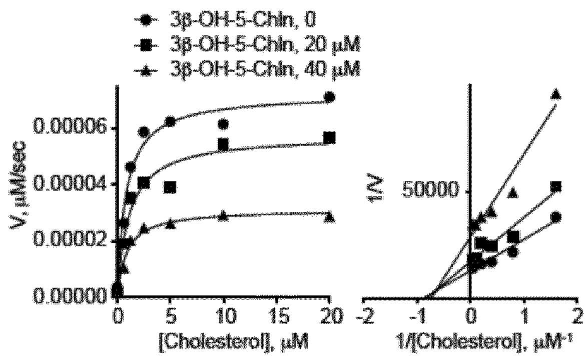


C

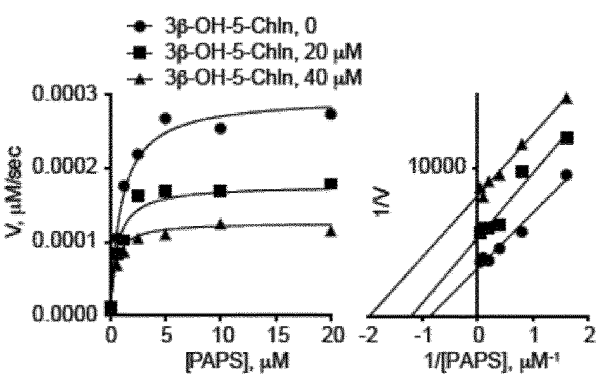

**INTERNATIONAL SEARCH REPORT**

International application No.
**PCT/JP2022/039416**

**A. CLASSIFICATION OF SUBJECT MATTER**

***A61K 31/575***(2006.01)i; ***A61K 31/57***(2006.01)i; ***A61K 31/58***(2006.01)i; ***A61K 39/395***(2006.01)i; ***A61K 45/00***(2006.01)i; ***A61P 35/00***(2006.01)i; ***A61P 37/02***(2006.01)i; ***A61P 43/00***(2006.01)i; ***C07J 9/00***(2006.01)i; ***C07K 16/28***(2006.01)i; ***C12N 15/13***(2006.01)i

FI: A61K31/575 ZNA; A61K31/57; A61P43/00 111; A61P35/00; A61P37/02; A61K45/00; A61K39/395 N; A61K31/58; C07K16/28; C12N15/13; C07J9/00 CSP

According to International Patent Classification (IPC) or to both national classification and IPC

**B. FIELDS SEARCHED**

Minimum documentation searched (classification system followed by classification symbols)

A61K31/575; A61K31/57; A61K31/58; A61K39/395; A61K45/00; A61P35/00; A61P37/02; A61P43/00; C07J9/00; C07K16/28; C12N15/13

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| P, X | TATSUGUCHI, T. et al., Pharmacological intervention of cholesterol sulfate-mediated T cell exclusion promotes antitumor immunity, Biochemical and Biophysical Research Communications, 10 April 2022, 609, pp. 183-188<br>entire text | 1-13 |
| P, A | TATSUGUCHI, T. et al., Cancer-derived cholesterol sulfate is a key mediator to prevent tumor infiltration by effector T cells, International Immunology, 30 January 2022, 34(5), pp. 277-289<br>entire text | 1-13 |
| X | CN 108727453 A (EAST CHINA UNIVERSITY OF SCIENCE AND TECHNOLOGY) 02 November 2018 (2018-11-02)<br>entire text | 1-9 |
| X | JP 2003-342176 A (JAPAN SCIENCE AND TECHNOLOGY CORP.) 03 December 2003 (2003-12-03)<br>entire text | 1, 2 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 November 2022** | **13 December 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
PCT/JP2022/039416

C. DOCUMENTS CONSIDERED TO BE RELEVANT

| Category* | Citation of document, with indication, where appropriate, of the relevant passages | Relevant to claim No. |
|---|---|---|
| X | JP 2002-502423 A (UNIVERSITY OF UTAH RESEARCH FOUNDATION) 22 January 2002 (2002-01-22) entire text | 1 |
| X | JP 2002-514168 A (UNIVERSITY OF UTAH RESEARCH FOUNDATION) 14 May 2002 (2002-05-14) entire text | 1 |
| X | FILE REGISTRY ON STN, RN 1294502-87-6, Entered STN: 13 May 2011 entire text | 10 |
| X | FILE REGISTRY ON STN, RN 1266398-28-0, Entered STN: 04 March 2011 entire text | 10 |
| X | FILE REGISTRY ON STN, RN 1263360-54-8, Entered STN: 22 February 2011 entire text | 10 |
| X | FILE REGISTRY ON STN, RN 1106675-81-3, Entered STN: 16 February 2009 entire text | 10 |
| X | FILE REGISTRY ON STN, RN 881386-65-8, Entered STN: 21 April 2006 entire text | 10 |
| X | FILE REGISTRY ON STN, RN 66674-32-6, Entered STN: 16 November 1984 entire text | 10 |
| X | FILE REGISTRY ON STN, RN 58652-18-9, Entered STN: 16 November 1984 entire text | 10 |
| X | FILE REGISTRY ON STN, RN 30035-16-6, Entered STN: 16 November 1984 entire text | 10 |
| X | FILE REGISTRY ON STN, RN 2266588-81-0, Entered STN: 05 February 2019 entire text | 10 |
| X | FILE REGISTRY ON STN, RN 898284-05-4, Entered STN: 03 August 2006 entire text | 10 |
| X | FILE REGISTRY ON STN, RN 883879-91-2, Entered STN: 11 May 2006 entire text | 10 |
| X | FILE REGISTRY ON STN, RN 75883-02-2, Entered STN: 16 November 1984 entire text | 10 |
| X | FILE REGISTRY ON STN, RN 67052-83-9, Entered STN: 16 November 1984 entire text | 10 |
| X | WO 2017/086436 A1 (KYUSHU UNIVERSITY) 26 May 2017 (2017-05-26) entire text | 11-13 |

Form PCT/ISA/210 (second sheet) (January 2015)

**INTERNATIONAL SEARCH REPORT**

International application No.
**PCT/JP2022/039416**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

The "form of an Annex C/ST.25 text file" is replaced with the "form of ST.26."

Form PCT/ISA/210 (continuation of first sheet) (January 2015)

**INTERNATIONAL SEARCH REPORT**
**Information on patent family members**

International application No.
**PCT/JP2022/039416**

| Patent document cited in search report | Publication date (day/month/year) | Patent family member(s) | Publication date (day/month/year) |
|---|---|---|---|
| CN 108727453 A | 02 November 2018 | (Family: none) | |
| JP 2003-342176 A | 03 December 2003 | (Family: none) | |
| JP 2002-502423 A | 22 January 2002 | US 5977095 A<br>entire text<br>US 5753640 A<br>US 5846963 A<br>US 5635496 A<br>US 5686438 A<br>US 5811418 A<br>US 5587369 A<br>US 5532230 A<br>US 5859000 A<br>US 6150348 A<br>US 6187767 B1<br>WO 1998/055074 A2<br>EP 1011607 A2 | |
| JP 2002-514168 A | 14 May 2002 | US 5929060 A<br>entire text<br>US 5922701 A<br>US 5686438 A<br>US 5532230 A<br>WO 1998/005338 A2<br>EP 915702 A2 | |
| WO 2017/086436 A1 | 26 May 2017 | US 2018/0325918 A1<br>entire text<br>US 2022/0054506 A1<br>EP 3378492 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017086436 A

### Non-patent literature cited in the description


- *CHEMICAL ABSTRACTS,* 1256-86-6
- *CHEMICAL ABSTRACTS,* 5255-17-4
- **A RIBAS ; J. D. WOLCHOK.** Cancer immunotherapy using checkpoint blockade. *Science,* 2018, vol. 359, 1350-1355
- **T. SAKURAI ; T. URUNO ; Y. SUGIURA ; T. TATSUGUCHI ; K. YAMAMURA ; M. USHIJIMA ; Y. HATTORI ; M. KUKIMOTO-NIINO ; C. MISHIMA-TSUMAGARI ; M. WATANABE.** Cholesterol sulfate is a DOCK2 inhibitor that mediates tissue-specific immune evasion in the eye. *Sci. Signal.,* 2018, vol. 11, eaao4874
- **L. HU ; G. Z. YANG ; Y. ZHANG ; D. FENG ; Y. X. ZHAI ; H. GONG ; C. Y. QI ; H. FU ; M. M. YE ; Q. P. CAI.** Overexpression of SULT2B1b is an independent prognostic indicator and promotes cell growth and invasion in colorectal carcinoma. *Lab. Invest.,* 2015, vol. 95, 1005-1018
- **L. S. EBERLIN ; A. L. DILL ; A. B. COSTA ; D. R. IFA ; L. CHENG ; T. MASTERSON ; M. KOCH ; T. L. RATLIFF ; R. G. COOKS.** Cholesterol sulfate imaging in human prostate cancer tissue by desorption electrospray ionization mass spectrometry. *Anal. Chem.,* 2010, vol. 82, 3430-3434
- **G. DELEZE ; G. PAUMGARTNER ; G. KARLAGANIS ; W. GIGER ; M. REINHARD ; D. SIDIROPOULOS.** Bile acid pattern in human amniotic fluid. *Eur. J. Cli. Invest.,* 1978, vol. 8, 41-45
- **E. I. MINDER ; G. KARLAGANIS ; G. PAUMGARTNER.** Radioimmunological determination of serum 3β-hydroxy-5-cholenoic acid in normal subjects and patients with liver disease. *J. Lipid. Res.,* 1979, vol. 20, 986-993
- **VERYSER, C. ; DEMAEREL, J. ; BIELIUNAS, V. ; GILLES, P. ; DE BORGGRAEVE, W. M.** *Org. Lett.,* 2017, vol. 19, 5244
- **T. H. CORBETT ; D. P. GRISWOLD JR. ; B. J. ROBERTS ; J. C. PECKHAM ; F. M. SCHABEL JR.** Tumor induction relationship in development of transplantable cancers of the colon in mice for chemotherapy assays with a note on carcinogen structure. *Cancer Res.,* 1975, vol. 35, 2434-2439
- **R. J. MOTZER ; B. ESCUDIER ; D.F. MCDERMOTT ; S. GEORGE ; H.J. HAMMERS ; S. SRINIVAS ; S.S. TYKODI ; J.A. SOSMAN ; G. PROCOPIO ; E.R. PLIMACK.** Nivolumab versus Everolimus in advanced renal-cell carcinoma. *N. Engl. J. Med.,* 2015, vol. 373, 1803-1813
- **M. W. MOORE ; F. R. CARBONE ; M. J. BEVAN.** Introduction of soluble protein into the class I pathway of antigen processing and presentation. *Cell,* 1988, vol. 54, 777-785
- **K. A. HOGQUIST ; S. C. JAMESON ; W. R. HEATH ; J. L. HOWARD ; M. J. BEVAN ; F. R. CARBONE.** T cell receptor antagonist peptides induce positive selection. *Cell,* 1994, vol. 76, 17-27
- **C. N. FALANY ; K. J. ROHN-GLOWACKI.** SULT2B1: unique properties and characteristics of a hydroxysteroid sulfotransferase family. *Drug Metab. Rev.,* 2013, vol. 45, 388-400
- **W. CHEN ; G. CHEN ; D. L. HEAD ; D. J. MANGELSDORF ; D. W. RUSSELL.** Enzymatic reduction of oxysterols impairs LXR signaling in cultured cells and the livers of mice. *Cell Metab.,* 2007, vol. 5, 73-79
- **THORNQVIST, V. ; MANNER, S. ; WENDT, O. F. ; FREJD, T.** *Tetrahedron,* 2006, vol. 62, 11793
- **DOMON, K. ; PURIPAT, M ; FUJIYOSHI, K ; HATANAKA, M. ; KAWASHIMA, S. A. ; YAMATSUGU, K. ; KANAI, M.** *ACS Cent. Sci.,* 2020, vol. 6 (2), 283
- **BAUDER, C.** *Org. Biomol. Chem.,* 2008, vol. 6, 2952
- *CHEMICAL ABSTRACTS,* 434-13-9
- *CHEMICAL ABSTRACTS,* 1038743-27-9
- *CHEMICAL ABSTRACTS,* 1029693-41-1
- *CHEMICAL ABSTRACTS,* 19462-13-6
- *CHEMICAL ABSTRACTS,* 1029696-09-0
- *CHEMICAL ABSTRACTS,* 447458-03-9
- *CHEMICAL ABSTRACTS,* 3604-60-2
- *CHEMICAL ABSTRACTS,* 51-98-9
- *CHEMICAL ABSTRACTS,* 68-22-4
- *CHEMICAL ABSTRACTS,* 1047632-27-8
- **SANDIP PRAVIN PATEL et al.** *Molecular Cancer Therapeutics,* 2015, vol. 14 (4), 847-856